(19) Europäisches Patentamt
European Patent Office
Office européen des brevets

(11) **EP 1 246 841 B1**

(12) **EUROPEAN PATENT SPECIFICATION**

(45) Date of publication and mention of the grant of the patent:
**20.09.2006 Bulletin 2006/38**

(21) Application number: **00977763.2**

(22) Date of filing: **01.12.2000**

(51) Int Cl.:
***C07K 14/47*** *(2006.01)* ***C12N 15/12*** *(2006.01)*
***C07K 16/18*** *(2006.01)* ***A61K 38/17*** *(2006.01)*
***A61K 39/00*** *(2006.01)* ***G01N 33/53*** *(2006.01)*
***G01N 33/68*** *(2006.01)* ***C12Q 1/68*** *(2006.01)*

(86) International application number:
**PCT/GB2000/004590**

(87) International publication number:
**WO 2001/040303 (07.06.2001 Gazette 2001/23)**

(54) **TRANSCRIPTION FACTORS CONTAINING TWO POTENTIAL DNA BINDING MOTIFS**

TRANSKRIPTIONSFAKTOREN WELCHE ZWEI POTENTIELLE DNA BINDENDE MOTIVE ENTHALTEN

FACTEURS DE TRANSCRIPTION CONTENANT DEUX MOTIFS DE LIAISON A L'ADN POTENTIELS

(84) Designated Contracting States:
**AT BE CH CY DE DK ES FI FR GB GR IE IT LI LU MC NL PT SE TR**

(30) Priority: **02.12.1999 GB 9928543**
**11.07.2000 GB 0017042**

(43) Date of publication of application:
**09.10.2002 Bulletin 2002/41**

(73) Proprietor: **ISIS INNOVATION LIMITED**
**Summertown,**
**Oxford OX2 7SG (GB)**

(72) Inventors:
- **BANHAM, Alison, Hilary,**
**University of Oxford**
**Headington,**
**Oxfordshire OX3 9DU (GB)**
- **CORDELL, Jacqueline, Loelia,**
**University of Oxford**
**Headington,**
**Oxfordshire OX3 9DU (GB)**
- **JONES, Margaret,**
**University of Oxford**
**Headington,**
**Oxfordshire OX3 9DU (GB)**

(74) Representative: **Baldock, Sharon Claire et al**
**BOULT WADE TENNANT,**
**Verulam Gardens**
**70 Gray's Inn Road**
**London WC1X 8BT (GB)**

(56) References cited:
**WO-A-99/55858**

- **DATABASE EMBL [Online] Accession Number AA048214, 14 January 1997 (1997-01-14) MARRA M ET AL: "mj26a02.r1 Soares mouse embryo cDNA clone" XP002163657**
- **FERBUS D ET AL: "PRODUCTION AND CHARACTERIZATION OF MOUSE MONOCLONAL ANTIBODIES TO HUMAN ZINC FINGER OZF PROTEIN OVEREXPRESSED IN PANCREATIC CARCINOMAS" HYBRIDOMA, vol. 18, no. 5, 1999, pages 431-436, XP000870286**
- **BURLEY STEPHEN K: "DNA-binding motifs from eukaryotic transcription factors." CURRENT OPINION IN STRUCTURAL BIOLOGY, vol. 4, no. 1, 1994, pages 3-11, XP000985676 ISSN: 0959-440X**
- **ADCOCK IM, BARNES PJ: "Transcription Factors" ASTHMA, vol. 1, 1997, pages 337-350, XP000985655**
- **BANHAM AH ET AL.: "FOXP1 a novel candidate tumor suppressor gene on chromosome 3p" CANCER DETECTION AND PREVENTION ONLINE, [Online] October 2000 (2000-10), XP002163656 Retrieved from the Internet: < URL:http://www.cancerprev.org/Meetings/2000/Abstracts/Show?Num=186> [retrieved on 2001-03-16]**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 246 841 B1

SEQUENCE LISTING

<110> ISIS INNOVATION LTD et al

<120> Novel Transcription Factors

<130> 52555/001

<140> PCT/GB00/04590
<141> 2000-12-01

<150> GB 9928543.9
<151> 1999-12-02

<150> GB 0017042.3
<151> 2000-07-11

<160> 10

<170> PatentIn Ver. 2.0

<210> 1
<211> 2337
<212> DNA
<213> Mus musculus

<400> 1

```
ggcaatggtg agggcttcga tcccttctct gatttgctgt cagccatgaa cggatggatg 60
tgatgcctgc tagccaaaag gcttccctct gtgtgttgca gtcctgtggc attatgcatg 120
ccccctccca gtgaccccag gctttttatg gctgtgagac acgttaaaat ttcaggggta 180
agacgtgacc ttttgaggtg actataactg aagattgctt tacagaagcc aaaaaaggtt 240
tttgagtcat gatgcaagaa tctgggactg agacaaaaag taacggttca gccatccaga 300
atgggtcggg cggcagcaac cacttactag agtgcggcgg tcttcgggag gggcggtcca 360
acggagagac gccggccgtg gacatcgggg cagctgacct cgcccacgcc cagcagcagc 420
agcaacaggc acttcaggtg gcaagacagc tccttcttca gcagcaacag cagcagcaag 480
ttagtggatt aaaatctccc aagaggaatg acaaacaacc agctcttcag gttcccgtgt 540
cagtggctat gatgacacct caagttatca ctccccagca aatgcagcag atcctccagc 600
aacaagtgct gagccctcag cagctccagg ttctcctcca gcagcagcag gccctcatgc 660
ttcaacagca gcagcttcaa gagttttata aaaaacaaca ggaacagttg cagcttcaac 720
ttttacaaca acaacatgct ggaaaacagc ctaaagagca acagcaggtg gctacccagc 780
agttggcttt tcagcagcag cttttacaga tgcagcagtt acagcagcag cacctcctgt 840
ctttgcagcg ccaaggcctt ctgacaattc agcccgggca gcctgccctt ccccttcaac 900
ctcttgctca aggcatgatt ccaacagaac tgcagcagct ctggaaagaa gtgacaagtg 960
ctcatactgc agaagaaacc acaggcaaca atcacagcag tttggatctg accacgacat 1020
gtgtctcctc ctctgcacct tccaagacct ccttaataat gaacccacat gcctctacca 1080
atggacagct ctcagtccac actcccaaaa gggaaagttt gtcccatgag gagcaccccc 1140
atagccatcc tctctatgga catggtgtat gcaagtggcc aggctgtgaa gcagtgtgcg 1200
aagatttcca atcatttcta aaacatctca acagtgagca tgcgctggac gatagaagta 1260
```

cagcccaatg tagagtacaa atgcaggttg tacagcagtt agagctacag cttgcaaaag 1320
acaaagaacg cctgcaagcc atgatgaccc acctgcatgt gaagtctaca gaacccaaag 1380
ccgcccctca gcccttgaat ctggtatcaa gtgtcactct ctccaagtcc gcatcggagg 1440
cttctccaca gagcttacct catactccaa cgaccccaac cgccccccctg actcccgtca 1500
cccaaggccc ctctgtcatc acaaccacca gcatgcacac ggtgggaccc atccgcaggc 1560
ggtactcaga caaatacaac gtgcccattt cgtcagcaga tattgcgcag aaccaagaat 1620
tttataagaa cgcagaagtt agaccaccat ttacatatgc atctttaatt aggcaggcca 1680
ttctcgaatc tccagaaaag cagctaacac taaatgagat ctataactgg ttcacacgaa 1740
tgtttgctta cttccgacgc aacgcggcca cgtggaagaa tgcagtgcgt cataatctta 1800
gtcttcacaa gtgttttgtg cgagtagaaa acgttaaagg ggcagtatgg acagtggatg 1860
aagtagaatt ccaaaaacga aggccacaaa agatcagtgg taacccttcc cttattaaaa 1920
acatgcagag cagccacgcc tactgcacac ctctcaatgc agctttacag gcttcaatgg 1980
ctgagaatag tatacctcta tacactaccg cttccatggg aaatcccact ctgggcaact 2040
tagccagcgc aatacgggaa gagctgaacg gggcaatgga gcataccaac agcaacgaga 2100
gtgacagcag tccaggcaga tctcctatgc aagccgtgca tcctgtacac gtcaaagaag 2160
agcccctcga tccagaggaa gctgaagggc ccctgtcctt agtgacaaca gccaaccaca 2220
gtccagattt tgaccatgac agagattacg aagatgaacc agtaaacgag gacatggagt 2280
gactatcggg gcgggccaac cccgagaatg aagattggaa aaaaaaaaaa aaaaaaa 2337

<210> 2
<211> 2031
<212> DNA
<213> Mus musculus

<400> 2

```
atgatgcaag aatctgggac tgagacaaaa agtaacggtt cagccatcca gaatgggtcg 60
ggcggcagca accacttact agagtgcggc ggtcttcggg aggggcggtc caacggagag 120
acgccggccg tggacatcgg ggcagctgac ctcgcccacg cccagcagca gcagcaacag 180
gcacttcagg tggcaagaca gctccttctt cagcagcaac agcagcagca agttagtgga 240
ttaaaatctc ccaagaggaa tgacaaacaa ccagctcttc aggttcccgt gtcagtggct 300
atgatgacac ctcaagttat cactccccag caaatgcagc agatcctcca gcaacaagtg 360
ctgagccctc agcagctcca ggttctcctc cagcagcagc aggccctcat gcttcaacag 420
cagcagcttc aagagtttta taaaaaacaa caggaacagt tgcagcttca acttttacaa 480
caacaacatg ctggaaaaca gcctaaagag caacagcagg tggctaccca gcagttggct 540
tttcagcagc agcttttaca gatgcagcag ttacagcagc agcacctcct gtctttgcag 600
cgccaaggcc ttctgacaat tcagcccggg cagcctgccc ttccccttca acctcttgct 660
caaggcatga ttccaacaga actgcagcag ctctggaaag aagtgacaag tgctcatact 720
gcagaagaaa ccacaggcaa caatcacagc agtttggatc tgaccacgac atgtgtctcc 780
tcctctgcac cttccaagac ctccttaata atgaacccac atgcctctac caatggacag 840
ctctcagtcc acactcccaa aagggaaagt ttgtcccatg aggagcaccc ccatagccat 900
cctctctatg gacatggtgt atgcaagtgg ccaggctgtg aagcagtgtg cgaagatttc 960
caatcatttc taaaacatct caacagtgag catgcgctgg acgatagaag tacagcccaa 1020
tgtagagtac aaatgcaggt tgtacagcag ttagagctac agcttgcaaa agacaaagaa 1080
cgcctgcaag ccatgatgac ccacctgcat gtgaagtcta cagaacccaa agccgcccct 1140
cagcccttga atctggtatc aagtgtcact ctctccaagt ccgcatcgga ggcttctcca 1200
cagagcttac ctcatactcc aacgacccca accgcccccc tgactcccgt cacccaaggc 1260
ccctctgtca tcacaaccac cagcatgcac acggtgggac ccatccgcag gcggtactca 1320
gacaaataca acgtgcccat ttcgtcagca gatattgcgc agaaccaaga attttataag 1380

aacgcagaag ttagaccacc atttacatat gcatctttaa ttaggcaggc cattctcgaa 1440
tctccagaaa agcagctaac actaaatgag atctataact ggttcacacg aatgtttgct 1500
tacttccgac gcaacgcggc cacgtggaag aatgcagtgc gtcataatct tagtcttcac 1560
aagtgttttg tgcgagtaga aaacgttaaa ggggcagtat ggacagtgga tgaagtagaa 1620
ttccaaaaac gaaggccaca aaagatcagt ggtaaccctt cccttattaa aaacatgcag 1680
agcagccacg cctactgcac acctctcaat gcagctttac aggcttcaat ggctgagaat 1740
agtatacctc tatacactac cgcttccatg ggaaatccca ctctgggcaa cttagccagc 1800
gcaatacggg aagagctgaa cggggcaatg gagcatacca acagcaacga gagtgacagc 1860
agtccaggca gatctcctat gcaagccgtg catcctgtac acgtcaaaga agagcccctc 1920
gatccagagg aagctgaagg gcccctgtcc ttagtgacaa cagccaacca cagtccagat 1980
tttgaccatg acagagatta cgaagatgaa ccagtaaacg aggacatgga g          2031
```

<210> 3
<211> 2028
<212> DNA
<213> Mus musculus

<400> 3

```
atgcaagaat ctgggactga gacaaaaagt aacggttcag ccatccagaa tgggtcgggc 60
ggcagcaacc acttactaga gtgcggcggt cttcgggagg ggcggtccaa cggagagacg 120
ccggccgtgg acatcggggc agctgacctc gcccacgccc agcagcagca gcaacaggca 180
cttcaggtgg caagacagct ccttcttcag cagcaacagc agcagcaagt tagtggatta 240
aaatctccca agaggaatga caaacaacca gctcttcagg ttcccgtgtc agtggctatg 300
atgacacctc aagttatcac tccccagcaa atgcagcaga tcctccagca acaagtgctg 360
agccctcagc agctccaggt tctcctccag cagcagcagg ccctcatgct tcaacagcag 420
cagcttcaag agttttataa aaaacaacag gaacagttgc agcttcaact tttacaacaa 480
caacatgctg gaaaacagcc taaagagcaa cagcaggtgg ctacccagca gttggctttt 540
cagcagcagc ttttacagat gcagcagtta cagcagcagc acctcctgtc tttgcagcgc 600
caaggccttc tgacaattca gcccgggcag cctgcccttc cccttcaacc tcttgctcaa 660
ggcatgattc caacagaact gcagcagctc tggaaagaag tgacaagtgc tcatactgca 720
gaagaaacca caggcaacaa tcacagcagt ttggatctga ccacgacatg tgtctcctcc 780
tctgcacctt ccaagacctc cttaataatg aacccacatg cctctaccaa tggacagctc 840
tcagtccaca ctcccaaaag ggaaagtttg tcccatgagg agcaccccca tagccatcct 900
ctctatggac atggtgtatg caagtggcca ggctgtgaag cagtgtgcga agatttccaa 960
tcatttctaa aacatctcaa cagtgagcat gcgctggacg atagaagtac agcccaatgt 1020
agagtacaaa tgcaggttgt acagcagtta gagctacagc ttgcaaaaga caaagaacgc 1080
ctgcaagcca tgatgaccca cctgcatgtg aagtctacag aacccaaagc cgcccctcag 1140
ccccttgaatc tggtatcaag tgtcactctc tccaagtccg catcggaggc ttctccacag 1200
agcttacctc atactccaac gaccccaacc gcccccctga ctcccgtcac ccaaggcccc 1260
tctgtcatca caaccaccag catgcacacg gtgggaccca tccgcaggcg gtactcagac 1320
aaatacaacg tgcccatttc gtcagcagat attgcgcaga accaagaatt ttataagaac 1380
gcagaagtta gaccaccatt tacatatgca tctttaatta ggcaggccat tctcgaatct 1440
ccagaaaagc agctaacact aaatgagatc tataactggt tcacacgaat gtttgcttac 1500
ttccgacgca acgcggccac gtggaagaat gcagtgcgtc ataatcttag tcttcacaag 1560
tgttttgtgc gagtagaaaa cgttaaaggg gcagtatgga cagtggatga agtagaattc 1620
caaaaacgaa ggccacaaaa gatcagtggt aacccttccc ttattaaaaa catgcagagc 1680
agccacgcct actgcacacc tctcaatgca gctttacagg cttcaatggc tgagaatagt 1740
atacctctat acactaccgc ttccatggga aatcccactc tgggcaactt agccagcgca 1800

atacgggaag agctgaacgg ggcaatggag catacccaaca gcaacgagag tgacagcagt 1860
ccaggcagat ctcctatgca agccgtgcat cctgtacacg tcaaagaaga gcccctcgat 1920
ccagaggaag ctgaagggcc cctgtcctta gtgacaacag ccaaccacag tccagatttt 1980
gaccatgaca gagattacga agatgaacca gtaaacgagg acatggag               2028
```

<210> 4
<211> 677
<212> PRT
<213> Mus musculus

<400> 4

```
Met Met Gln Glu Ser Gly Thr Glu Thr Lys Ser Asn Gly Ser Ala Ile
 1               5                  10                  15

Gln Asn Gly Ser Gly Gly Ser Asn His Leu Leu Glu Cys Gly Gly Leu
            20                  25                  30

Arg Glu Gly Arg Ser Asn Gly Glu Thr Pro Ala Val Asp Ile Gly Ala
        35                  40                  45

Ala Asp Leu Ala His Ala Gln Gln Gln Gln Gln Gln Ala Leu Gln Val
    50                  55                  60

Ala Arg Gln Leu Leu Leu Gln Gln Gln Gln Gln Gln Gln Val Ser Gly
65                  70                  75                  80

Leu Lys Ser Pro Lys Arg Asn Asp Lys Gln Pro Ala Leu Gln Val Pro
                85                  90                  95

Val Ser Val Ala Met Met Thr Pro Gln Val Ile Thr Pro Gln Gln Met
            100                 105                 110

Gln Gln Ile Leu Gln Gln Gln Val Leu Ser Pro Gln Gln Leu Gln Val
        115                 120                 125

Leu Leu Gln Gln Gln Gln Ala Leu Met Leu Gln Gln Gln Gln Leu Gln
    130                 135                 140

Glu Phe Tyr Lys Lys Gln Gln Glu Gln Leu Gln Leu Gln Leu Leu Gln
145                 150                 155                 160

Gln Gln His Ala Gly Lys Gln Pro Lys Glu Gln Gln Gln Val Ala Thr
                165                 170                 175

Gln Gln Leu Ala Phe Gln Gln Gln Leu Leu Gln Met Gln Gln Leu Gln
            180                 185                 190

Gln Gln His Leu Leu Ser Leu Gln Arg Gln Gly Leu Leu Thr Ile Gln
```

195 200 205

Pro Gly Gln Pro Ala Leu Pro Leu Gln Pro Leu Ala Gln Gly Met Ile
210 215 220

Pro Thr Glu Leu Gln Gln Leu Trp Lys Glu Val Thr Ser Ala His Thr
225 230 235 240

Ala Glu Glu Thr Thr Gly Asn Asn His Ser Ser Leu Asp Leu Thr Thr
245 250 255

Thr Cys Val Ser Ser Ser Ala Pro Ser Lys Thr Ser Leu Ile Met Asn
260 265 270

Pro His Ala Ser Thr Asn Gly Gln Leu Ser Val His Thr Pro Lys Arg
275 280 285

Glu Ser Leu Ser His Glu Glu His Pro His Ser His Pro Leu Tyr Gly
290 295 300

His Gly Val Cys Lys Trp Pro Gly Cys Glu Ala Val Cys Glu Asp Phe
305 310 315 320

Gln Ser Phe Leu Lys His Leu Asn Ser Glu His Ala Leu Asp Asp Arg
325 330 335

Ser Thr Ala Gln Cys Arg Val Gln Met Gln Val Val Gln Gln Leu Glu
340 345 350

Leu Gln Leu Ala Lys Asp Lys Glu Arg Leu Gln Ala Met Met Thr His
355 360 365

Leu His Val Lys Ser Thr Glu Pro Lys Ala Ala Pro Gln Pro Leu Asn
370 375 380

Leu Val Ser Ser Val Thr Leu Ser Lys Ser Ala Ser Glu Ala Ser Pro
385 390 395 400

Gln Ser Leu Pro His Thr Pro Thr Thr Pro Thr Ala Pro Leu Thr Pro
405 410 415

Val Thr Gln Gly Pro Ser Val Ile Thr Thr Thr Ser Met His Thr Val
420 425 430

Gly Pro Ile Arg Arg Arg Tyr Ser Asp Lys Tyr Asn Val Pro Ile Ser
435 440 445

Ser Ala Asp Ile Ala Gln Asn Gln Glu Phe Tyr Lys Asn Ala Glu Val

450 455 460

Arg Pro Pro Phe Thr Tyr Ala Ser Leu Ile Arg Gln Ala Ile Leu Glu
465 470 475 480

Ser Pro Glu Lys Gln Leu Thr Leu Asn Glu Ile Tyr Asn Trp Phe Thr
485 490 495

Arg Met Phe Ala Tyr Phe Arg Arg Asn Ala Ala Thr Trp Lys Asn Ala
500 505 510

Val Arg His Asn Leu Ser Leu His Lys Cys Phe Val Arg Val Glu Asn
515 520 525

Val Lys Gly Ala Val Trp Thr Val Asp Glu Val Glu Phe Gln Lys Arg
530 535 540

Arg Pro Gln Lys Ile Ser Gly Asn Pro Ser Leu Ile Lys Asn Met Gln
545 550 555 560

Ser Ser His Ala Tyr Cys Thr Pro Leu Asn Ala Ala Leu Gln Ala Ser
565 570 575

Met Ala Glu Asn Ser Ile Pro Leu Tyr Thr Thr Ala Ser Met Gly Asn
580 585 590

Pro Thr Leu Gly Asn Leu Ala Ser Ala Ile Arg Glu Glu Leu Asn Gly
595 600 605

Ala Met Glu His Thr Asn Ser Asn Glu Ser Asp Ser Ser Pro Gly Arg
610 615 620

Ser Pro Met Gln Ala Val His Pro Val His Val Lys Glu Glu Pro Leu
625 630 635 640

Asp Pro Glu Glu Ala Glu Gly Pro Leu Ser Leu Val Thr Thr Ala Asn
645 650 655

His Ser Pro Asp Phe Asp His Asp Arg Asp Tyr Glu Asp Glu Pro Val
660 665 670

Asn Glu Asp Met Glu
675

<210> 5
<211> 1785

<212> DNA
<213> Mus musculus

<400> 5

```
tcgcagaaac agccagaacc catctacagc aagaagacgg aaatccaaag gcagacagta 60
cgggctccct tcgccaaact cttcatttc tctgcacttc aggtggcaag acagctcctt 120
cttcagcagc aacagcagca gcaagttagt ggattaaaat ctcccaagag gaatgacaaa 180
caaccagctc ttcagcaaca gcaggtggct acccagcagt tggcttttca gcagcagctt 240
ttacagatgc agcagttaca gcagcagcac ctcctgtctt tgcagcgcca aggccttctg 300
acaattcagc ccgggcagcc tgcccttccc cttcaacctc ttgctcaagg catgattcca 360
acagaactgc agcagctctg gaaagaagtg acaagtgctc atactgcaga agaaaccaca 420
ggcaacaatc acagcagttt ggatctgacc acgacatgtg tctcctcctc tgcaccttcc 480
aagacctcct taataatgaa cccacatgcc tctaccaatg gacagctctc agtccacact 540
cccaaaaggg aaagtttgtc ccatgaggag cacccccata gccatcctct ctatggacat 600
ggtgtatgca agtggccagg ctgtgaagca gtgtgcgaag atttccaatc atttctaaaa 660
catctcaaca gtgagcatgc gctggacgat agaagtacag cccaatgtag agtacaaatg 720
caggttgtac agcagttaga gctacagctt gcaaaagaca aagaacgcct gcaagccatg 780
atgacccacc tgcatgtgaa gtctacagaa cccaaagccg cccctcagcc cttgaatctg 840
gtatcaagtg tcactctctc caagtccgca tcggaggctt ctccacagag cttacctcat 900
actccaacga ccccaaccgc ccccctgact cccgtcaccc aaggcccctc tgtcatcaca 960
accaccagca tgcacacggt gggacccatc cgcaggcggt actcagacaa atacaacgtg 1020
cccatttcgt cagcagatat tgcgcagaac caagaatttt ataagaacgc agaagttaga 1080
ccaccattta catatgcatc tttaattagg caggccattc tcgaatctcc agaaaagcag 1140
ctaacactaa atgagatcta taactggttc acacgaatgt ttgcttactt ccgacgcaac 1200
gcggccacgt ggaagaatgc agtgcgtcat aatcttagtc ttcacaagtg ttttgtgcga 1260
gtagaaaacg ttaaaggggc agtatggaca gtggatgaag tagaattcca aaaacgaagg 1320
ccacaaaaga tcagtggtaa cccttccctt attaaaaaca tgcagagcag ccacgcctac 1380
tgcacacctc tcaatgcagc tttacaggct tcaatggctg agaatagtat acctctatac 1440
actaccgctt ccatgggaaa tcccactctg ggcaacttag ccagcgcaat acgggaagag 1500
ctgaacgggg caatggagca taccaacagc aacgagagtg acagcagtcc aggcagatct 1560
cctatgcaag ccgtgcatcc tgtacacgtc aaagaagagc ccctcgatcc agaggaagct 1620
gaagggcccc tgtccttagt gacaacagcc aaccacagtc cagattttga ccatgacaga 1680
gattacgaag atgaaccagt aaacgaggac atggagtgac tatcggggcg ggccaacccc 1740
gagaatgaag attggaaaaa ggaaaaaaaa aaaaaaaaac aaaaa                 1785
```

<210> 6
<211> 572
<212> PRT
<213> Mus musculus

<400> 6

```
Ser Gln Lys Gln Pro Glu Pro Ile Tyr Ser Lys Lys Thr Glu Ile Gln
  1               5                  10                  15

Arg Gln Thr Val Arg Ala Pro Phe Ala Lys Leu Phe Ile Phe Ser Ala
                20                  25                  30

Leu Gln Val Ala Arg Gln Leu Leu Leu Gln Gln Gln Gln Gln Gln Gln
            35                  40                  45
```

Val Ser Gly Leu Lys Ser Pro Lys Arg Asn Asp Lys Gln Pro Ala Leu
50 55 60

Gln Gln Gln Gln Val Ala Thr Gln Gln Leu Ala Phe Gln Gln Gln Leu
65 70 75 80

Leu Gln Met Gln Gln Leu Gln Gln Gln His Leu Leu Ser Leu Gln Arg
85 90 95

Gln Gly Leu Leu Thr Ile Gln Pro Gly Gln Pro Ala Leu Pro Leu Gln
100 105 110

Pro Leu Ala Gln Gly Met Ile Pro Thr Glu Leu Gln Gln Leu Trp Lys
115 120 125

Glu Val Thr Ser Ala His Thr Ala Glu Glu Thr Thr Gly Asn Asn His
130 135 140

Ser Ser Leu Asp Leu Thr Thr Thr Cys Val Ser Ser Ser Ala Pro Ser
145 150 155 160

Lys Thr Ser Leu Ile Met Asn Pro His Ala Ser Thr Asn Gly Gln Leu
165 170 175

Ser Val His Thr Pro Lys Arg Glu Ser Leu Ser His Glu Glu His Pro
180 185 190

His Ser His Pro Leu Tyr Gly His Gly Val Cys Lys Trp Pro Gly Cys
195 200 205

Glu Ala Val Cys Glu Asp Phe Gln Ser Phe Leu Lys His Leu Asn Ser
210 215 220

Glu His Ala Leu Asp Asp Arg Ser Thr Ala Gln Cys Arg Val Gln Met
225 230 235 240

Gln Val Val Gln Gln Leu Glu Leu Gln Leu Ala Lys Asp Lys Glu Arg
245 250 255

Leu Gln Ala Met Met Thr His Leu His Val Lys Ser Thr Glu Pro Lys
260 265 270

Ala Ala Pro Gln Pro Leu Asn Leu Val Ser Ser Val Thr Leu Ser Lys
275 280 285

Ser Ala Ser Glu Ala Ser Pro Gln Ser Leu Pro His Thr Pro Thr Thr
290 295 300

Pro Thr Ala Pro Leu Thr Pro Val Thr Gln Gly Pro Ser Val Ile Thr
305 310 315 320

Thr Thr Ser Met His Thr Val Gly Pro Ile Arg Arg Arg Tyr Ser Asp
325 330 335

Lys Tyr Asn Val Pro Ile Ser Ser Ala Asp Ile Ala Gln Asn Gln Glu
340 345 350

Phe Tyr Lys Asn Ala Glu Val Arg Pro Pro Phe Thr Tyr Ala Ser Leu
355 360 365

Ile Arg Gln Ala Ile Leu Glu Ser Pro Glu Lys Gln Leu Thr Leu Asn
370 375 380

Glu Ile Tyr Asn Trp Phe Thr Arg Met Phe Ala Tyr Phe Arg Arg Asn
385 390 395 400

Ala Ala Thr Trp Lys Asn Ala Val Arg His Asn Leu Ser Leu His Lys
405 410 415

Cys Phe Val Arg Val Glu Asn Val Lys Gly Ala Val Trp Thr Val Asp
420 425 430

Glu Val Glu Phe Gln Lys Arg Arg Pro Gln Lys Ile Ser Gly Asn Pro
435 440 445

Ser Leu Ile Lys Asn Met Gln Ser Ser His Ala Tyr Cys Thr Pro Leu
450 455 460

Asn Ala Ala Leu Gln Ala Ser Met Ala Glu Asn Ser Ile Pro Leu Tyr
465 470 475 480

Thr Thr Ala Ser Met Gly Asn Pro Thr Leu Gly Asn Leu Ala Ser Ala
485 490 495

Ile Arg Glu Glu Leu Asn Gly Ala Met Glu His Thr Asn Ser Asn Glu
500 505 510

Ser Asp Ser Ser Pro Gly Arg Ser Pro Met Gln Ala Val His Pro Val
515 520 525

His Val Lys Glu Glu Pro Leu Asp Pro Glu Glu Ala Glu Gly Pro Leu
530 535 540

Ser Leu Val Thr Thr Ala Asn His Ser Pro Asp Phe Asp His Asp Arg
545 550 555 560

Asp Tyr Glu Asp Glu Pro Val Asn Glu Asp Met Glu
565 570

<210> 7
<211> 1643
<212> DNA
<213> Mus musculus

<400> 7

```
acggggtact cccagctgaa ccggctctga atgtagctaa ctcaactgtc agaactgcat 60
gaaggacggt tcccgtgtca gtggctatga tgacacctca agttatcact ccccagcaaa 120
tgcagcagat cctccagcaa caagtgctga gccctcagca gctccaggtt ctcctccagc 180
agcagcaggc cctcatgctt caactgcagc agctctggaa agaagtgaca agtgctcata 240
ctgcagaaga aaccacaggc aacaatcaca gcagtttgga tctgaccacg acatgtgtct 300
cctcctctgc accttccaag acctccttaa taatgaaccc acatgcctct accaatggac 360
agctctcagt ccacactccc aaaagggaaa gtttgtccca tgaggagcac ccccatagcc 420
atcctctcta tggacatggt gtatgcaagt ggccaggctg tgaagcagtg tgcgaagatt 480
tccaatcatt tctaaaacat ctcaacagtg agcatgcgct ggacgataga agtacagccc 540
aatgtagagt acaaatgcag gttgtacagc agttagagct acagcttgca aaagacaaag 600
aacgcctgca agccatgatg acccacctgc atgtgaagtc tacagaaccc aaagccgccc 660
ctcagccctt gaatctggta tcaagtgtca ctctctccaa gtccgcatcg gaggcttctc 720
cacagagctt acctcatact ccaacgaccc caaccgcccc cctgactccc gtcacccaag 780
gcccctctgt catcacaacc accagcatgc acacggtggg acccatccgc aggcggtact 840
cagacaaata caacgtgccc atttcgtcag cagatattgc gcagaaccaa gaattttata 900
agaacgcaga agttagacca ccatttacat atgcatcttt aattaggcag gccattctcg 960
aatctccaga aaagcagcta acactaaatg agatctataa ctggttcaca cgaatgtttg 1020
cttacttccg acgcaacgcg gccacgtgga agaatgcagt gcgtcataat cttagtcttc 1080
acaagtgttt tgtgcgagta gaaaacgtta aaggggcagt atggacagtg gatgaagtag 1140
aattccaaaa acgaaggcca caaaagatca gtggtaaccc ttcccttatt aaaaacatgc 1200
agagcagcca cgcctactgc acacctctca atgcagcttt acaggcttca atggctgaga 1260
atagtatacc tctatacact accgcttcca tgggaaatcc cactctgggc aacttagcca 1320
gcgcaatacg ggaagagctg aacggggcaa tggagcatac caacagcaac gagagtgaca 1380
gcagtccagg cagatctcct atgcaagccg tgcatcctgt acacgtcaaa gaagagcccc 1440
tcgatccaga ggaagctgaa gggcccctgt ccttagtgac aacagccaac cacagtccag 1500
attttgacca tgacagagat tacgaagatg aaccagtaaa cgaggacatg gagtgactat 1560
cggggcgggc caaccccgag aatgaagatt ggaaaaagga aaaaaaaaaa aacacgtcaa 1620
aagttaaaaa aaaaaaaaaa aaa                                         1643
```

<210> 8
<211> 489
<212> PRT
<213> Mus musculus

<400> 8

Met Met Thr Pro Gln Val Ile Thr Pro Gln Gln Met Gln Gln Ile Leu
1               5                   10                  15

Gln Gln Gln Val Leu Ser Pro Gln Gln Leu Gln Val Leu Leu Gln Gln
20 25 30

Gln Gln Ala Leu Met Leu Gln Leu Gln Gln Leu Trp Lys Glu Val Thr
35 40 45

Ser Ala His Thr Ala Glu Glu Thr Thr Gly Asn Asn His Ser Ser Leu
50 55 60

Asp Leu Thr Thr Thr Cys Val Ser Ser Ser Ala Pro Ser Lys Thr Ser
65 70 75 80

Leu Ile Met Asn Pro His Ala Ser Thr Asn Gly Gln Leu Ser Val His
85 90 95

Thr Pro Lys Arg Glu Ser Leu Ser His Glu Glu His Pro His Ser His
100 105 110

Pro Leu Tyr Gly His Gly Val Cys Lys Trp Pro Gly Cys Glu Ala Val
115 120 125

Cys Glu Asp Phe Gln Ser Phe Leu Lys His Leu Asn Ser Glu His Ala
130 135 140

Leu Asp Asp Arg Ser Thr Ala Gln Cys Arg Val Gln Met Gln Val Val
145 150 155 160

Gln Gln Leu Glu Leu Gln Leu Ala Lys Asp Lys Glu Arg Leu Gln Ala
165 170 175

Met Met Thr His Leu His Val Lys Ser Thr Glu Pro Lys Ala Ala Pro
180 185 190

Gln Pro Leu Asn Leu Val Ser Ser Val Thr Leu Ser Lys Ser Ala Ser
195 200 205

Glu Ala Ser Pro Gln Ser Leu Pro His Thr Pro Thr Thr Pro Thr Ala
210 215 220

Pro Leu Thr Pro Val Thr Gln Gly Pro Ser Val Ile Thr Thr Thr Ser
225 230 235 240

Met His Thr Val Gly Pro Ile Arg Arg Arg Tyr Ser Asp Lys Tyr Asn
245 250 255

Val Pro Ile Ser Ser Ala Asp Ile Ala Gln Asn Gln Glu Phe Tyr Lys
260 265 270

```
Asn Ala Glu Val Arg Pro Pro Phe Thr Tyr Ala Ser Leu Ile Arg Gln
        275                 280                 285

Ala Ile Leu Glu Ser Pro Glu Lys Gln Leu Thr Leu Asn Glu Ile Tyr
    290                 295                 300

Asn Trp Phe Thr Arg Met Phe Ala Tyr Phe Arg Arg Asn Ala Ala Thr
305                 310                 315                 320

Trp Lys Asn Ala Val Arg His Asn Leu Ser Leu His Lys Cys Phe Val
                325                 330                 335

Arg Val Glu Asn Val Lys Gly Ala Val Trp Thr Val Asp Glu Val Glu
            340                 345                 350

Phe Gln Lys Arg Arg Pro Gln Lys Ile Ser Gly Asn Pro Ser Leu Ile
        355                 360                 365

Lys Asn Met Gln Ser Ser His Ala Tyr Cys Thr Pro Leu Asn Ala Ala
    370                 375                 380

Leu Gln Ala Ser Met Ala Glu Asn Ser Ile Pro Leu Tyr Thr Thr Ala
385                 390                 395                 400

Ser Met Gly Asn Pro Thr Leu Gly Asn Leu Ala Ser Ala Ile Arg Glu
                405                 410                 415

Glu Leu Asn Gly Ala Met Glu His Thr Asn Ser Asn Glu Ser Asp Ser
            420                 425                 430

Ser Pro Gly Arg Ser Pro Met Gln Ala Val His Pro Val His Val Lys
        435                 440                 445

Glu Glu Pro Leu Asp Pro Glu Glu Ala Glu Gly Pro Leu Ser Leu Val
    450                 455                 460

Thr Thr Ala Asn His Ser Pro Asp Phe Asp His Asp Arg Asp Tyr Glu
465                 470                 475                 480

Asp Glu Pro Val Asn Glu Asp Met Glu
                485
```

<210> 9
<211> 1437
<212> DNA
<213> Mus musculus

<400> 9

```
tgcccttccc cttcaacctc ttgctcaagg catgattcca acagaactgc agcagctctg 60
gaaagaagtg acaagtgctc atactgcaga agaaaccaca ggcaacaatc acagcagttt 120
ggatctgacc acgacatgtg tctcctcctc tgcaccttcc aagacctcct taataatgaa 180
cccacatgcc tctaccaatg gacagctctc agtccacact cccaaaaggg aaagtttgtc 240
ccatgaggag caccccata gccatcctct ctatggacat ggtgtatgca agtggccagg 300
ctgtgaagca gtgtgcgaag atttccaatc atttctaaaa catctcaaca gtgagcatgc 360
gctggacgat agaagtacag cccaatgtag agtacaaatg caggttgtac agcagttaga 420
gctacagctt gcaaaagaca aagaacgcct gcaagccatg atgacccacc tgcatgtgaa 480
gtctacagaa cccaaagccg cccctcagcc cttgaatctg gtatcaagtg tcactctctc 540
caagtccgca tcggaggctt ctccacagag cttacctcat actccaacga ccccaaccgc 600
ccccctgact cccgtcaccc aaggcccctc tgtcatcaca accaccagca tgcacacggt 660
gggacccatc cgcaggcggt actcagacaa atacaacgtg cccatttcgt cagatattgc 720
gcagaaccaa gaattttata agaacgcaga agttagacca ccatttacat atgcatcttt 780
aattaggcag gccattctcg aatctccaga aaagcagcta acactaaatg agatctataa 840
ctggttcaca cgaatgtttg cttacttccg acgcaacgcg gccacgtgga agaatgcagt 900
gcgtcataat cttagtcttc acaagtgttt tgtgcgagta gaaaacgtta aggggcagt 960
atggacagtg gatgaagtag aattccaaaa acgaaggcca caaaagatca gtggtaaccc 1020
ttcccttatt aaaaacatgc agagcagcca cgcctactgc acacctctca atgcagcttt 1080
acaggcttca atggctgaga atagtatacc tctatacact accgcttcca tgggaaatcc 1140
cactctgggc aacttagcca gcgcaatacg ggaagagctg aacggggcaa tggagcatac 1200
caacagcaac gagagtgaca gcagtccagg cagatctcct atgcaagccg tgcatcctgt 1260
acacgtcaaa gaagagcccc tcgatccaga ggaagctgaa gggcccctgt ccttagtgac 1320
aacagccaac cacagtccag attttgacca tgacagagat tacgaagatg aaccagtaaa 1380
cgaggacatg gagtgactat cggggcgggc caaccccgag aatgaagatt ggaaaaa    1437
```

<210> 10
<211> 464
<212> PRT
<213> Mus musculus

<400> 10

```
Ala Leu Pro Leu Gln Pro Leu Ala Gln Gly Met Ile Pro Thr Glu Leu
  1               5                  10                  15

Gln Gln Leu Trp Lys Glu Val Thr Ser Ala His Thr Ala Glu Glu Thr
            20                  25                  30

Thr Gly Asn Asn His Ser Ser Leu Asp Leu Thr Thr Thr Cys Val Ser
        35                  40                  45

Ser Ser Ala Pro Ser Lys Thr Ser Leu Ile Met Asn Pro His Ala Ser
    50                  55                  60

Thr Asn Gly Gln Leu Ser Val His Thr Pro Lys Arg Glu Ser Leu Ser
 65                  70                  75                  80
```

His Glu Glu His Pro His Ser His Pro Leu Tyr Gly His Gly Val Cys
85 90 95

Lys Trp Pro Gly Cys Glu Ala Val Cys Glu Asp Phe Gln Ser Phe Leu
100 105 110

Lys His Leu Asn Ser Glu His Ala Leu Asp Asp Arg Ser Thr Ala Gln
115 120 125

Cys Arg Val Gln Met Gln Val Val Gln Gln Leu Glu Leu Gln Leu Ala
130 135 140

Lys Asp Lys Glu Arg Leu Gln Ala Met Met Thr His Leu His Val Lys
145 150 155 160

Ser Thr Glu Pro Lys Ala Ala Pro Gln Pro Leu Asn Leu Val Ser Ser
165 170 175

Val Thr Leu Ser Lys Ser Ala Ser Glu Ala Ser Pro Gln Ser Leu Pro
180 185 190

His Thr Pro Thr Thr Pro Thr Ala Pro Leu Thr Pro Val Thr Gln Gly
195 200 205

Pro Ser Val Ile Thr Thr Thr Ser Met His Thr Val Gly Pro Ile Arg
210 215 220

Arg Arg Tyr Ser Asp Lys Tyr Asn Val Pro Ile Ser Ser Asp Ile Ala
225 230 235 240

Gln Asn Gln Glu Phe Tyr Lys Asn Ala Glu Val Arg Pro Pro Phe Thr
245 250 255

Tyr Ala Ser Leu Ile Arg Gln Ala Ile Leu Glu Ser Pro Glu Lys Gln
260 265 270

Leu Thr Leu Asn Glu Ile Tyr Asn Trp Phe Thr Arg Met Phe Ala Tyr
275 280 285

Phe Arg Arg Asn Ala Ala Thr Trp Lys Asn Ala Val Arg His Asn Leu
290 295 300

Ser Leu His Lys Cys Phe Val Arg Val Glu Asn Val Lys Gly Ala Val
305 310 315 320

Trp Thr Val Asp Glu Val Glu Phe Gln Lys Arg Arg Pro Gln Lys Ile
325 330 335

```
Ser Gly Asn Pro Ser Leu Ile Lys Asn Met Gln Ser Ser His Ala Tyr
            340                 345                 350

Cys Thr Pro Leu Asn Ala Ala Leu Gln Ala Ser Met Ala Glu Asn Ser
        355                 360                 365

Ile Pro Leu Tyr Thr Thr Ala Ser Met Gly Asn Pro Thr Leu Gly Asn
    370                 375                 380

Leu Ala Ser Ala Ile Arg Glu Glu Leu Asn Gly Ala Met Glu His Thr
385                 390                 395                 400

Asn Ser Asn Glu Ser Asp Ser Ser Pro Gly Arg Ser Pro Met Gln Ala
                405                 410                 415

Val His Pro Val His Val Lys Glu Glu Pro Leu Asp Pro Glu Glu Ala
            420                 425                 430

Glu Gly Pro Leu Ser Leu Val Thr Thr Ala Asn His Ser Pro Asp Phe
        435                 440                 445

Asp His Asp Arg Asp Tyr Glu Asp Glu Pro Val Asn Glu Asp Met Glu
    450                 455                 460
```

## Description

The present invention is concerned with a novel family of proteins, in particular with a family of proteins which contain two potential DNA binding motifs commonly found in transcription factors; a winged helix motif and a $Cys_2$-$His_2$ zinc finger motif.

Transcription factors play a central role in regulating an organisms development as the majority of gene regulation in developmental processes occurs at the transcriptional level (Darnell, J. 1982. Nature 297:365-371). Transcriptional regulators are divided into families which are usually based on the conserved structure of their DNA binding domains. The forkhead domain was defined in 1990 by the homology between the DNA binding domains of hepatocyte nuclear factor (HNF-3) and the Drosophila forkhead gene (Lai, E., V. R. Prezioso, E. Smith, O. Litvin, R. H. Costa, and J. E. Darnell. 1990. Genes & Dev. 4:1427-1436; Weigel, D. and H. Jackle. 1990. Cell 63:455-456; Weigel, D., G. Jurgens, F. Knuttner, E. Seifert, and H. Jackle. 1989. Cell 57:645-658; Weigel, D., E. Seifert, D. Reuter, and H. Jackle. 1990. EMBO J. 9:1199-1207). The family is currently known as the winged helix family, named after its three-dimensional structure when bound to DNA (Clark, K. L., E. D. Halay, E. Lai, and S. K. Burley. 1993. Nature 364:412-420). Members of this family take part in a wide range of normal developmental events including the control of cellular differentiation and proliferation, pattern formation and signal transduction (Kaufmann, E. and W. Knochel. 1996. Mech. Dev. 57:3-20; Lai, E., K. L. Clark, S. K. Burley, and J. E. Darnell Jr. 1991. Proc. Natl. Acad. Sci. U.S.A. 90:10421-10423).

In addition to their normal roles, members of this family participate in mammalian oncogenesis. Qin is a retrovirally transduced murine oncogene (Li, J. and P. K. Vogt. 1993. Proc. Natl. Acad. Sci. U.S.A. 90:4490-4494); a rat and mouse nude mutation disrupts the winged helix gene whn (Nehls, M., Pfeifer, D., Schorpp, M., Hedrich, H. and Boehm, T. (1994) Nature 372: 103-107; Reth, M. (1995) Curr. Biol. 5: 18-20). There are also three winged helix genes which are involved in chromosomal translocations of human malignancies: AFX and AF6q21 have both been identified fused to MLL in t(X;11) (q13;q23)(Parry, P., Wei, Y. and Evans, G. (1994) Cancer 11: 79-84; Borkhardt, A., Repp, R., Haas, O. A., Leis, T., Harbott, J., Kreuder, J., Hammermann, J., Henn, T. and Lampert, F. (1997) Oncogene 14: 195-202) and t (6;11) (q21; q23) (Hillion, J., Le Coniat, M., Jonveaux, P., Berger, R. and Bernard, O. A. (1997) Blood 90: 3714-3719) respectively, in cases of acute leukaemia. Also, FKHR is found fused to members of the paired box or PAX transcription factor family, PAX3 and PAX7, as a result of two translocations t(2;13) and t(1;13) characteristic of alveolar rhabdomyosarcoma (Galili, N., Davis, R. J., Fredericks, W. J., Mukhopadhyay, S., Rauscher, F. J. I., Emanuel, B. S., Rovera, G. and Barr, F. G. (1993) Nat. Genet. 5: 230-235; Davis, R., D'Cruz, C., Lovell, M., Biegel, J. and Barr, F. (1994) Cancer Res. 54: 2869-2872). More recently this family of transcription factors have been identified as additional targets for the PI3K/PKB signalling pathway which has been implicated in tumorigenicity. Reviewed by Kops, G.J. & Burgering B.M. 1999. Forkhead transcription factors: new insights into protein kinase B(c-akt) signalling. J. Mol. Med 77: 656-65.

The present inventors have identified, cloned and sequenced a novel gene which encodes a protein containing a winged helix motif which is widely expressed in both normal and neoplastic human cells. Interestingly, the novel protein contains a second putative nucleic acid binding motif in addition to the winged helix and may therefore represent a previously unknown subclass of winged helix transcription factor proteins.

Accordingly, in a first aspect the invention provides an isolated protein comprising (i) a winged helix motif which has the potential capability of binding to DNA and (ii) a $Cys_2$-$His_2$ zinc finger motif which can also bind nucleic acids.

Unlike any other previously reported members of the winged helix family of proteins the protein of the invention is unique in that it contains a second DNA binding motif which is commonly found in other transcription factors; a $Cys_2$-$His_2$ zinc finger motif. The protein of the invention may therefore be hereinafter referred to as the winged helix/zinc finger protein.

The protein of the invention may additionally contain one or more transcriptional activation domains. The term "transcriptional activation domains" refers to regions of amino acid sequence which are commonly found in transcription factors involved in the regulation of gene expression and which have previously been shown to function by interaction with the basal transcription machinery by both *in vitro* and in *in vivo* assays (Truant, R., Xiao, H., Ingles, C. J. and Greenblatt, J. (1993) J. Biol. Chem. 268, 2284-2287; Xiao, H., Pearson, A., Coulombe, B., Truant, R., Zhang, S., Regier, J. L., Triezenberg, S. J., Reinberg, D., Flores, O. and Ingles, C. J. (1994) Mol. Cell. Biol. 14, 7013-7024). These regions are generally classified according to their amino acid content and include glutamine, acidic amino acid, proline or serine and threonine rich domains.

In a preferred embodiment the protein according to the invention comprises the amino acid sequence set forth in Figure 2 or an amino acid sequence which differs from that shown in Figure 2 only in conservative amino acid changes. As will be shown in the examples given below, in addition to the winged helix and zinc finger motifs this protein further contains two potential nuclear localisation signals and a number of transcriptional activation domains. The presence of these motifs indicates that the protein of the invention may function as a transcription factor.

The invention further provides variants of the above-described winged helix/zinc finger protein which lack potentially functional domains. Accordingly, the invention provides an isolated protein comprising the amino acid sequence set forth in Figure 3C or an amino acid sequence which differs from that shown in Figure 3C only in conservative amino acid changes and an isolated protein comprising the amino acid sequence set forth in Figure 4B or 4D or an amino acid sequence which differs from that shown in Figure 4B or 4D only in conservative amino acid changes.

One of the proteins embodying the invention has been designated FOXP1 in line with a new unified nomenclature for the winged helix/forkhead transcription factors (Kaestner, K. H., W. Knüchel and D. E. Martinez, 2000. 14:142-146). This name has been assigned by Dr Daniel Martinez of the Fox Nomenclature Committee on behalf of the HUGO Nomenclature Committee. All further references to this protein will be as FOXP1 and this will be the name which appears in the Genbank accessions AF146696-AF146698 and AF275309. The *FOXP1* homologues human cDNA clone YX52E07 (accession AF086040) and mouse *QRF1* (accession A49395) have been named *FOXP2* and *Foxp1* respectively. Collectively these genes define a new subgroup of winged helix proteins. The Fox Nomenclature Index maintained on the Web at http://www.biology.pomona.edu/foxindex.html identifies the *FOXP1* gene sequence as submitted by A. Banham.

As is discussed in example 1 below, the native nucleic acid sequence encoding the protein set forth in Figure 2 contains two consecutive in-frame ATG initiation codons. The amino acid sequence set forth in Figure 2 corresponds to the amino acid sequence of a translated protein product initiating at the first (i.e. upstream) ATG codon. However, it is postulated that the majority of ribosomes will actually initiate translation at the second of these initiation codons since it has a better Kozak consensus. Accordingly, translated protein products which initiate at the second (i.e. downstream) ATG codon and therefore possess only one N-terminal methionine are also to be included within the scope of the invention, including a protein having the amino acid sequence set forth in Figure 2 but lacking the extreme N-terminal methionine residue.

The invention further provides isolated nucleic acid molecules encoding the proteins of the invention.

Also provided by the invention is an isolated nucleic acid molecule comprising the sequence of nucleotides shown from position 264 or position 267 to position 2294 of the nucleic acid sequence set forth in Figure 2, an isolated nucleic acid molecule comprising the complete sequence of nucleotides set forth in Figure 2, an isolated nucleic acid molecule comprising the sequences of nucleotides set forth in Figure 3A and 3B and an isolated nucleic acid molecule comprising the sequences of nucleotides set forth in Figure 4A or 4C.

A further aspect of the invention comprises nucleic acids capable of hybridising to the nucleic acid molecules according to the invention, and preferably capable of hybridising to the sequence of nucleotides set forth in any of Figures 2, 3A and 3B, 4A or 4C, under high stringency conditions.

Stringency of hybridisation as used herein refers to conditions under which polynucleic acids are stable. The stability of hybrids is reflected in the melting temperature (Tm) of the hybrids. Tm can be approximated by the formula:

$$81.5°C+16.6(\log_{10}[Na^+]+0.41\ (\%G\&C)-600/1$$

wherein 1 is the length of the hybrids in nucleotides. Tm decreases approximately by 1-1.5°C with every 1% decrease in sequence homology.

The term "stringency" refers to the hybridisation conditions wherein a single-stranded nucleic acid joins with a complementary strand when the purine or pyrimidine bases therein pair with their corresponding base by hydrogen bonding. High stringency conditions favour homologous base pairing whereas low stringency conditions favour non-homologous base pairing.

"Low stringency" conditions comprise, for example, a temperature of about 37°C or less, a formamide concentration of less than about 50%, and a moderate to low salt (SSC) concentration; or, alternatively, a temperature of about 50°C or less, and a moderate to high salt (SSPE) concentration, for example 1M NaCl.

"High stringency" conditions comprise, for example, a temperature of about 42°C or less, a formamide concentration of less than about 20%, and a low salt (SSC) concentration; or, alternatively, a temperature of about 65°C, or less, and a low salt (SSPE) concentration. For example, high stringency conditions comprise hybridization in 0.5 M $NaHPO_4$, 7% sodium dodecyl sulfate (SDS), 1 mM EDTA at 65°C (Ausubel, F.M. et al. Current Protocols in Molecular Biology, Vol. I, 1989; Green Inc. New York, at 2.10.3).

"SSC" comprises a hybridization and wash solution. A stock 20X SSC solution contains 3M sodium chloride, 0.3M sodium citrate, pH 7.0.

"SSPE" comprises a hybridization and wash solution. A 1X SSPE solution contains 180 mM NaCl, 9mM $Na_2HP0_4$ and 1 mM EDTA, pH 7.4.

The nucleic acid capable of hybridising to nucleic acid molecules according to the invention will generally be at least 70%, preferably at least 80 or 90% and more preferably at least 95% homologous to the nucleotide sequences according to the invention.

An antisense molecule capable of hybridising to the nucleic acid according to the invention may be used as a probe or as a medicament or may be included in a pharmaceutical composition with a pharmaceutically acceptable carrier, diluent or excipient therefor.

The term "homologous" describes the relationship between different nucleic acid molecules or amino acid sequences wherein said sequences or molecules are related by partial identity or similarity at one or more blocks or regions within said molecules or sequences. Homology may be determined by means of computer programs known in the art.

Substantial homology preferably carries with it that the nucleotide and amino acid sequences of the protein of the invention comprise a nucleotide and amino acid sequence fragment, respectively, corresponding and displaying a certain degree of sequence identity to the amino acid and nucleic acid sequences identified in the figures. Preferably they share an identity of at least 30 %, preferably 40 %, more preferably 50 %, still more preferably 60 %, most preferably 70%, and particularly an identity of at least 80 %, preferably more than 90 % and still more preferably more than 95 % is desired with respect to the nucleotide or amino acid sequences depicted in Figures 2, 3A, 3B, 3C, 4A, 4B, 4C or 4D, respectively. A preferred method for determining the best overall match between a query sequence (a sequence of the present invention) and a subject sequence, also referred to as a global sequence alignment, can be determined using, for example, the FASTDB computer program based on the algorithm of Brutlag et al. (Comp. App. Biosci. 6 (1990), 237-245.) In a sequence alignment the query and subject sequences are both DNA sequences. An RNA sequence can be compared by converting U's to T's. The result of said global sequence alignment is in percent identity. Further programs that can be used in order to determine homology/identity are described below and in the examples. The sequences that are homologous to the sequences described above are, for example, variations of said sequences which represent modifications having the same biological function, in particular encoding proteins with the same or substantially the same specificity, e.g. binding specificity. They may be naturally occurring variations, such as sequences from other mammals, or mutations. These mutations may occur naturally or may be obtained by mutagenesis techniques. The allelic variations may be naturally occurring allelic variants as well as synthetically produced or genetically engineered variants. In a preferred embodiment the sequences are derived from human.

The nucleic acid molecules according to the invention may, advantageously, be included in a suitable expression vector to express the proteins encoded therefrom in a suitable host. Incorporation of cloned DNA into a suitable expression vector for subsequent transformation of said cell and subsequent selection of the transformed cells is well known to those skilled in the art as provided in Sambrook et al. (1989), Molecular cloning: A Laboratory Manual, Cold Spring Harbour Laboratory.

An expression vector according to the invention includes a vector having a nucleic acid according to the invention operably linked to regulatory sequences, such as promoter regions, that are capable of effecting expression of said DNA fragments. The term "operably linked" refers to a juxtaposition wherein the components described are in a relationship permitting them to function in their intended manner. Such vectors may be transformed into a suitable host cell to provide for expression of a protein according to the invention. Thus, in a further aspect, the invention provides a process for preparing proteins according to the invention which comprises cultivating a host cell, transformed or transfected with an expression vector as described above under conditions to provide for expression by the vector of a coding sequence encoding the protein, and recovering the expressed protein. As will be illustrated in the accompanying Examples, the winged helix/zinc finger protein of the invention has been successfully expressed in both bacterial and eukaryotic host cells.

In this regard, the nucleic acid molecule may encode a mature protein or a protein having a prosequence, including encoding a leader sequence on the preprotein which is cleaved by the host cell to form a mature protein.

The vectors may be, for example, plasmid, virus or phage vectors provided with an origin of replication, and optionally a promoter for the expression of said nucleotide and optionally a regulator of the promoter. The vectors may contain one or more selectable markers, such as, for example, an antibiotic resistance.

Regulatory elements required for expression include promoter sequences to bind RNA polymerase and to direct an appropriate level of transcription initiation and also translation initiation sequences for ribosome binding. For example, a bacterial expression vector may include a promoter such as the lac promoter and for translation initiation the Shine-Dalgarno sequence and the start codon AUG. Similarly, a eukaryotic expression vector may include a heterologous or homologous promoter for RNA polymerase II, a downstream polyadenylation signal, the start codon AUG, and a termination codon for detachment of the ribosome. Such vectors may be obtained commercially or be assembled from the sequences described by methods well known in the art.

Transcription of DNA encoding the polypeptides of the present invention by higher eukaryotes is optimised by including an enhancer sequence in the vector. Enhancers are cis-acting elements of DNA that act on a promoter to increase the level of transcription. Vectors will also generally include origins of replication in addition to the selectable markers.

Nucleic acid molecules according to the invention may be inserted into the vectors described in an antisense orientation in order to provide for the production of antisense RNA. Antisense RNA or other antisense nucleic acids, including antisense peptide nucleic acid (PNA), may be produced by synthetic means.

In accordance with the present invention, a defined nucleic acid includes not only the identical nucleic acid but also any minor base variations including in particular, substitutions in cases which result in a synonymous codon (a different codon specifying the same amino acid residue) due to the degenerate code in conservative amino acid substitutions. The term "nucleic acid sequence" also includes the complementary sequence to any single stranded sequence given regarding base variations.

The present invention also advantageously provides oligonucleotides comprising at least 10 consecutive nucleotides of a nucleic acid according to the invention and preferably from 10 to 40 consecutive nucleotides of a nucleic acid according to the invention. These oligonucleotides may, advantageously be used as probes or primers to initiate replication, or the like. Oligonucleotides having a defined sequence may be produced according to techniques well known in the art, such as by recombinant or synthetic means. They may also be used in diagnostic kits or the like for detecting the presence of a nucleic acid according to the invention. These tests generally comprise contacting the probe with the sample under hybridising conditions and detecting for the presence of any duplex or triplex formation between the probe and any nucleic acid in the sample.

According to the present invention these probes may be anchored to a solid support. Preferably, they are present on an array so that multiple probes can simultaneously hybridize to a single biological sample. The probes can be spotted onto the array or synthesised *in situ* on the array. (See Lockhart et al., Nature Biotechnology, vol. 14, December 1996 "Expression monitoring by hybridisation to high density oligonucleotide arrays".

The nucleic acid sequences according to the invention may be produced using recombinant or synthetic techniques, such as for example using PCR which generally involves making a pair of primers, which may be from approximately 10 to 50 nucleotides to a region of the gene which is desired to be cloned, bringing the primers into contact with cDNA, or genomic DNA from a human cell, performing a polymerase chain reaction under conditions which brings about amplification of the desired region, isolating the amplified region or fragment and recovering the amplified DNA. Generally, such techniques are well known in the art, such as described in Sambrook et al. (Molecular Cloning: a Laboratory Manual, 1989).

The nucleic acids or oligonucleotides according to the invention may carry a revealing label. Suitable labels include radioisotopes such as $^{32}P$ or $^{35}S$, enzyme labels or other protein labels such as biotin or fluorescent markers. Such labels may be added to the nucleic acids or oligonucleotides of the invention and may be detected using known techniques *per se.*

Advantageously, human allelic variants or polymorphisms of the nucleic acid according to the invention may be identified by, for example, probing cDNA or genomic libraries from a range of individuals, for example, from different populations. Furthermore, nucleic acids and probes according to the invention may be used to sequence genomic DNA from patients using techniques well known in the art, such as the Sanger Dideoxy chain termination method, which may, advantageously, ascertain any predisposition of a patient to disorders associated with variants of the winged helix/zinc finger protein.

The nucleotide sequences identified herein according to the invention can be used in numerous ways as a reagent. The following description should be considered exemplary and utilizes known techniques. There exists an ongoing need to identify new chromosome markers, since few chromosome marking reagents, based on actual sequence data (repeat polymorphisms), are presently available. The FOXP1 sequence maps to chromosome 3. Thus, nucleotide sequences encoding FOXP1 can be used in linkage analysis as a marker for chromosome 3. The sequence has been mapped to a particular region between markers D351261-D351604 of the chromosome using well known techniques. These include in situ hybridization to chromosomal spreads, flow-sorted chromosomal preparations, or artificial chromosome constructions such as yeast artificial chromosomes, bacterial artificial chromosomes, bacterial P1 constructions or single chromosome cDNA libraries as reviewed in Price (Blood Rev. 7 (1993), 127-134) and Trask (Trends Genet. 7 (1991), 149-154). The technique of fluorescent in situ hybridization of chromosome spreads has been described, among other places, in Verma, (1988) Human Chromosomes: A Manual of Basic Techniques, Pergamon Press, New York NY. Fluorescent *in situ* hybridization of chromosomal preparations and other physical chromosome mapping techniques may be correlated with additional genetic map data. Examples of genetic map data can be found in the art. Correlation between the location of the gene encoding a FOXP1 polypeptide on a physical chromosomal map and a specific feature, e.g., a disease related to the dysfunction of the gene may help to delimit the region of DNA associated with this feature. The nucleotide sequences of the subject invention may be used to detect differences in gene sequences between normal, carrier or affected individuals. Furthermore, the means and methods described herein can be used for marker-assisted animal breeding.

*In situ* hybridization of chromosomal preparations and physical mapping techniques such as linkage analysis using established chromosomal markers may be used for extending genetic maps. For example a sequence tagged site based map of the human genome was recently published by the Whitehead-MIT Center for Genomic Research (Hudson, Science 270 (1995), 1945-1954) and is also available on the internet. Often the placement of a gene on the chromosome of another species may reveal associated markers even if the number or arm of a particular chromosome is not known. New sequences can be assigned to chromosomal arms, or parts thereof, by physical mapping. This provides valuable information to investigators searching for interacting genes using positional cloning or other gene discovery techniques. Once such gene has been crudely localized by genetic linkage to a particular genomic region, any sequences mapping to that area may represent associated or regulatory genes for further investigation. The nucleotide sequence of the subject invention may also be used to detect differences in the chromosomal location due to translocation, inversion, etc. among normal, carrier or affected individuals.

Briefly, sequences can be mapped to chromosomes by preparing PCR primers (preferably 15-25 bp) from the sequences shown in any of Figures 2, 3A, 4A or 4C. Primers can be selected using computer analysis so that primers do not span more than one predicted exon in the genomic DNA. These primers are then used for PCR screening of somatic cell hybrids containing individual human chromosomes. Only those hybrids containing the human gene of interest corresponding to the above sequences will yield an amplified fragment.

Similarly, somatic hybrids provide a rapid method of PCR mapping the nucleotide sequences to particular chromosomes. Three or more clones can be assigned per day using a single thermal cycler. Moreover, sublocalization of the nucleotide sequences can be achieved with panels of specific chromosome fragments. Other gene mapping strategies that can be used include in situ hybridization, prescreening with labeled flow-sorted chromosomes, and preselection by hybridization to construct chromosome specific cDNA libraries.

Precise chromosomal location of the nucleotide sequences can also be achieved using fluorescence in situ hybridization (FISH) of a metaphase chromosomal spread. This technique uses nucleotide sequences as short as 300 to 600 bases; however, nucleotide sequences 1,000-4,000 bp are preferred. For a review of this technique, see Verma et al., "Human Chromosomes: a Manual of Basic Techniques," Pergamon Press, New York (1988).

For chromosome mapping, the nucleotide sequences can be used individually (to mark a single chromosome or a single site on that chromosome) or in panels (for marking multiple sites and/or multiple chromosomes).

Once a nucleotide sequence has been mapped to a precise chromosomal location, the physical position of the nucleotide sequence can be used in linkage analysis. Linkage analysis establishes coinheritance between a chromosomal location and presentation of a particular disease. (Disease mapping data are found, for example, in McKusick, Mendelian Inheritance in Man (available on line through Johns Hopkins University Welch Medical Library)). Assuming 1 megabase mapping resolution and one gene per 20 kb, a cDNA precisely localized to a chromosomal region associated with the disease could be one of 50-500 potential causative genes.

Thus, once coinheritance is established, differences in the nucleotide sequences of the invention and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide encoded and the corresponding gene from several normal individuals is required to distinguish the mutation from a polymorphism. If a new polymorphism is identified, this polymorphic polypeptide can be used for further linkage analysis. In the very least, the nucleotide sequences can be used as molecular weight markers on Southern gels, as diagnostic probes for the presence of a specific mRNA in a particular cell type, as a probe to "subtract-out" known sequences in the process of discovering novel nucleotide sequences, for selecting and making oligomers for attachment to a "gene chip" or other support, to raise anti-DNA antibodies using DNA immunization techniques, and as an antigen to elicit an immune response.

The protein according to the invention includes all possible amino acid variants encoded by the nucleic acid molecule according to the invention including a protein encoded by said molecule and having conservative amino acid changes. Proteins or polypeptides according to the invention further include variants of such sequences, including naturally occurring allelic variants which are substantially homologous to said proteins or polypeptides. In this context, substantial homology is regarded as a sequence which has at least 70%, preferably 80 or 90% and preferably 95% amino acid homology with the proteins or polypeptides encoded by the nucleic acid molecules according to the invention. The protein according to the invention may be recombinant, synthetic or naturally occurring, but is preferably recombinant.

The present invention is further directed to inhibiting expression of the proteins of the invention *in vivo* by the use of antisense technology. Antisense technology can be used to control gene expression through triple-helix formation of antisense DNA or RNA, both of which methods are based on binding of a polynucleotide to DNA or RNA. For example, the 5' coding portion or the mature protein sequence, which encodes for the protein of the present invention, is used to design an antisense RNA oligonucleotide of from 10 to 40 base pairs in length. The antisense RNA oligonucleotide hybridises to the mRNA *in vivo* and blocks translation of an mRNA molecule into the protein (antisense - Okano, J. Neurochem., 56:560 (1991); Oligodeoxynucleotides as Antisense Inhibitors of Gene Expression, CRC Press, Boca Raton, FL (1988)). A DNA oligonucleotide is designed to be complementary to a region of the gene involved in transcription (triple-helix - see Lee et al. Nucl. Acids Res., 6:3073 (1979); Cooney et al., Science, 241:456 (1988); and Dervan et al., Science, 251: 1360 (1991), thereby preventing transcription and the production of the protein.

Encompassed within the scope of the invention are hybrid and modified forms of the protein according to the invention including fusion proteins and fragments. The hybrid and modified forms include, for example, when certain amino acids have been subjected to some modification or replacement, such as for example, by point mutation and yet which results in a protein which possesses the same function as the proteins of the invention.

The antisense oligonucleotide described above can be delivered to cells by procedures in the art such that the anti-sense RNA and DNA may be expressed *in vivo* to inhibit production of the protein in the manner described above.

A further aspect of the invention provides a host cell or organism, transformed or transfected with an expression vector according to the invention. The host cell or organism may advantageously be used in a method of producing protein, which comprises recovering any expressed protein from the host or organism transformed or transfected with the expression vector.

According to a further aspect of the invention there is also provided a transgenic cell, tissue or organism comprising a transgene capable of expressing a protein according to the invention. The term "transgene capable of expressing" as used herein encompasses any suitable nucleic acid sequence which leads to expression of proteins having the same function and/or activity. The transgene, may include, for example, genomic nucleic acid isolated from human cells or synthetic nucleic acid, including DNA integrated into the genome or in an extrachromosomal state. Preferably, the transgene comprises the nucleic acid sequence encoding the proteins according to the invention as described herein, or a functional fragment of said nucleic acid. A functional fragment of said nucleic acid should be taken to mean a fragment of the gene comprising said nucleic acid coding for the proteins according to the invention or a functional equivalent, derivative or a non-functional derivative such as a dominant negative mutant, or bioprecusor of said proteins.

Knock-out mice may also be generated to further investigate the role of FOXP1 *in vivo.* Furthermore, transgenic animals, such as mice, may be used to overexpress the FOXP1 protein according to the invention to further investigate its role *in vivo.*

The protein expressed by said transgenic cell, tissue or organism or a functional equivalent or bioprecusor of said protein also forms part of the present invention. Recombinant proteins may be recovered and purified from host cell cultures by methods known in the art, including ammonium sulfate or ethanol precipitation, acid extraction, anion or cation exchange chromatography, phosphocellulose, chromatography, hydrophobic interaction chromatography, affinity chromatography, hydroxyapatite chromatography and lectin chromatography.

The protein of the present invention may be a naturally purified product, or a product of chemical synthetic procedures, or produced by recombinant techniques from a prokaryotic or eukaryotic host (for example, by bacterial yeast, higher plant, insect and mammalian cells in culture). Depending upon the host employed in a recombinant production procedure, the expressed protein may lack the initiating methionine residue as a result of post-translational cleavage. Proteins which have been modified in this way are also included within the scope of the invention.

In a still further aspect the invention provides an antibody which is capable of binding to the winged helix/zinc finger proteins of the invention or an epitope thereof. An antibody according to the invention may be raised according to standard techniques well known to those skilled in the art by using the protein of the invention or a fragment or single epitope thereof as the challenging antigen. A preferred antibody is the monoclonal antibody designated JC12 which is obtainable from a hybridoma deposited in accordance with the provisions of The Budapest Treaty of 1977 with the European Collection of Cell Cultures, Centre for Applied Microbiology & Research, Salisbury, Wiltshire, SP4 0JG, UK, on 14 April 1999 under accession No. 99041425.

The present invention includes not only complete antibody molecules but fragments thereof. Antibody fragments which contain the idiotype of the molecule can be generated by known techniques, for example, such fragments include but are not limited to the $F(ab')_2$ fragment which can be produced by pepsin digestion of the antibody molecule; the Fab' fragments which can be generated by reducing the disulfide bridges of the $F(ab')_2$ fragments and the Fab fragments which can be generated by treating the antibody molecule with papain and a reducing agent. Chimeric humanized and fully humanized mAb can now be made by recombinant engineering. By addition of the human constant chain to $F(ab')_2$ fragments it is possible to create a humanized monoclonal antibody which is useful in immunotherapy applications where patients making antibodies against the mouse Ig would otherwise be at a disadvantage. Breedveld F.C. Therapeutic Monoclonal Antibodies. Lancet 2000 Feb 26; 335, P735-40.

A further aspect of the present invention also provides a method of identifying a protein of the invention in a sample, which method comprises contacting said sample with an antibody as described herein and monitoring for any specific binding of any proteins to said antibody. A kit for identifying the presence of such proteins in a sample is also provided comprising an antibody according to the invention and means for contacting said antibody with said sample.

In a further aspect the invention provides an in vitro method of detecting expression of a protein comprising a winged helix motif and a $Cys_2$-$His_2$ zinc finger motif in a mammalian subject, which method comprises contacting a sample of tissue or cells removed from the mammalian subject with an antibody which is capable of binding to the winged helix/zinc finger protein of the invention or an epitope thereof and detecting specific binding of the antibody to its target protein in the said tissue.

Preferably the method of the invention is performed on cells or tissues removed from a human subject. However, it is also within the scope of the invention to perform the method on cells or tissues removed from non-human mammals such as mouse or monkey by using an antibody which is cross-reactive against a homologous protein expressed in the non-human mammalian species.

As is demonstrated in Example 2 below, immunostaining with an antibody immunologically specific for the winged helix/zinc finger protein, such as the monoclonal antibody JC12 described herein, can be used to detect expression of the winged helix/zinc finger protein in samples of normal and neoplastic human tissues. Using this method the winged helix/zinc finger protein was initially shown to be expressed to varying levels by the majority of haematopoietic neoplasms, including many lymphomas (of both B cell and T cell origin) and also by some carcinomas.

Interestingly, the sub-cellular localisation of the winged helix/zinc finger protein (FOXP1) is observed to be different in different types of cells. As shown in the Examples included herein, staining of three diffuse large B-cell lymphomas of anaplastic morphology with the JC12 monoclonal antibody was more cytoplasmic than observed in other cases of diffuse large B-cell lymphomas, in which staining was almost exclusively nuclear. However, expression of this protein in non-haematological malignancies, again identified by immunohistochemical staining with JC12 monoclonal antibody, revealed a significantly different expression pattern, where a significant decrease or loss of expression in the nucleus was often observed or alternatively expression only occurred in the cytoplasm.

Staining of tissues with an antibody immunologically specific for the winged helix/zinc finger protein may therefore be useful both in the diagnosis of lymphomas and carcinomas and also to distinguish between different subtypes of diffuse large B-cell lymphoma and different subtypes of mantle cell lymphoma. The latter may be important both in assessing the prognosis of patients with these lymphomas and in selecting an appropriate course of treatment. Furthermore, the level of expression of the winged helix/zinc finger protein may be related to the grade of tumour, i.e. how aggressive the tumour is. Evaluating the level of expression by staining of tissue sections using an antibody immunologically specific to the winged helix/zinc finger protein may therefore also have prognostic implications. In both stomach and colon tumours the change in JC12 staining which is observed between normal and malignant cells is detectable in pre-malignant lesions. Immunostaining with the antibody specific to the winged helix/zinc finger protein may be diagnostically useful in identifying early changes in cells which occur before they are malignant. The antibody may therefore be useful in screening programmes to detect pre-malignant cells.

Proteins which interact with the polypeptide of the invention may be identified by identification of proteins which co-immunoprecipitate with the protein of the invention using the JC12 monoclonal antibody. Alternatively, such interacting proteins may be identified by investigating protein-protein interactions using the two-hybrid vector system first proposed by Chien *et al* (1991), Proc. Natl. Acad. Sci. USA 88: 9578-9582.

This technique is based on functional reconstitution *in vivo* of a transcription factor which activates a reporter gene. More particularly the technique comprises providing an appropriate host cell with a DNA construct comprising a reporter gene under the control of a promoter regulated by a transcription factor having a DNA binding domain and an activating domain, expressing in the host cell a first hybrid DNA sequence encoding a first fusion of a fragment or all of a nucleic acid sequence according to the invention and either said DNA binding domain or said activating domain of the transcription factor, expressing in the host at least one second hybrid DNA sequence, such as, a library or the like, encoding putative binding proteins to be investigated together with the DNA binding or activating domain of the transcription factor which is not incorporated in the first fusion; detecting any binding of the proteins to be investigated with a protein according to the invention by detecting for the presence of any reporter gene product in the host cell; optionally isolating second hybrid DNA sequences encoding the binding protein.

The nucleic acid molecules and the amino acid or protein sequences, may advantageously be used in the treatment of the human or animal body or alternatively in the manufacture of a medicament for treating cancer. They may also be included in a pharmaceutical composition together with any suitable pharmaceutically acceptable carrier diluent or excipient therefor. The nucleic acid molecule or the amino acid sequence or protein may be encapsulated and/or combined with suitable carriers in solid dosage forms for oral administration which would be well known to those of skill in the art or alternatively with suitable carriers for administration in an aerosol spray.

In the pharmaceutical composition of the invention, preferred compositions include pharmaceutically acceptable carriers including, for example, non-toxic salts, sterile water or the like. A suitable buffer may also be present allowing the compositions to be lyophilized and stored in sterile conditions prior to reconstitution by the addition of sterile water for subsequent administration. The carrier can also contain other pharmaceutically acceptable excipients for modifying other conditions such as pH, osmolarity, viscosity, sterility, lipophilicity, somobility or the like. Pharmaceutical compositions which permit sustained or delayed release following administration may also be used.

Furthermore, as would be appreciated by the skilled practitioner, the specific dosage regime may be calculated according to the body surface area of the patient or the volume of body space to be occupied, dependent on the particular route of administration to be used. The amount of the composition actually administered will, however, be determined by a medical practitioner based on the circumstances pertaining to the disorder to be treated, such as the severity of the symptoms, the age, weight and response of the individual.

Also provided by the present invention is a method of treating cancer in a patient which method comprises administering to said patient an amount of a nucleic acid according to the invention or a protein according to the invention, or antibody JC12 or a humanised derivative thereof.

It has recently been suggested by Boussiotis, V.A., *et al.,* 2000, Nature Medicine 6 290-297 that certain pharmacological agents up regulate the expression of or prevent the degradation of $p27^{kip1}$ during antigen recognition. Thus, advantageously, it may, therefore be possible to modify the levels of FOXP1 in a cell to control $p27^{kip1}$ expression and thus regulate antigen specific T-cell responsiveness which may be particularly useful, for example, in preventing graft-versus-host disease in transplants. Therefore, there is provided a method of controlling T-cell responsiveness in a mammal, which method comprises increasing or decreasing the level of FOXP1 expression/function, wherein high levels of expression of FOXP1 induce or mediate antigen-specific T-cell unresponsiveness in said individual.

Furthermore, as shown in more detail in the examples provided, the loss of FOXP1 protein expression in the nucleus may be functionally linked to the observations that loss of heterozygosity on chromosome 3p and decrease in $p27^{kip1}$ protein expression are known to be early events in the development of solid tumours and are associated with a poor prognosis. Thus, analysis of the FOXP1 gene sequence or aberrant patterns of its mRNA or protein expression could be used to develop screening programmes for early detection of premalignant lesions in a range of tumour types, e.g. cervical, prostate, stomach, colon, head and neck, renal, breast or lung. Thus, the present invention also provides a method of diagnosing the medicinal significance of premalignant lesions in a patient which method comprises detecting a change from the normal pattern of expression or function of a protein according to the invention in said patient, wherein the change in expression pattern or function of said protein is indicative of the likelihood of said patient's premalignant lesions developing into a malignant tumour. The loss of normal function may occur because of changes in the level of expression and/or sublocalisation of the FOXP1 protein. The method according to the aspect of the invention will preferably be used to detect non-haematological malignancies and preferably, cervical, breast, prostate, stomach, colon, head and neck, renal and lung malignancies. Further provided is a method of screening for predisposition to cancer in an individual which comprises screening for an inherited genetic mutation in a nucleic acid sequence from said individual encoding a protein according to the invention.

Methylation as a means of controlling of gene expression is currently under investigation by many groups working on human cancers. In tumours which retain a methylated but otherwise functional copy of the gene of interest the use of methylation inhibitors can be used therapeutically to restore gene expression. The lack of reported loss of heterozygosity at the chromosome 3p12-14 in haematological malignancies despite the frequent loss of expression of the FOXP1 protein which we have observed in DLBCL indicates that methylation of this gene may be a mechanism for its inactivation. Therefore, either hyper or hypo methylation of the FOXP1 promoter may be used in diagnostic detection of diseased conditions associated with changes in FOXP1 expression levels. For example methylation of the FOXP1 promoter could be used to assess neoplastic progression/prognosis. Current Topics in Microbiology and Immunology. Vol. 249: DNA Methylation and Cancer edited by P. A. Jones and P. K. Vogt Springer-Verlag (2000) pp. 170. ISBN 3-540-66608-7. Herman J.G. and Baylin S.B. Promoter-region hypermethylation and gene silencing in human cancer; Curr Top Microbiol Immunol. 2000;249:35-54. Momparler R.L., Bovenzi, V. DNA methylation and cancer. J Cell Physiol. 2000 May;183(2):145-54; Herman, J.G. Hypermethylation of tumor suppressor genes in cancer. Semin Cancer Biol. 1999 Oct:9(5):359-67.

Therefore, according to a further aspect of the invention, there is provided a method of detecting cancer associated with reduced or increased levels of expression of a FOXP1 protein according to the invention, comprising detecting respectively increased or decreased levels of methylation of a regulatory region (promoter) of the FOXP1 genomic DNA according to the invention.

Furthermore, there is also provided a method of treating or alleviating cancer associated with reduced levels of expression of a FOXP1 protein according to the invention comprising administering to an individual in need thereof, a therapeutic amount of a methylation inhibitor.

A further aspect of the invention also comprises a method of detecting or diagnosing cancer in an individual which is associated with increased levels of expression of a protein according to the invention, which method comprises testing in a cell of said individual for decreased levels of methylation of a regulatory region of a nucleic acid molecule encoding a protein according to the invention.

Similarly, a further aspect comprises a method of treating a disease or condition in a patient associated with overexpression of a FOXP1 protein according to the invention, which method comprises administering to an individual in need thereof a therapeutic amount of an antisense molecule according to the invention or a peptide from the FOXP1 protein according to the invention, or an antibody according to the invention.

Immunotherapy targeting cell surface molecules is a recognised technique currently in a variety of clinical trials. However peptide antigens derived from intracellular proteins can be presented on the cell surface in association with HLA molecules and recognised by cytotoxic lymphocytes (CTLs). Clinical trials with some of these molecules are underway reviewed by Cebon, J. MacGregor, D, Scott, A. and DeBoer, R. 1997. Australas J. Dermatol. 38; S66-72. Therefore, the FOXP1 peptides or antibodies according to the invention, may, advantageously be used in immunotherapy applications.

The invention will be further understood with reference to the following experimental Examples, together with the accompanying Figures in which:

Figure 1 (A). Western blotting of bacterially expressed proteins using monoclonal antibody JC12. "Vector" shows *E.coli* expressing β-galactosidase from the 'empty' vector pBK-CMV. Other lanes show *E.* coli expressing recombinant proteins from plasmids pAB195-200 respectively. All six recombinant proteins and their degradation products are recognised by the JC12 antibody when expressed in *E. coli.* pAB195 expresses the highest molecular weight protein which is recognised by the antibody when expressed in eukaryotic cells. Molecular weight standards are indicated to the left. (B). Antibody JC12 detects an 85kDa nuclear protein in Western blotted tonsil extracts.

Figure 2 shows the nucleotide sequence of the insert of pAB195 and the amino acid sequence of the human winged helix/zinc finger protein which is encoded by this sequence. Protein domains of interest are underlined and labelled. NLS refers to putative nuclear localisation signals and these sequences are underlined with dashes. Potential phosphorylation sites for protein kinase C (PKC), caesin kinase II (CK2) and cyclic AMP protein kinases (cAMP) are indicated with the amino acid recognition site in italics.

Figure 3A shows the nucleotide sequence of plasmid pAB195.

Figure 3B shows the nucleotide sequence of plasmid pAB196.

Figure 3C shows the corresponding amino acid sequence encoded by the nucleotide sequence of Figure 3B.

Figure 4A shows the nucleotide sequence of clone pAB199.

Figure 4B shows the corresponding amino acid sequence encoded by the nucleotide sequence of Figure 4A.

Figure 4C shows the nucleotide sequences of clone pAB200.

Figure 4D shows the corresponding amino acid sequence encoded by the nucleotide sequence of Figure 4C.

Figure 5 is an illustration of the results obtained from immunoperoxidase labelling of normal human tissues for the winged helix/zinc finger protein with antibody JC12. (A) In a routinely fixed tonsil section the nuclei and cytoplasm of some cells in the germinal centre (GC) are stained, but the surrounding mantle zone (MZ) is more strongly reactive and shows a predominantly nuclear pattern (seen more clearly in the higher power inset). (B) In a cryostat section of testis, spermatogonia show cytoplasmic labelling (bottom left of figure) while the spermatocytes show nuclear labelling and also a "capped" pattern, as shown in the high power inset.

Figure 6 is an illustration of the results obtained by immunoperoxidase labelling of human cell lines for the winged helix/zinc finger protein. (A) A Burkitt's lymphoma B-cell line (Namalwa) shows predominantly nuclear labelling, which becomes cytoplasmic in mitotic cells (arrows). (B) The rhabdomyosarcoma cell line Rh30 shows some cytoplasmic labelling together with punctate nuclear staining. Arrows indicate toroidal-shaped structures in the nuclei of some cells. (C) Heterogeneous labelling of both the nuclei and cytoplasm in the A431 carcinoma cell line.

Figure 7 is an illustration of the results obtained by immunocytochemical labelling of tumour cases for the winged helix/zinc finger protein. A case of chronic lymphocytic leukaemia with frozen (A) and routinely fixed (B) sections shows heterogeneous punctate labelling of the tumour cells and the absence of labelling in surrounding cells (arrows) in both. Strong nuclear labelling of the tumour cells with no labelling of surrounding cells is also seen in cases of follicular lymphoma (C); Burkitt's lymphoma, where the apoptotic cells are unstained (arrows) (D), and diffuse large B-cell lymphoma (E). In cases of diffuse large B-cell lymphoma of anaplastic morphology (F & G) .there is a considerable amount of cytoplasmic labelling, and in one case all the nuclear labelling is in focal structures, some of which are toroidal as shown in the high power inset (arrows) (G). The tumour cells in a T-cell lymphoma (H) also show a punctate nuclear staining pattern with the nucleoli remaining unstained (arrows). (I) A case of anaplastic large cell lymphoma (T cell phenotype) also shows toroidal structures in the cell nuclei (arrows). (J) A ductal carcinoma case showing both heterogeneous nuclear and cytoplasmic labelling of the tumour cells. (K) A basal cell carcinoma showing strong nuclear labelling of the tumour cells. (L) A squamous cell carcinoma showing some nuclear but predominantly cytoplasmic labelling seen most clearly in the high power inset.

Figure 8 is a schematic representation of the FOXP1 protein encoded by plasmid pAB195 (full length winged helix/zinc finger protein).

Figure 9 is a multiple alignment of FOXP1 in addition to splice variant proteins expressed by plasmids pAB196, pAB199 and pAB200.

Figure 10 is an illustration of the results obtained from Southern Blotting of *FOXP1.* 5μg of each genomic DNA sample was digested with *Eco*RI and Southern Blotting was performed using standard procedures (Sambrook, J., E. F. Fritsch and T. Maniatis, 1989.). The *FOXP1* probe was prepared by *Eco*RI digestion of the pAB195 plasmid and the 1.9kb fragment was gel purified and labelled with $^{32}P$ by random priming. The U2020 genomic DNA was a kind gift from Dr Pamela Rabbitts (Cambridge, U.K.). U refers to genomic DNA from cell line U2020, C refers to genomic DNA from CML patients and N refers to genomic DNA from normal individuals. An internal rennin control probe was used.

Figure 11 is an illustration of the results obtained from immunohistochemical staining of head and neck tumours with the JC12 monoclonal antibody.

Figure 12A is an illustration of the results obtained from CLONTECH Matched Tumor/Normal Expression Array probed with *FOXP1* cDNA. Tissue sources for cDNAs on the array are as follows: Normals row A /Tumours row B, kidney 1-14; Normals row D /Tumours row E, breast 1-9, prostate 11-13; Normals row G /Tumours row H, uterus 1-7, ovary 10-12, cervix 14; Normals row J /Tumours row K, colon 1-11, lung 13-15; Normals row M /Tumours row N, stomach 1-8, rectum 10-16, small intestine 18. Row P human cancer cell lines: 1 HeLa, 2 Daudi, 3 K562, 4 HL-60, 5 G361, 6 A549, 7 MOLT-4, 8 SW480, 9 Raji.

Figure 12B is an illustration of the results obtained from Clontech's MTE Normal Tissue Expression Array probed with FOXP1 cDNA.

Figure 13 is an illustration of the results obtained following transfection of FOXP1 into COS cells. Plasmids pAB195, pAB196, pAB199, pAB200 or the empty vector pBK-CMV were transfected into COS cells using DEAE dextran. Cytospins of transfected cells were prepared and immunostained with either JC12 or the $p27^{Kip1}$ antibodies as described in the legend for figure 14.

Figure 14 is an illustration of the results obtained from immunohistochemical staining of normal tonsil and kidney. Normal tonsil and kidney paraffin embedded sections were immunostained with either JC12, the DAKO MIB-1 monoclonal antibody diluted 1/50 or $p27^{Kip1}$ antibodies. Paraffin sections were dewaxed and then pressure cooked for 3 minutes in DAKO(R) Target Retrieval Buffer before staining. Staining was carried out using the DAKO Envision™ system. The peroxidase blocking solution from the kit was added to the sections for 5 minutes and the sections were then washed in TBS for 2 minutes. Primary antibody was added to the section (either JC12 diluted 1/80 in PBS + 10% normal human serum; DAKO $p27^{kip1}$ monoclonal antibody SX53G8 tissue culture supernatant, or Transduction Laboratories $p27^{kip1}$ monoclonal antibody K25020 dil 1/500) and incubated in a humid chamber at room temperature for 30 minutes. The sections were then washed in TBS for two minutes before incubation with Envision™ HRP for 30 minutes. The sections were washed in TBS for 5 minutes and incubated with the chromogenic substrate solution for 10 minutes. The sections were counterstained with haematoxylin (Sigma-Gill's No.3) and mounted in Aquamount (Merck/BDH).

Figure 15 is an illustration of results obtained from immunohistochemical staining of breast and lung tumours with antibody JC12. Paraffin embedded tumour sections were immunostained as described in the legend for figure 14. Each horizontal row labelled A-D represents an individual case stained with either JC12 or the $p27^{Kip1}$ antibodies as indicated on the figure.

Figure 16 is an illustration of results obtained from immunohistochemical staining of renal tumours and mouse spleen. For immunohistochemistry using a mouse antibody on mouse tissues the InnoGenexTM iso-IHC Fast Red kit was used according to the manufacturers instructions. The data is illustrated in the top right hand corner of the figure. The renal tumours were immunostained as described in the legend for figure 14. Each horizontal row labelled A-F represents an individual case stained with either JC12 or the $p27^{Kip1}$ antibodies as indicated on the figure.

Figure 17 is an illustration of the results obtained from experiments investigating the levels of expression of FOXP1 protein in pancreatic tumours.

Figure 18 is an illustration of results obtained from a study of FOXP1 expression in normal stomach and in stomach tumours. To detect the expression of the FOXP1 protein paraffin embedded sections were immunostained using the JC12 monoclonal antibody. A) Left: low power field (original magnification x40). Right: high power field (original magnification x200) with detail of the lower foveolar lining and upper glandular part): Representative normal fundic-type mucosa showing the strong cytoplasmic and weak to absent nuclear staining of the foveolar epithelial lining. The fundic glands have a weak to moderate cytoplasmic staining and a weak to absent nuclear expression (sample from the normal surgical margin of a case of early/intramucosal signet ring cell carcinoma ( as shown in Fig. 18L). **B**) High magnification (x200) of the foveolar lining. Note the moderate to strong nuclear staining of few small lymphocytes in the lamina propria (internal positive control). C) Left: low power field (x40). Right: high power field (x200) with detail of the antral glands: Expression of FOXP1 protein in normal antropyloric-type mucosa. The staining pattern of the foveolar lining is the same as in the previous case (A and B); the mucus secreting antral glands have a stronger cytoplasmic expression than the fundic-type glands. The nuclear expression is the same, e.g. weak to absent (sample from a resection for PUD). D) (original magnification x200): Base of foveolae with intestinal metaplasia showing no cytoplasmic staining and weak to moderate nuclear expression (normal surgical margin from an intestinal-type well-differentiated adenocarcinoma-Fig. 18F). **E)** (x200): Well-differentiated adenocarcinoma. Weak expression of FOXP1 protein in more than 50% of the nuclei, no cytoplasmic staining. Left upper corner: bases of preserved foveolae. F) Composite picture of different levels of nuclear FOXP1 protein expression in a case of well-differentiated adenocarcinoma, intestinal-type. Left: area of weak to absent nuclear staining. Right: moderate nuclear expression in more than 50% of the lining cells. G) Focal area of strong nuclear expression in a case of moderately differentiated intestinal-type adenocarcinoma (same case as in J). Weak cytoplasmic staining. H) Well-differentiated intestinal-type adenocarcinoma, weak nuclear and cytoplasmic expression. Right side: normal foveola (same case as E). **I**) Well-differentiated adenocarcinoma, intestinal-type, with strong nuclear expression and weak cytoplasmic staining. J) Moderately differentiated intestinal-type adenocarcinoma with weak and moderate nuclear expression in about 50% of the cells and no cytoplasmic staining. K) Mucinous well-differentiated adenocarcinoma with weak to moderate nuclear expression of FOXP1 protein in more than 50% of the cells. Weak cytoplasmic staining was observed in most of the cells and moderate intensity staining in a few. L) Intra-mucosal (early) signet-ring cell adenocarcinoma. Absent nuclear and cytoplasmic expression in the tumour cells. M) Diffuse-type (signet-ring-cell) adenocarcinoma. Weak nuclear expression in most of the malignant cells. No cytoplasmic staining.

Figure 19 is an illustration of the results obtained from JC12 immunohistochemical staining of DLBCL cases which had been sub-typed as having a germinal centre (GC, top row) or post-germinal centre (post-GC, bottom row) phenotype.

Figure 20 illustrates Kaplan-Meier Cumulative Survival Plots for both overall survival and disease free survival in DLBCL cases which do not express FOXP1 protein (0.000) or show strong FOXP1 expression (3.000).

Figure 21 is an illustration of results obtained from a study of FOXP1 expression in normal colon and colon tumours. **Panel A**) Left: low power field (original magnification x100). Right: high power field (original magnification x400) showing the staining pattern of the normal colonic mucosa (control case from patient without cancer). We observed weak to moderate nuclear staining of cells found in the basal part of the crypts and weak to absent nuclear expression in the cells from the upper region of the crypt near the surface epithelium. The cytoplasmic staining showed a reciprocal distribution, with weak to absent cytoplasmic staining of cells in the base of the crypts and a progressive increase towards strong cytoplasmic staining of cells towards the surface epithelium. Panel B) Nuclear dots (arrows) are seen in few cases within the nuclei of epithelial cells from normal crypts (original magnification x1000). Panel C) Normal mucosa (surgical margin of a well-differentiated adenocarcinoma with mucinous differentiation, same case as in panel I), showing the strong cytoplasmic staining of the superficial part of the crypts. Panel D) Strong cytoplasmic perinuclear staining (surgical margin from a case of well-differentiated adenocarcinoma, same case as shown in panel F). This pattern was infrequently seen. Panel E) Transition (arrows) from normal epithelium (left) to neoplastic epithelium (right) from a tubulovillous adenoma (with foci of in-situ carcinoma, not illustrated in the figure). In this case we observed a loss of cytoplasmic and increased nuclear staining of the neoplastic cells. Panel F) Well differentiated adenocarcinoma with no nuclear or cytoplasmic staining. Note the abrupt transition (arrows) between the normal epithelium with weak nuclear and cytoplasmic staining and the negative neoplastic cells. Panel G) Well differentiated adenocarcinoma (right) showing weak to moderate nuclear staining in a few cells and weak cytoplasmic staining. On the left side, are shown the base of non-neoplastic crypts with strong nuclear staining. Panel H) Composite picture of a well-differentiated adenocarcinoma with mucinous differentiation showing the heterogeneous nuclear expression of FOXP1 protein, from moderate nuclear JC12 staining in the better differentiated areas of the tumour (upper) to absent nuclear staining in the less differentiated parts (lower). The cytoplasm is not stained. **Panel I)** Moderately differentiated adenocarcinoma: strong nuclear expression with weak cytoplasmic staining. Panel J) Composite picture: heterogeneous nuclear and cytoplasmic expression of the FOXP1 protein in a well-differentiated adenocarcinoma with mucinous differentiation.

Left inset: at the top of the picture is a well-differentiated neoplastic mucosa with weak to moderate nuclear staining and no cytoplasmic staining. At the bottom there are ribbons of poorly differentiated neoplastic cells with strong cytoplasmic staining and no nuclear staining. Right inset: well-differentiated neoplastic gland with moderate nuclear staining.

Panel K) Poorly differentiated adenocarcinoma: no nuclear or cytoplasmic staining in the tumour cells. Left lower corner: base of non-neoplastic crypts showing weak nuclear staining.

Figure 22 is an illustration of JC12 immunostaining of cases of mantle cell lymphoma (MCL, top row) or the blastic variant of mantle cell lymphoma (MCL-blastic, bottom row) to detect the expression of the FOXP1 protein.

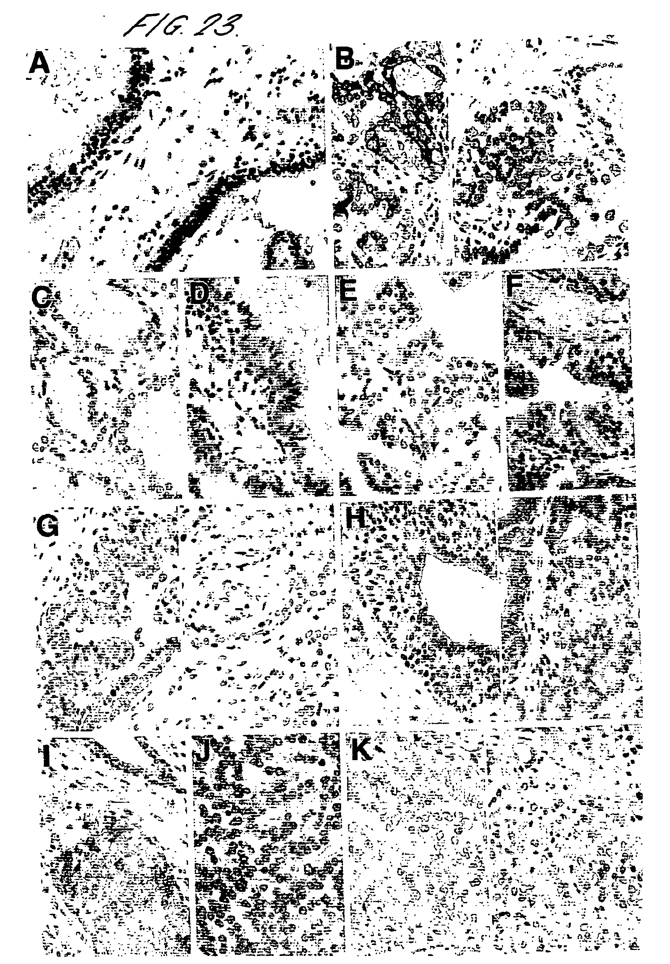

Figure 23 is an illustration of prostate and prostate tumours stained with the JC12 monoclonal antibody. A) Normal epithelium shows scattered cytoplasmic and nuclear staining of the upper epithelial cells which is generally not present in the lower myoepithelial layer. B) In some normal prostate (and other tissues) there are scattered cells which stain very strongly in the cytoplasm (seen here in left panel) and which have an unusual morphology. These may be for example either macrophages or modified epithelial cells. In the right panel is a prostate tumour from the same patient showing exclusively nuclear labelling of the tumour cells. C) Hyperplastic prostate tissue showing scattered nuclear positivity. D) Normal duct showing nuclear staining in the majority of the upper epithelium. E) Benign prostate showing scattered positive nuclei. F) Hyperplasia showing focal cytoplasmic staining and some nuclear positivity. G) Papillary hyperplasia showing moderate nuclear positivity and some focal cytoplasmic staining in the left panel. Poorly differentiated prostate tumour from the same patient showing nuclear labelling of the tumour cells (right-panel). H) The left hand panel shows cytoplasmic staining of the upper epithelium in a duct which has an in situ tumour underneath which is not immunostained. The right hand panel shows an area of tumour from the same patient which is less differentiated and which shows predominantly focal cytoplasmic staining. **I)** Prostate tumour with predominantly strong cytoplasmic staining of the tumour while only weak, if any, cytoplasmic staining is seen in the normal gland at the top of the panel. J) Prostate tumour which is negative for JC12 staining. K) Left hand panel shows large aggregation of tumour cells which are largely negative. The right hand panel shows cells from the same tumour which show positive nuclear staining.

Table 1: Reactivity of the JC12 monoclonal antibody on normal human tissues.

Table 2: Summary of the expression of the winged helix/zinc finger protein in human cell lines.

Table 3: Summary of the expression of the winged helix/zinc finger protein in human neoplastic cells.

<u>Example 1-cloning of a novel winged helix/zinc finger protein.</u>

Mouse monoclonal antibody JC12 was raised during a fusion intended to make antibodies against a peptide (NAAAESRKGQERFNC) from the Bclx gene coupled to PPD (purified protein derivative). The JC12 antibody did not recognise the Bclx immunogen or stain cells transfected with the Bclx cDNA confirming that this antibody recognised an unidentified antigen. The nuclear antigen recognised by this antibody was found to be widely expressed in both normal and neoplastic human tissues. The hybridoma secreting antibody JC12 has been deposited in accordance with the provisions of The Budapest Treaty of 1977 with the European Collection of Cell Cultures, Centre for Applied Microbiology & Research, Salisbury, Wiltshire, SP4 0JG, UK, on 14 April 1999 under accession No. 99041425.

The cDNA encoding the antigen recognised by the JC12 antibody was subsequently identified by expression cloning. 100 000 clones from both a circulating blood cDNA library and a testis cDNA library in lambda ZAP Express (Stratagene) were screened with monoclonal antibody JC12. 10 000 plaques per 15cm plate were grown on *Esherichia coli* strain XL1 Blue MRF' (Stratagene) and protein expression on filters was induced as described previously (Banham, A. H., Turley, H., Pulford, K., Gatter, K. and Mason, D. Y. (1997) J. Clin. Pathol. 50: 485-489.). Filters were rinsed in PBST (PBS + 0.05% Tween 20) for 5 minutes then washed in fresh PBST for 30 minutes before blocking in PBST + 5% Marvel at room temperature for 30 minutes. JC12 tissue culture supernatant was then added at a 1/1000 dilution at room temperature for 30 minutes. Filters were rinsed and then washed for 10 minutes with PBST. Filters were incubated for 30 minutes with a 1/750 dilution of goat anti-mouse peroxidase conjugated secondary antibody (DAKO) in PBST, before four 15 minute washes in PBST. Antigen-antibody complexes were visualised using diaminobenzidine/$H_2O_2$ with metal ion enhancement (Harlow, E. and Lane, D. (1988) Antibodies. A laboratory manual. Cold Spring Harbor: Cold Spring Harbor Laboratory Press). A second screen was performed to isolate individual positive clones.

The cDNAs encoding the JC12 antigen in plasmid pBK-CMV were excised *in vivo* from the lambda ZAP Express vector (Stratagene) using the manufacturer's instructions to yield the plasmids pAB195-200. Overall, six positive clones were isolated, two from the testis library (designated pAB195 and pAB199) and four from the blood library (designated pAB196-8 and pAB200).

To confirm that these cDNAs encoded proteins recognised by the JC12 antibody the proteins encoded by plasmids pAB195-200 were expressed in *E. coli.* The pBK-CMV vector contains a bacterial promoter enabling expression of the cloned cDNA in *E. coli* as an in-frame fusion with β-galactosidase. *E. coli* strain XLOLR (Stratagene) containing either plasmids pAB195-200 or the empty vector pBK-CMV was grown overnight in LB medium containing (100μg/ml) ampicillin at 37°C. Next day, cultures were diluted 1/10 into fresh medium and were allowed to grow for a further 1.5 hours. Protein expression was induced by the addition of 1mM IPTG. After 3 hours at 37°C 1ml samples were removed and the cell pellets were resuspended in 100μl of SDS-PAGE sample buffer (Laemmli, U. K. (1970) Nature 227: 680-685). Samples were boiled for 2 minutes and run on a 12% SDS-PAGE gel (Sambrook, J., Fritsch, E. F. and Maniatis, T. (1989) Molecular cloning. A laboratory manual. Cold Spring Harbor: Cold Spring Harbor Laboratory Press) alongside high molecular weight rainbow markers (Amersham).

The expressed protein was then Western blotted with antibody JC12. Briefly, proteins were transferred electrophoretically using a Semi-Phor (Hoeffer) apparatus to PVDF membrane (Millipore). The filter was then incubated with undiluted JC12 hybridoma (accession number 99041425) culture supernatant followed by secondary goat anti-mouse peroxidase conjugate as above. Antibody-antigen complexes were detected using an ECL kit (Amersham) following manufacturer's instructions.

The bacterial expression data illustrated in fig 1A show that the JC12 antibody did not recognize any *E. coli* proteins or the β-galactosidase expressed by the 'empty' vector pBK-CMV. However all six of the cloned cDNAs expressed proteins which were recognised by the JC12 antibody confirming their identity as positive clones. As a further measure to check the validity of the clones the plasmids were transfected into eukaryotic cells and stained for JC12 antigen expression using immunohistochemistry (data not shown). Briefly, plasmids pAB195-200 were transfected into the mouse fibroblast line WOP (kindly provided by Dr C. Basilico) using the DEAE dextran method (Seed, B. and Aruffo, A. (1987) Proc. Natl. Acad. Sci. U.S.A. 84: 2427-2445). Cells were used for transfection at 75% confluence and 5μg of plasmid DNA was used for each 25cm$^2$ flask. After 3 days in culture the cells were recovered by EDTA treatment for immunocytochemical staining. Only four of the clones, pAB195-6, pAB199 and pAB200, expressed a protein recognised by the JC12 antibody when they were eukaryotically expressed. All four eukaryotically expressed proteins recognised by the JC12 antibody were localised to the nucleus (although the protein encoded by pAB196 was also cytoplasmic). Proteins encoded by plasmids pAB197 and 198 were not recognised by the JC12 antibody when expressed and properly processed in eukaryotic cells and were not studied further. However, partial DNA sequencing of these plasmids demonstrated that they contained cDNAs encoding the SIG11 putative secreted protein (Accession No. AF 072733).

The cDNA insert in plasmid pAB195 was entirely sequenced in both directions using both overlapping restriction fragment sub-clones and internal oligonucleotides. DNA sequencing was performed using either M13 Universal and Reverse primers or internal oligonucleotides, a Cy5 Autoread sequencing kit and an ALF Express DNA sequencer (Pharmacia). This clone was selected as it contained the longest cDNA insert and expressed the largest protein of approximately 89kDa (Figure 1A) which is roughly comparable to the molecular weight of 85kDa determined for the JC12 antigen from a tonsil nuclear extract (Figure 1B). The remaining cDNAs were also subsequently fully sequenced in both directions so that their identity could be compared to that of the pAB195 clone.

All four plasmid clones contained sequences from the same cDNA confirming that this gene encodes the JC12 antigen. However none of the three smaller clones has complete homology to the pAB195 cDNA. Both pAB196 and pAB199 contained internal deletions (nt 545-772 and nt 679-942 of the pAB195 sequence illustrated in Figure 2 respectively) which may be the result of alternative splicing events. Certainly the 5' exon-intron sequence of pAB196 being AG/gt represents the consensus splice junction sequence and the 3' sequence of ag/CA is close to the consensus sequence ag/GC for the 3' exon-intron junction. The splice sites in the pAB199 clone are 5' TC/aa and 3' ag/AA which are less typical, however only 5% of donor sites have been reported to match the strict consensus (Smith, C. W. J., Patton, J. G. and Nadal-Ginard, B. (1989) Ann. Rev. Genet. 23: 527-577). The pAB200 cDNA contained a 3bp deletion corresponding to amino acid 450. Nucleotide sequences for the cDNA inserts of pAB196, pAB199 and pAB200 are shown in Figures 3B and 4A and 4C respectively.

A potential open reading frame of 677 amino acids, bordered by stop codons at both the 5' and 3' sides, was encoded by nucleotides 264-2294 of the pAB195 cDNA sequence, which is illustrated in Figure 2 with the amino acid sequence shown below. There are two potential in-frame methionine ATG initiation codons, the first has purines in both positions -3 and +4 and the second only has a purine at -3. However the second ATG has a better Kozak consensus (GCCA/GCCATGG) (Kozak, M. (1987) J. Mol. Biol. 196: 947-950) than the first because of the occurrence of A at position -3 and G at position -6 (Kozak, M. (1987) Nuc. Acids Res. 15: 8125-8148). The upstream non-coding region has a higher GC-rich content than the coding sequence which is frequently associated with the 5' end of genes. At least two other winged helix proteins, WIN (Yao, K.-M., Sha, M., Lu, Z. and Wong, G. G. (1997) J. Biol. Chem. 272: 19827-19836) and AF6q21 (Hillion, J., Le Coniat, M., Jonveaux, P., Berger, R. and Bernard, O. A. (1997) Blood 90: 3714-3719), also start with two ATG codons and both use the second, further supporting the hypothesis that translation starts at the second ATG. Interestingly upstream ATG codons occur in fewer than 10% of vertebrate mRNAs-at-large although a notable exception are oncogene transcripts, two-thirds of which have ATG codons preceding the start of the major open reading frame (Kozak, M. (1987) Nuc. Acids Res. 15: 8125-8148).

Analysis of the sequence of the protein encoded by the insert of pAB195 (hereinafter referred to as the winged helix/zinc finger protein) revealed the presence of a winged helix domain at amino acid positions 465-548. These amino acid coordinates, and all amino acid coordinates used herein to describe regions of the winged helix/zinc finger protein encoded by the nucleic acid sequence shown in Figure 2, refer to the amino acid sequence set forth in Figure 2 which corresponds to a translated protein product which initiates at the first (i.e. upstream) ATG codon. Amino acids 308-331 were found to be a perfect match for the $Cys_2$-$His_2$ zinc finger consensus which is Cx{2,4}Cx3(L,I,V,M,F,Y,W,C)x8Hx {3,5}H.

The sequence of the winged helix/zinc finger protein was further analysed using the PSORT II program to predict the presence of subcellular localization sites. This programme identified two potential nuclear localization signals (NLS) within the protein, the PIRRYS sequence at amino acid 434-440 and the KRRP sequence at amino acid 543-546 (Nakai, K. and Kanehisa, M. (1992) Genomics 14: 897-911). Although there is no definitive structural motif for an NLS (reviewed by Garcia-Bustos, J., Heitman, J. and Hall, M. N. (1991) Biochim. Biophys. Acta 1071: 83-101; Jans, D. A. and Hübner, S. (1996) Physiol. Rev. 76: 651-685), they are typically short sequences, usually rich in lysine and arginine residues, and often contain proline. The JC12 antibody staining which shows that the antigen recognised by JC12 is present in the nucleus supports the prediction that the winged helix/zinc finger protein is a nuclear protein. The KRRP sequence occurs at the end of the second wing of the fork head domain in a region which has been shown to encode an NLS in other family members eg HNF-3 (Qian, X. and Costa, R. H. (1995) Nuc. Acids Res. 23: 1184-1191). Although the second NLS in many other winged helix genes is found in helix one of the fork head domain (Qian, X. and Costa, R. H. (1995) Nuc. Acids Res. 23: 1184-1191) this region is not conserved in the novel winged helix/zinc finger protein and the NLS is predicted to occur upstream of this domain as is predicted for the FKHL15 winged helix gene (Chadwick, B. P., Obermayr, F. and Frischauf, A.-M. (1997) Genomics 41: 390-396).

The winged helix/zinc finger protein also contains regions which are found in many transcriptional activators involved in gene expression. These activation domains can be grouped into several categories based on their amino acids content, including glutamine, acidic, proline, serine and threonine rich domains. These domains have been shown to function by interaction with the basal transcription machinery by both *in vitro* and *in vivo* assays (Truant, R., Xiao, H., Ingles, C. J. and Greenblatt, J. (1993) J. Biol. Chem. 268: 2284-2287; Xiao, H., Pearson, A., Coulombe, B., Truant, R., Zhang, S., Regier, J. L., Triezenberg, S. J., Reinberg, D., Flores, O. and Ingles, C. J. (1994) Mol. Cell. Biol. 14: 7013-7024) . The N-terminus of the winged helix/zinc finger protein contains two glutamine rich domains between aa 55-77 and aa 110-194, containing 65% and 49% glutamine residues respectively, and a 52% serine/threonine rich region between aa 244-268. While the C-terminus contains two serine/threonine/proline rich regions aa 387-431 and aa 613-626 containing 60% and 57% S/T/P residues respectively and an acidic domain at the extreme C-terminus aa 637-677 containing 39% aspartate and glutamate residues. Significantly both the glutamine rich regions contain adjacent bulky hydrophobic groups which have been shown in other proteins eg Sp1 (Gill, G., Pascal, E., Tseng, Z. H. and Tjian, R. (1994) Proc. Natl. Adad. Sci. U.S.A. 91: 192-196) and Oct-2 (Tanaka, M. and Herr, W. (1994) Mol. Cell. Biol. 14: 6056-6067) to be important for the function of these transactivation domains. The winged helix/zinc finger protein also contains two potential PEST sequences in its C-terminus (aa 612-623 and 636-656) as predicted by the PEST Find Program developed by Rechsteiner, M.C., and Rogers, S.W. PEST sequences and regulation by proteolysis. Trends Biochem. Sci., 1996. 21: P267-271. These regions are rich in proline, glutamate, serine and threonine residues, they range in length from 12-60 amino acids and are often flanked by charged residues. Functionally they are known to mediate rapid protein degradation of enzymes, transcription factors and components of receptor signalling pathways for example, they mediate protein degradation in immediate-early-response AP-1 transcription factors (Rechsteiner, M. and Rogers, S. W. (1996) Trends Biochem. Sci. 21: 267-271). An overlap between acidic activation domains and destruction elements has been described in other unstable transcription factors destroyed by ubiquitin-mediated proteolysis. (Salgetti, S.E., Muratani, M., W. Jnen H., Futcher, B., Tansey, W.P., 2000 PNAS 97 3118-23). Thus, inhibition of the proteosome could be used to upregulate FOXP1 levels.

Another interesting feature of the winged helix/zinc finger protein is the prediction that there are two regions between aa 124-155 and aa 344-369 which have the potential to form coiled coils (Lupas, A., Van Dyke, M. and Stock, J. (1991) Science 252: 1162-1164). These motifs are important in a wide range of transcription factors (and other proteins) where they mediate protein-protein interactions and act as dimerization motifs (reviewed in Baxevanis, A. D. and Vinson, C. R. (1993) Curr. Opin. Genet. Dev. 3: 278-285). The coiled coil motifs identified in this winged helix/zinc finger protein are not a characteristic of the winged helix family in general as our analysis of a number of other winged helix proteins (HNF-3$\alpha$, Genesis, FKHRLI, FREAC-1 and HFH-4) did not predict the presence of these motifs.

Analysis of the FOXP1 sequence also links this protein to the cell cycle. The present inventors have identified two potential cdk phosphorylation sites within the FOXP1 protein (starting at aa 83 and 481 respectively). These consist of a serine/threonineproline (S/T-P) phosphoacceptor site and a preference for a basic residue at position +3 (where S/T is position 0 Zhang, J., R.J. Sanchez, S. Wang, C. Guarnaccia, A. Tossi, S. Zahariev, and S. Pongor. Biophys., 1994. 315: p. 415-424; Srinivasan, J., M. Koszelak, M. Mendelow, Y.-G. Kwon, and D.S. Lawrence. Biochem. J., 1995.

309: p. 927-931). Physical association with the cdk kinase may also play a role in establishing substrate specificity and cyclin-cdk2 complexes bind stably to a number of cell cycle regulatory proteins. The ZRXL sequence (where Z and X are typically basic) has been identified as the cyclin-cdk2 binding motif in a number of these proteins including E2F1, p107 and p21 (Zhu, L., E. Harlow, and B.D.. Genes Dev., 1995. 9: p. 1740-1752; Adams, P.D., W.R. Sellers, S.K. Sharma, A.D. Wu, C.M. Nalin, and W.G. Kaelin Jr. Mol. Cell. Biol., 1996. 16: p. 6623-6633; Schulman, B., D. Lindstrom, and E. Harlow. Proc. Natl. Acad. Sci. USA, 1998. 95: p. 10453-10458). In both p45/Skp2 and pRB the sequence KXL is used instead of RXL (Lisztwan, J., A. Marti, H. Sutterluty, M. Gstaiger, C. Wirbelauer, and W. EMBO J., 1998. 17: p. 368-383; Adams, P.D., X. Li, W.R. Sellers, K.B. Baker, X. Leng, J.W. Harper, Y. Taya, and W.G. Kaelin Jr. Mol. Cell. Biol., 1999. 19: p. 1068-1080.). The inventors have identified the presence of both potential RXL and KXL motifs within the FOXP1 protein sequence starting at aa 66, 325 and 484 respectively. These data suggest a previously unreported mechanism by which winged helix transcription factors may have a role linked to the cell cycle.

As mentioned previously, sequencing the additional cDNAs isolated during the expression cloning using JC12 identified two variant cDNAs (pAB196 and pAB199) which may possibly be the result of alternative splicing. These variants would encode proteins with in-frame deletions in the N-terminus of the protein.

A number of proteins with different biological activities are commonly generated from a single gene by alternative splicing and splice variants for a number of other winged helix genes have also been identified (Cockell, M., D. Stolarczyk, S. Frutiger, G.J. Hughes, O. Hagenbüchle, and Wellauer P.K. Mol. Cell. Biol., 1995. 15: p. 1933-1941; Ye, H., T.F. Kelly, U. Samadani, L. Lim, S. Rubio, D.G. Overdier, K.A. Roebuck, and R.H. Costa. Mol. Cell. Biol., 1997. 17: p. 1626-1641; Yang, Q., R. Bassel-Duby, and R.S. Mol. Cell. Biol., 1997. 17: p. 5236-5243).

Both variants lack the N-terminal coiled-coil and a large proportion of all of the second glutamine rich domain. These regions may be functionally important for mediating either protein-protein interactions or transcriptional activation respectively, thus it is possible that these deletion variants are functionally different to the full length protein. When transfected into COS cells the FOXP1 protein expressed by plasmid pAB196 has a largely cytoplasmic localisation (Figure 13). This splice variant provides one potential mechanism for cytoplasmic expression of the FOXP1 protein. Preliminary experiments investigating the transcriptional activity of different regions of the FOXP1 protein suggest that the N-terminal glutamine rich domain may be functionally able to repress transcription. The following plasmids were constructed to fuse portions of the FOXP1 gene to the yeast GAL4 DNA binding domain. pAB373; 482 bp *Pvu*II-*Sma*I fragment from pAB195 encoding aa 50-210 was cloned into vector p13H cut with *Sma*I. pAB374; the 500bp EcoRI-XhoI fragment from pAB195 encoding aa540-677 was cloned into vector p13H cut with *Eco*RI and *Sal*I. pAB375 ; the 1.99kb PvuII-XhoI fragment encoding aa 50-677 was cloned into the vector p13H cut with *Sma*I and *Sal*I. These plasmids and the p13H vector were then transformed into *Saccharomyces cerevisiae* strains GGY1:171, SS19-8 or SS38-G4 and the ability of the expressed GAL4-FOXP1 fusion proteins to either activate or repress transcription of the β-galactosidase reporter gene in these strains was assessed by streaking the transformants on X-Gal plates. The theory behind this technique the methodology, the p13H vector and yeast cell lines are described in Asante-Owusu et al. 1996. Gene 172: 25-31. The results of the crude plate assay indicated that none of the plasmids activated β-galactosidase expression when transformed into the GGY1:171 cell line while plasmid pAB373 did appear to partially repress the constitutive expression of β-galactosidase when transformed into strains SS19-8 or SS38-G4 as indicated by a lighter blue colour on X-Gal plates. These data are preliminary and need to be confirmed by an accurate enzymic assay of β-galactosidase activity in the transformed cells. However, our data do suggest that the N-terminal region of the FOXP1 protein between aa 50-210 may contain a functional domain that is able to repress transcription. The FOXP1 variant proteins encoded by pAB196 and pAB199 lack portions of this region which may alter or prevent the ability of these proteins to repress the transcription of FOXP1 target genes. The C-terminus of the pAB200 protein has amino acid 450 of the FOXP1 protein deleted. This amino acid is part of a consensus site for CK2 phosphorylation within the protein and it is possible that this deletion may alter the behaviour of this form of the protein in response to protein kinase regulation compared to those which retain this phosphorylation site. It is thus possible that all these proteins (if translated *in vivo)* differ in their function(s) from the full length protein.

## Example 2-Expression of the winged helix/zinc finger protein in normal tissues, human cell lines and neoplastic tissues.

### (1) Preparation of tissues and cell lines.

Tissues obtained from the Histopathology Department at the John Radcliffe Hospital were immediately snap frozen in liquid nitrogen and stored at -70°C. Human cell lines were obtained from either the Sir William Dunn School of Pathology, Oxford or the American Type Culture Collection (ATCC, Rockville, MD), while the JOK-1 cell line was a kind gift from Dr Leif Andersson. Cells were cultured in RPMI 1640 medium containing 10% fetal calf serum (GIBCO Biocult Ltd) at 37°C in 5% $CO_2$.

Cryostat tissue sections (5-8 μm) on glass multiwell slides were dried overnight at room temperature, fixed in acetone for 10 minutes at room temperature and then stored, wrapped in aluminium foil, at -20°C before use. Formalin fixed, paraffin wax embedded sections were cut at approximately 5 μm and collected on superfrost plus glass slides (Speci-microsystems). Cytocentrifuge preparations of cell lines were prepared as previously described (Erber, W. N., A. J. Pinching, and D. Y. Mason. 1984. Lancet 1: 1042-1046) and then stored wrapped in aluminium foil, at -20°C before use.

**(2) Two step immunoperoxidase staining of cell lines.**

Cytocentrifuge cell preparations were fixed in acetone for 10 minutes at room temperature, and air dried before incubation with the primary antibody for 30 minutes. The slides were then washed in PBS, incubated with horseradish peroxidase-conjugated goat anti-mouse secondary antibody (DAKO, Copenhagen, Denmark), washed again and then developed using diaminobenzidine/$H_2O_2$. Slides were counterstained with haematoxylin and mounted in Aquamount (Merk Ltd, Poole, U.K.).

**(3) Immunostaining of tissue sections.**

Staining was carried out using the DAKO Envision™ system. The peroxidase blocking solution from the kit was added to the sections for 5 minutes and the sections were then washed in TBS or PBS for 2 minutes, but these steps were omitted when labelling cryostat sections. Antibody JC12, diluted 1/80 in PBS, was added to the section and incubated in a humid chamber at room temperature for 30 minutes. The sections were then washed in TBS or PBS for two minutes before incubation with Envision™ HRP for 30 minutes. The sections were washed in TBS for 5 minutes and incubated with the chromogenic substrate solution for 10 minutes. The sections were counterstained with haematoxylin (Sigma- Gill's No.3) and mounted in Aquamount (Merck/BDH).

Results.

**Expression of the winged helix/zinc finger protein in normal tissues.**

The distribution of the winged helix/zinc finger protein was analysed by immunocytochemical labelling of frozen and paraffin embedded tissues using monoclonal antibody JC12. The protein was found to be widely expressed, with a predominantly nuclear distribution, in normal tissues including tonsil (Fig 5A), spleen, blood, thymus, testis, kidney, liver, large bowel, cerebellum, skin and ovary. However cytoplasmic labelling was also seen in some cells, particularly in epithelial tissues, lung macrophages and in spermatogonia in the testis (Fig. 5B). In the latter tissue type the spermatocyte nuclei also showed an unusual "capped" immunostaining pattern for winged helix/zinc finger protein (Fig. 5B).

**Expression of the winged/helix/zinc finger protein in human cell lines.**

The protein was found in all the cell lines tested (Table 1), in keeping with its broad distribution in normal tissues. The intracellular localisation of the winged helix/zinc finger protein was predominantly nuclear in haematopoietic cell lines (Fig. 6A) and in some cell lines, for example the rhabdomyosarcoma cell line Rh30 (Fig. 6B), this immunostaining identified intranuclear structures, some of which were ring-like or toroidal. Cytoplasmic staining, which was generally weaker, was also observed in most cell lines. In epithelial cell lines, such as A431 (Fig. 6C), strong staining of both the nucleus and cytoplasm was observed, which varied in intensity from cell to cell. Cytoplasmic labelling in these cells (Fig. 6C) was considerably stronger than in the haematopoietic cell lines.

**Expression of the winged helix/zinc finger protein in neoplastic tissues.**

The winged helix/zinc finger protein was expressed by almost all haematopoietic malignancies (Table 2), although both the intensity and the degree of cell-to-cell heterogeneity of expression varied from case to case. However, the tumour cells in only two of five cases of lymphocyte predominance Hodgkin's disease were immunostained for the winged helix/zinc finger protein (and nuclear expression of the protein in the surrounding reactive lymphocytes confirmed that this was not a false negative reaction).

In both frozen (Fig. 7A) and routinely fixed (Fig. 7B) sections of most tumours (particularly those with strong nuclear expression of the winged helix/zinc finger protein) surrounding reactive lymphoid cells were almost negative for the winged helix/zinc finger protein, and stained more weakly than these cells in normal tissues. Strong nuclear expression of the winged helix/zinc finger protein by the malignant cells is seen in a number of other B-cell tumours including follicular lymphoma (Fig. 7C), Burkitt's lymphoma (Fig. 7D) and diffuse large B-cell lymphoma (Fig 7E).

The staining of three diffuse large B cell lymphomas of anaplastic morphology was more cytoplasmic (Fig. 7F & G) than observed in other cases of diffuse large B-cell lymphomas, in which staining was almost exclusively nuclear (Fig. 7E). In one of these three cases (Fig. 7G), nuclear staining was exclusively focal and the toroidal structures seen in some cell lines and in some T cell ALCLs (Fig. 7I) were observed. The winged helix/zinc finger protein was also strongly expressed by other T-cell lymphomas (Fig. 7H) where both cytoplasmic and nuclear labelling could be seen.

**Genomic Map Location of the FOXP1 Gene**

The TIGR gene index contained a theoretical clone THC300432 which corresponded to the 3' end of the *FOXP1* cDNA clone pAB195 (nucleotides 1023-2338 of the sequence in Figure 2). The opposite end theoretical clone THC353343 did not align with any of the *FOXP1* cDNA clones. The expressed sequence tags (ESTs) which make up clone THC300432 originally formed UniGene cluster Hs.7891, ESTs, Weakly similar to *JM2* [*H. sapiens*], in the NCBI database. A number of the cDNAs in the cluster contained a mapped sequence-tagged site (STS) sts-W89007 which mapped them to chromosome 3 in the interval between D3S1261-D3S1604 on the Gene Map 98.

These EST sequences were then moved into Unigene cluster Hs.8997 for Heat Shock 70kD protein 1 although the mRNA sequence for this gene does not correspond to that for FOXP1. Subsequently yet another Unigene Cluster (Hs.274344) has been created for the FOXP1 ESTs called LOC51245. The "full length" protein sequence is reported (NM_016477) but this starts at aa 559 of the FOXP1 sequence which we have isolated, is only 118 aa long and lacks the winged helix domain.

Alignment of several EST sequences containing sts-W89007 to the *FOXP1* cDNA confirms that they are the same. The *FOXP1* C-terminus therefore contains the mapped sequence tag sts-W89007 mapping this gene to between markers D3S1261-D3S1604 which approximates to chromosome bands 3p12.3-3p14.1 (Todd, S., W. A. Franklin, M. Varella-Garcia, T. Kennedy, C. E. Hilliker Jr., L. Hahner, M. Anderson, J. S. Wiest, H. A. Drabkin and R. M. Gemmill, 1997. Cancer Res. 57:1344-1352).

**An unidentified tumour suppressor gene(s) localises to the short arm of chromosome 3.**

Knowledge of chromosomal deletions has made a significant contribution to the detection of tumour suppressor genes. According to the two-hit hypothesis the inactivation of one allele often results from a deletion on the chromosome level; while the other copy may be inactivated by point mutation, methylation changes or small deletions (Knudson, A. G., 1971. PNAS 68:820-823). The CCAP (Cancer chromosome aberration project) breakpoint map of recurrent chromosome aberrations has identified the short arm of chromosome 3 as a region found to have been deleted in a number of types of cancer (Mitelman, F., F. Mertens and B. Johansson, 1997. Nat. Genet. 15:417-474). Deletions at chromosome 3 have been reported to be the third most common of all known deletions in human tumours (Sezinger, B. R. et al., 1991. Cytogenet. Cell. Genet. 58:1080-1096).

The two most commonly used approaches to determine the location of a tumour suppressor gene are karyotying and analysis of loss of heterozygosity (LOH) of mapped polymorphic markers while the basic criteria which define tumour suppressor genes are:-

1) the presence of loss of function mutations
2) inactivation in both familial and sporadic tumours
3) the fact that the tumour phenotype can be rescued by the wild allele.

Hemizygosity and homozygosity mapping studies show that many common sporadic cancers including lung (Brauch, H., et al. 1990. Genes Chrm. Cancer. 1:240-246), breast (Devillee, P., et al., 1989. Genomics 5:554-560), kidney (Foster, K., et al., 1994. Br. J. Cancer 69:230-234), cervical (Jones, M. H. and Y. Nakamura, 1992. 7:1631-1634; Yokata, J., et al., 1989. Cancer Res. 49:3598-3601), pancreatic (Gorunova, L., et al., 1998. Genes Chrm. Cancer 23: 81-99), ovarian (Sato, T., et al., 1991. Cancer Res. 51:5118-5122), and head and neck cancer (Maestro, R., et al., 1993. Cancer Res. 53:5775-5779) display deletions on the short arm of chromosome 3. Studies using cytogenetic and loss of heterozygosity (LOH) techniques have identified four regions at 3p which may contain tumour suppressor genes. They involve chromosome 3 bands p12, p14.2, p21.3 and p25 (reviewed by (Kok, K., S. L. Naylor and C. H. Buys, 1997. Cancer Res. 71:27-92; Le Beau, M. M., et al., 1998. Genes Chrm. Cancer 21:281-289)).

The von Hippel-Lindau (VHL) tumour suppressor gene locates to 3p25-p26 and mutations in this gene define a familial cancer syndrome with susceptibility to the development of several neoplasms, such as renal cell carcinoma (Béroud, C., et al., 1998. NAR 26:256-258). *VHL* mutations are particularly significant in sporadic renal cell carcinomas with 30-60% displaying homozygous gene loss (reviewed by (Decker, H. J., E. J. Weidt and J. Brieger, 1997. Cancer Genet. Cytogenet 93:74-83)). The DNA repair gene *XPC* is also located at 3p25. A region homozygously deleted in a breast cancer case maps to 3p21.3 (Sekido, Y., et al., 1998. Oncogene 16:3151-3157) and the DNA mismatch repair gene *MLH1* resides at 3p21.3-p23. The *FHIT* gene spans the fragile site FRA3B at 3p14.2 and is also a candidate tumour suppressor gene (Le Beau, M. M., H. Drabkin, T. W. Glover, R. Gemmill, F. V. Rassool, T. W. McKeithan and D. I. Smith, 1998. Genes Chrm. Cancer 21:281-289).

Homozygous deletions are relatively rare and are thought to result from second allelic deletion of a chromosomal region that has already undergone hemizygous loss. They have, however, been central to the precise localisation of tumour suppressor genes such as CDKN2 (9p21), PTEN or MMAC1 (10q23.3), and DPC4 (18q21.1) (Cairns, P., et al., 1995. Nat. Genet. 11:210-212; Hahn, S. A., et al., 1997. Science 15:356-362). Several homozygous deletions have been reported in the 3p12-p14 region. In cell lines a homozygous deletion in this region has been reported in a small cell lung cancer line (U2020) (Rabbitts, P., et al., 1990. Genes Chrm. Cancer 2:231-238), a non-small-cell lung cancer line NCI-H2195 (Virmani, A. et al., 1998. Genes Chrm. Cancer 21:308-319) and the HCC38 breast primary ductal carcinoma cell line (Gazdar, A. F., et al., 1998. Cold Spring Harb. Symp. Quant. Biol. 78:766-774). This observation is not restricted to cell lines as homozygous deletions within 3p12-p14 have also been reported in an uncultured small cell lung tumour sample (Todd, S., et al., 1997. Cancer Res. 57:1344-1352), a cervical carcinoma sample (Aburent, H., Y. Wang, Y. Shibata, T. Noda, D. Schwartz and H. D., 1994. Am. J. Hum. Genet. 55 Suppl.:269) a short term culture isolated from apparently normal bronchial epithelium in a lung tumour-bearing patient (Sundaresan, V. et al., 1995. Ann. Oncol. 6 (Suppl. 1):S27-S32), short term cultures from several breast carcinomas (Pandis, N., et al., 1993. Genes Chrm. Cancer 6:151-155) and a case of breast cancer (Chen, L. et al., 1994. Cancer Res. 54:3021-3024). Interestingly although the lung tumour case contained a homozygous deletion within 3p12 there was considerable heterogeneity within the tumour with a subpopulation of cells only showing LOH at this region (Todd, S., et al., 1997. Cancer Res. 57:1344-1352).

***FOXP1* does not reside on the region of chromosome 3p which is homozygously deleted in the U2020 cell line.**

To investigate whether the FOXP1 gene localised to the region of DNA homozygously deleted in the U2020 lung cancer cell line we performed Southern Blotting using genomic DNA from the U2020 cell line and control genomic DNA samples from two patients with CML and from two normal individuals. All five samples showed the same banding pattern when probed with the *FOXP1* cDNA and a rennin control probe (Figure 10) confirming that this gene was not deleted in the U2020 cell line. The deletions within the cell lines NCIH-2195 and HCC38 are contained within this region and therefore none of these homozygous deletions involve the *FOXP1* gene. This information further defines the chromosome localisation for *FOXP1* as occurring between markers D3S2583 and D3S1261. We have also detected very low levels of FOXP1 protein in the U2020 cell line by immunohistochemistry confirming its expression.

**Allelic Loss at 3p in not a common event in haematological malignancies.**

The initial immunohistochemical study described above showed that in haematological malignancies, some nuclear staining with JC12 was always observed, the possible exception being lymphocyte predominance Hodgkin's disease. In a significant number of cases overexpression of the FOXP1 protein was actually observed. A survey of 3p deletions in haematological malignancies concluded that these were not a common feature and it was concluded that many were secondary aberrations and were more distal (3p25-3p26) than those seen in solid tumours (Johansson, B., et al., 1997. 11:1207-1213). This was consistent with the results of the preliminary haematological immunohistochemistry study which did not implicate loss of FOXP1 protein expression in the majority of these neoplasms. However, further studies have also identified loss of FOXP1 protein expression in cases of both diffuse large B-cell lymphoma and mantle cell lymphoma, indicating that this phenomenon is more widespread.

**Expression of the FOXP1 protein in tumours.**

The present inventors further investigated the expression of FOXP1 protein in non-haematological malignancies by immunohistochemistry with the JC12 antibody. The results are significantly different to the expression pattern of the protein in haematological malignancies. A decreased level of FOXP1 protein in the nucleus was frequently observed. A significant number of cases were also identified where the FOXP1 protein is either present only in the cytoplasm or where the tumour cells lack FOXP1 expression completely.

**Head and Neck carcinomas**

LOH studies of head and neck squamous cell carcinomas (HNSCC) have identified loss at chromosome 3p in up to 70-75% of HNSCC cases (Maestro, R., et al., 1993. Cancer Res. 53:5775-5779). Loss at chromosome 3p12-3p21 was high in cell lines derived from HNSCCs (Buchhagen, D. L., et al., 1996. Head Neck 18:529-537) and a study using both LOH and microsatellite instability identified LOH at 3p12 in 61.5% of cases of oral and oropharyngeal epithelial carcinomas (Grati, F. R., et al., 2000. Cancer Genet. Cytogenet. 118:57-61). By examining the tumourigenicity of microcell hybrid clones, containing fragments derived from 3p, the proximal 3p13 region was functionally shown to suppress the tumour phenotype in SCC tumourigenic cell lines (Uzawa, N., et al., 1998. Cancer Genet. Cytogenet. 107:125-131).

LOH at 3p has been linked to a poor prognosis in head and neck carcinomas (Li, X., et al., 1994. J. Natl. Cancer Inst. 6:1524-1529). A more recent study of 48 primary oral squamous cell carcinomas (SCC) showed that patients with LOH at a number of loci, including 3p13, had a 25 times higher mortality rate than a patient without these losses (Partridge, M., et al., 1999. Br. J. Cancer 79:1821-1827).

The immunohistochemical data shows that only 3/10 cases of head and neck carcinomas expressed normal levels of nuclear FOXP1 protein; 1/10 cases shows loss of protein expression, 4/10 cases show only weak nuclear expression and 2/10 cases show cytoplasmic localisation of the protein. This preliminary study demonstrated aberrant FOXP1 expression in 70% of cases. Both of the well differentiated SCC cases showed only very weak nuclear expression in some tumour cells, this is consistent with a study showing that well differentiated head and neck carcinomas were defined by the deletion of 3p (also 5q and 9p) suggesting that this deletion was associated with early tumour development (Bockmühl, U., et al., 1998. Head & Neck 20:145-151).

Examples of the expression pattern of FOXP1 protein in head and neck cancers are shown in Figure 11. One poorly differentiated SCC shows nuclear expression of the FOXP1 protein (A) while another shows only cytoplasmic expression (D). Cytoplasmic expression in also seen in another SCC case (C) and some tumours seem to show both nuclear and cytoplasmic expression of the FOXP1 protein (B).

## Renal carcinomas

LOH studies have identified 3p as an important region frequently lost in renal tumours. While many of these deletions have been shown to involve the VHL gene, the *FHIT* gene and the familial renal cell carcinoma (RCC) breakpoint there is convincing evidence for the involvement of yet another unidentified tumour suppressor gene at 3p12-3p14 in some non-papillary renal cell carcinomas (Bugert, P., et al., 1996. Int. J. Cancer 68:723-726; Chino, K., et al., 1999. J. Urol. 162:614-618; Lovell, M., et al., 1999. Cancer Res. 59:2182-2189; Lubinski, J., et al., 1994. Cancer Res. 54: 3710-3713; Willers, C. et al., 1996. Br. J. Urol. 77:524-529). The *FHIT* gene and the FRA3B site have now been excluded from the genetics of renal cell carcinoma (Bugert, P. et al., 1997. Genes Chrm. Cancer 20:9-15).

Although karyotyping studies have suggested that, unlike non-papillary RCC tumours, papillary RCC tumours usually lack 3p deletions (Hughson, M. et al., 1993. Cancer Genet. Cytogenet. 62:89-124) the use of molecular genetic techniques has identified LOH at three different 3p loci (Hazaczek, P., et al., 1996. Virchows Arch. 429:37-42; Mehle, C., et al., 1998. Int. J. Oncol. 13:289-295; Presti Jr., J. C. et al., 1993. Cancer Res. 53:5780-5783). Using this approach there was no obvious difference found in the frequency of any 3p deletion between non-papillary and papillary tumours (Mehle, C., et al., 1998. Int. J. Oncol. 13:289-295). It has been suggested that the LOH at 3p12-14 has been previously underestimated in papillary RCCs presumably as the deletions are often too small to be detected by karyotypic means (Mehle, C., et al., 1998. Int. J. Oncol. 13:289-295). Chromophobe tumours, however, have a lower frequency of 3p deletions e.g. 4-5% (Kovacs, G., 1993. Histpath 22:1-8).

Studies on a novel genetic locus *NRC-1* (non papillary renal cell carcinoma 1) have identified a tumour suppressor locus at 3p12 in a region which excludes the *FHIT* gene, FRA3B and the familial RCC breakpoint region (Lott, S. T., et al., 1998. Cancer Res. 58:3533-3537). This locus controls the growth of RCC cells by inducing rapid cell death *in vivo* (Sanchez, Y., et al., 1994. PNAS 91:3383-3387). Further studies show that *NRC-1* is involved not only in different cell types of RCC but also in papillary RCC. This report claims to demonstrate the first functional evidence for a *VHL*-independent pathway to tumourigenesis in the kidney (Lovell, M., et al., 1999. Cancer Res. 59:2182-2189).

Our immunohistochemistry data are consistent with the results of these recent studies: 3/10 cases of non-papillary RCC showed a reduction or loss of nuclear FOXP1 protein, 1/1 case of papillary RCC showed cytoplasmic FOXP1 protein expression while 2/3 cases of chromophobe showed reduced or loss of nuclear expression for FOXP1.

Additional information on the VHL status of non-papillary RCCs may also be significant, as 3/4 cases of RCC which were either known to have mutations in the VHL gene or to have VHL disease showed normal nuclear positivity for FOXP1.

## Lung cancer

Lung cancer is the leading cause of cancer deaths in the US (Ginsberg, R. J., et al., 1993. Cancer: Principles & Practise of Oncol. 673-723) and typically has very poor prognosis. Deletions of chromosome 3p have been shown to occur at a high frequency in all forms of lung cancer (Gazdar, A., et al., 1994. Cold Spring Harb. Symp. Quant. Biol. 59: 565-572). LOH studies have defined four non overlapping minimal deletion regions (OCLOHRs) in lung tumours. OCLOHR-4 is mapped between D3S1284 and D3S1274 at 3p12-13 (Fullwood, P., et al., 1999. Cancer Res. 59: 4662-4667). Allelotyping studies of 215 lung cancer cell lines have shown that the 3p chromosome region near the D3S3 locus (3p12-p13) appears to be involved in all forms of lung cancer (Buchhagen, D. L., 1996. J. Cell Biochem. Suppl. 24:198-209). There has been some debate about the relative frequencies of 3p LOH when comparing small cell lung carcinomas (SCLC) and non-small cell lung carcinomas (NSCLC) (Brauch, H., et al., 1987. N. Engl. J. Med. 317: 1109-1113; Brauch, H., et al., 1990. Genes Chrm. Cancer 1:240-246; Hibi, K., et al., 1992. Oncogene 7:445-449) and LOH at 3p12 has been reported as having a significantly higher frequency in SCLC (Virmani, A. K., et al., 1998. Genes Chrm. Cancer 21:308-319).

Our immunohistochemical staining for FOXP1 protein showed that none of the six NSCLC cases studied appeared to express this protein in the nucleus, 4/6 cases showed no FOXP1 expression in the tumour cells, while the remaining 2/6 cases showed FOXP1 protein being present in the cytoplasm. These data support the involvement of loss of FOXP1 nuclear protein in most if not all NSCLC cases and are consistent with the frequent deletion of 3p reported in this malignancy (Buchhagen, D. L., 1996. J. Cell. Biochem. Suppl. 24:198-209). The SCLC cases also showed almost complete loss of nuclear FOXP1 expression in 4/6 cases while 2/6 cases showed weak nuclear staining for FOXP1.

**Breast**

A number of tumour suppressor genes including *p53* and *BRCA2* are subject to LOH in breast cancer and these are both associated with a higher frequency of LOH at unrelated loci (Bergthorsson, J. T., et al., 1998. Eur. J. Cancer 34:142-147; Eiriksdottir, G., et al., 1998. Oncogene 16:21-26; Eyfjord, J. E., et al., 1995. Cancer Res 55:646-651; Tseng, S.-L., et al., 1997. Genes Chrm. Cancer 20:377-382). The most thoroughly investigated site for BCRA2-related LOH is 3p13-p14 (Euhus, D. M., et al., 1999. J. Surg. Res. 83:13-18). LOH at 3p12-14 has been identified in a number of other studies, for example (Chen, L. C., et al., 1994. Cancer Res. 54:3021-3024; Devillee, P., et al., 1989. Genomics 5:554-560). Reduced nuclear expression of FOXP1 protein in 6/9 cases of breast cancer has been observed by the present inventors.

Colon

As with other solid tumour types LOH at 3p loci has also been described in colon cancers (Devilee, P., et al., 1991. Int. J. Cancer 47:817-821). Our immunohistochemical staining with the JC12 antibody showed 1 case with a cytoplasmic distribution of FOXP1 and the remaining 3 cases showed only weak nuclear staining of the tumour cells. A more detailed analysis of colon tumours is presented below.

Immunohistochemical staining studies using the JC12 monoclonal antibody frequently show reduced or lack of nuclear FOXP1 expression in a wide range of non-haematopoietic tumours.

**Expression of FOXP1 mRNA in Tumours**

The 1.9kb *Eco*RI fragment containing the 5' end of the *FOXP1* cDNA from pAB195 was labelled with $^{32}P$ and used to probe CLONTECH's Matched Tumor/Normal Expression Array (Figure 12A) according to the manufacturers instructions. The array contains cDNA from 68 human tumours and corresponding normal tissue from the same individual immobilised on a nylon membrane. The cDNA pairs have each been normalised using the expression levels of three house keeping genes so that quantitative comparisons can be made between the expression levels of mRNAs in the normal verses malignant tissue samples. The cDNA pairs are also loaded in relatively equal amounts so that comparisons between different patients and different tissues can be made. Further information about tissues on the array can be obtained from the CLONTECH web site.

Any conclusions drawn from the array data must be drawn within the limitations of the technique. Although the matched array is useful for determining relative levels of gene expression it cannot distinguish closely related or polymorphic mRNAs of different sizes. Also despite appearing to be phenotypically normal, the normal control tissue has been taken from a diseased organ. Furthermore, tumour samples will contain some normal tissue so that a negative tumour may still give a positive signal.

The most obvious feature of the array is that, generally, kidney, breast, uterus, ovary, lung and small intestine show lower mRNA levels for *FOXP1* than the prostate, cervix, colon, rectum and stomach cDNA samples. Also evident is the trend for lower levels of *FOXP1* mRNA in the colon tumour samples compared to their normal counterparts and the trend for higher levels of *FOXP1* mRNA in the stomach tumour samples. Differential expression between normal and tumour samples was observed in nearly half of the cases on this array. The human cancer cell line data indicate, that the two Burkitt's lymphoma cell lines (Figure 12A, 2P and 9P) and the promyelocytic leukaemia line HL-60 (Figure 12, 4P) show high levels of expression of the *FOXP1* gene.

It is tempting to speculate that the low levels of *FOXP1* expression seen in both normal and tumour cDNAs from certain tissues may indicate that some of the phenotypically normal tissue already has alterations in the expression of this gene. LOH at 3p has been reported as an early event which can be detected in preneoplastic tissues from patients with both breast (Dietrich, C. U., et al., 1995. Int. J. Cancer 60:49-53; Euhus, D. M., et al., 1999. J. Surg. Res. 83:13-18) and lung cancer (Wistuba, I. I., et al., 2000. Cancer Res. 60:1949-1960).

To address the issue of the "normal" tissues on the matched tumor/normal array the inventors used the same *Eco*RI fragment from the *FOXP1* gene as a probe on Clontech's Multiple Tissue Array following the manufacturers instructions (Figure 12B). The normal tissues on this array come from non-diseased victims of sudden death/trauma and are pooled from a number of individuals.

The *FOXP1* mRNA is widely expressed in normal tissues and no samples were completely negative for the expression of this gene. Higher levels of mRNA are seen in some samples, particularly those from lymphoid and gastrointestinal tissues. What is particularly interesting is that most of the normal tissues show higher expression than is seen in any of the cancer cell lines. This confirms the original hypothesis that phenotypically normal tissues from patients with cancer may already have altered expression of the *FOXP1* gene. Also the *FOXP1* mRNA was widely expressed in foetal tissues in addition to those from adults indicating that it has a critical role throughout development. These two experiments confirm that the *FOXP1* mRNA is aberrantly either under or overexpressed in a range of tumour types. Additional information about the cases and tissues on these arrays can be obtained from the company website at www.clontech.com.

**AFX like winged helix transcription factors upregulate the expression of $p27^{Kip1}$.**

A recent paper in the Journal Nature, by Medema et. al., demonstrated that overexpression of the AFX-like (FOXO family) forkhead transcription factors caused growth suppression in a variety of cell lines; mediating cell-cycle regulation by Ras and PKB through $p27^{kip1}$. They also showed that AFX could upregulate transcription of the $p27^{kipl}$ gene. They suggested that the proclaimed role of PI(3)K/PKB signalling in tumourigenesis may need refinement as the disruption of the PI(3)K/PKB/Forkhead transcription factor pathway during tumourigenesis may override a cell-cycle block rather than bestow protection from apoptosis. They concluded that inactivation of these proteins is an important step in oncogenic transformation (Medema, R. H., G. J. P. L. Kops, J. L. Bos and B. M. T. Burgering, 2000. Nature 404: 782-787).

The inventors have confirmed that none of the AFX like transcription factors localise to chromosome 3.

| | | |
|---|---|---|
| FOXO1A | (FKHR) | 13q14.1 |
| FOXO1B | (FKHRP1) | 5q35.2-35.3 5q- syndrome 5q31.3-5q33 |
| FOXO2 | (AF6q21) | 6q21 |
| FOXO3A | (FKHRL1) | 6q21 |
| FOXO3B | (FKHRL1P1) | 17p11 |
| FOXO4 | (AFX1) | Xq13.1 |

This data suggests a mechanism by which loss of the FOXP1 protein might be important in the development of malignancy through it's loss affecting the expression of $p27^{kip1}$.

**Overexpression of full length FOXP1 protein in COS cells resulted in increased nuclear expression of $p27^{kip1}$.**

Plasmids pAB195, pAB196, pAB199, pAB200 and the empty vector pBK-CMV were transfected into the COS cell line. Cytospins of these transfected cells were immunostained with either the JC12 antibody recognising FOXP1 or with two MoAbs against $p27^{kip1}$ (Dako and Transduction Laboratories) some of the results are presented in figure 7.

A subset of cells of the *FOXP1* transfections showed increased nuclear staining (pAB195 and pAB196 /JC12) in addition to the background staining of endogenous FOXP1 protein (vector /JC12) indicating that the transfections had been successful. Staining with both of the $p27^{kip1}$ antibodies showed that transfection with the full length FOXP1 cDNA encoded by plasmid pAB195 resulted in an increased nuclear expression of the $p27^{kipl}$ protein (pAB195 /p27 DAKO and pAB195 /p27 TL). The splice variants of FOXP1 encoded by pAB196 and pAB199 and pAB200 did not appear to have this affect on the expression of $p27^{kip1}$ (data presented for pAB196 /p27 DAKO and pAB196 /p27 TL). The N-terminal region of the FOXP1 protein which contains potential transactivation and protein:protein interaction domains is absent from these variants and this region may therefore be functionally important in regulating the expression of $p27^{kip1}$.

Other tumour suppressor genes have variant forms which have different roles in malignancy. For example the RIZ protein, which binds the retinoblastoma tumour suppressor protein, is expressed as two forms of the protein encoded by the same gene. RIZ1 is the full length form which is commonly lost or underexpressed in tumours (lung, breast and neuroblastoma) and RIZ2, which lacks the PR domain (which mediates protein-protein interactions), that is always present (Jiang, G. L., et al., 1999. Int. J. Cancer 83:541-546). Some of the variant forms show increased rather than deceased expression for example with the RBM6 mRNA an increased abundance of the shorter transcript is seen in lung tumour cell lines (Timmer, T., P., et al., 1999. Eur. J. Hum. Genet. 7:478-486).

Since the JC12 antibody recognises the C-terminal region of the protein which is conserved in all the variant forms encoded by pAB196, pAB199, pAB200, our immunohistochemical study is unable to distinguish these different FOXP1 proteins. The *FOXP1* cDNA probe used on the CLONTECH array will also hybridise to all these cDNAs. It is possible therefore that these *FOXP1* mRNAs and the proteins they encode are differentially expressed in tumours and may have different roles in malignancy. Antibodies which can distinguish these different proteins may make better diagnostic reagents.

**INK and CIP/KIP families regulate cell entry into S by inhibiting cyclin/cyclin-dependent kinase complexes.**

The progression of cells through cell cycle control is positively controlled by cyclin/cyclin-dependent kinase (CDK) complexes and is negatively controlled by specific CDK inhibitors (CKI). There are two CKI families CIP/KIP including $p21^{Cip1/Waf1}$, $p27^{kip1}$, and $p57^{Kip2}$ which bind and inhibit cyclin E/cdk2 and cyclin A/cdk2 complexes (Sherr, C. J. and J. M. Roberts, 1995. Science 9:1149-1163) and the INK4 family including $p16^{INK4}$, $p15^{INK4b}$, $p18^{INK4c}$ and $p19^{INK4d}$ which inhibit cyclin D-associated kinases (Parry, D., S. Bates, D. J. Mann and G. Peters, 1995. EMBO J. 14:503-511; Sandhu, C., J. Garbe, J. Daksis et. al., 1997. Mol. Cell. Biol. 17:2458-2467).

The D-type cyclins are under cell cycle control while cyclin E is expressed in all tissues. Current evidence suggests that the S-phase promoting functions of cyclin D and cyclin E associated kinases are conferred by their ability to phosphorylate pRb and release the E2F transcription factors from an inactive or repressive pRb-E2F complex (reviewed (Yamasaki, L., 1998. Results Probl. Cell Differ. 22:199-227)).

The expression of cyclins, cdks, and cdk inhibitors are frequently deregulated in cancers (reviewed by Tsihlias, J., L. Kapusta and J. Slingerland, 1999. Ann. Rev. Med. 50:401-423) however among all the CKIs only $p16^{Ink4a}$ can be classified as a tumour suppressor by the genetic criteria of LOH (Ruas, M. and G. Peters, 1998. Biochem. Biophys. Acta. 1378:F115-177).

**Case for $p27^{kip1}$ as a candidate tumour suppressor gene.**

$p27^{kip1}$ was first identified as an inhibitor in cells arrested by transforming growth factor-β (TGF-β and is regulated by both growth inhibitory cytokines and contact inhibition (Hengst, L., et al., 1994. PNAS 91:5291-5294; Koff, A., et al., 1993. Science 260:536-539; Polyak, C., et al., 1994. Cell 78:59-66; Polyak, K., et al., 1994. Genes Dev. 8:9-22 and Slingerland, J. M., et al., 1994. Mol. Cell Biol. 14:3683-3694). In most normal tissues, during both foetal development and in adults, there is a negative correlation between $p27^{kip1}$ expression and proliferation (Fredersdorf, S. , et al., 1997. PNAS 94:6380-6385; Yatabe, Y., et al., 1998. Cancer Res. 58:1042-1047). While the $p27^{kip1}$ protein is strongly expressed in non-proliferating cells its levels decrease when cells are stimulated by growth factors (Kato, J.-Y., et al., 1994. Cell 79:487-496; Nourse, J., et al., 1994. Nature 372:570-573). Recent studies suggest that $p27^{kip1}$ functions in cellular differentiation and development (Hengst, L. and S. I. Reed, 1996. Science 271:1861-1864), apoptosis induction (Wang, X., M. Gorospe, Y. Huang and N. J. Holbrook, 1997. Oncogene 15:2991-2997) and chemotherapy resistance (St Croix, B., et al., 1996. Nat. Med. 2:1204-1210). $p27^{kip1}$ knockout mice manifest altered differentiation programs (Casaccia-Bonnefil, P., et al., 1997. Genes Dev. 11:2335-2346), and $p27^{kip1}$ expression increases during differentiation in many cell types both in tissue culture and *in vivo* (Durand, B., et al., 1997. EMBO J. 16:306-317; Koyama, H., et al., 1996. Cell 87:1069-1078). There is some disagreement over the association between $p27^{kip1}$ and proliferation in human cancer. The presence of elevated levels of $p27^{kip1}$ protein expression in subsets of tumours with a high proliferative index has been explained by suggesting that the abnormally expressed protein lacks the functional capacity to inhibit cell cycle progression (Fredersdorf, S., et al., 1997. PNAS 94:6380-6385; Quintanilla-Martinez, L., et al., 1998. Am. J. Path. 153: 175-182; Sgambata, A., et al., 1997. Clin. Cancer Res. 3:1879-1887; Yatabe, Y., et al., 1998. Cancer Res. 58:1042-1047).

Evidence that $p27^{kip1}$ may be involved in human tumour progression comes largely from studies that have directly measured the expression of $p27^{kip1}$ protein in clinical tumour samples using immunohistochemical assays. Absent or low levels of $p27^{kip1}$ expression in a subset of cancers of colon (Loda, M., et al., 1997. Nat. Med. 3:231-234; Tenjo, T., et al., 2000. Oncol. 58:45-51), breast (Catzavelos, C., et al., 1997. Nat Med. 3:227-230; Porter, P. L., et al., 1997. Nat. Med. 3:222-225; Tan, P., et al., 1997. Cancer Res. 57:1259-1263), lung (Catzavelos, C., et al., 1999. 59: 684-688; Esposito, V., et al., 1997. Cancer Res. 57:3381-3385), Barrett's esophagus (Singh, S. P., et al., 1998. Cancer Res. 58:1730-1735), melanoma (Flørenes, V. A., et al., 1998. Am. J. Path. 153:305-312), stomach (Mori, M., et al., 1997. Nat. Med. 3:593), hypopharyngeal cancer (Mineta, H., et al., 1999. Anticancer Res. 19:4407-4412), oral squamous cell carcinoma (Ito, R., et al., 1999. Pathobiol. 67:169-173), gastric (Mori, M., et al., 1997. Nat. Med. 3:593) and prostate (Cote, R. J., et al., 1998. J. Natl. Cancer. Inst. 90:916-920; Tsihlias, J., et al., 1998. Cancer Res. 58:542-548; Yang, R. M., et al., 1998. J. Urol. 159:941-945) cancers are associated with cases that have a poor prognosis. Loss of $p27^{kip1}$ protein could contribute to resistance to growth inhibitory factors, (Sandhu, C. et al. 1997. Mol. Cell Biol. 17 : 2458-2467; Koff, A., et al. 1993 Science 260, 536-539; Polyak, K., et al. 1994 Genes Dev. 8 9-22; Slingerland J. et al. 1994 Mol Cell Biol. 14 : 3683-3694; Kato, J-Y., et al. 1994 Cell 79 : 487-496; Hunter, T. and Pines, 1994. Cell 79 : 573-582), deregulation of cell proliferation, and oncogenic change (Hunter, T., and Pines, J., 1994. Cell. 79 573-582, Sherr, J.S., 1996. Science 274:1672-1677). Thus it is a common feature that low levels of p27$^{kip1}$ protein correlate with a poor prognosis in cancers (reviewed in (Clurman, B. E. and P. Porter, 1998. PNAS 95:15158-15160)), and p27$^{kip1}$ is becoming an important clinical marker for tumour progression. As down regulation of p27$^{kip1}$ has been reported to increase sensitivity to anti cancer agents (St Croix, B. et al. 1996. Nat. Med. 2 : 1204-1210), it has been suggested that patients with weak p27$^{kip1}$ expression in their tumours may be the best candidates for adjuvant chemoradiation therapy, considering their poor prognosis (Shamma, A., et al., 2000. Oncol 58:152-158).

The only direct evidence that p27$^{Kip1}$ is a tumour suppressor come from the studies of p27$^{Kip1}$-null mice. p27$^{Kip1}$-deficient mice display a generalised increase in body size, pituitary tumours and multiple organ hyperplasia (Kiyokawa, H., R. D. Kineman, K. O. Manova-Todorova, V. C. Soares, E. S. Hoffman, M. Ono, D. Khanam, A. C. Hayday, L. A. Frohman and A. Koff, 1996. Cell 85:721-732). Unlike classical tumour suppressors, in these mice, only a reduction in p27$^{Kip1}$ levels are necessary to predispose tissues to secondary tumours (Texeira, L. T., H. Kiyokawa, X. D. Peng, K. T. Christov, L. A. Frohman and R. D. Kineman, 2000. Oncogene 19:1875-1884). Thus reduced levels of nuclear FOXP1 protein expression could also be important in the development of malignancy.

Although it is a putative tumour suppressor gene, mutations or deletions in the p27$^{kip1}$ gene occur only rarely in human cancers (Kawamata, N., R. Morosettie, C. W. Miller, D. Park, K. S. Spirin, T. Nakamaki, S. Takeuchi, Y. Hatta, J. Simpson and S. Wilczynski, 1995. Cancer Res 55:2266-2269; Morosetti, R., N. Kawamata, A. F. Gombart, C. W. Miller, Y. Hatta, T. Hirama, J. W. Said, M. Tomonaga and H. P. Koeffler, 1995. Blood 86:1924-1930; Pietenpol, J. A., S. K. Bohlander, Y. Sato, N. Papadopoulos, B. Liu, C. Friedman, B. J. Trask, J. M. Roberts, K. W. Kinzler, J. D. Rowly and B. Vogelstein, 1995. Cancer Res. 55:1206-1210) while another CKI, p16$^{INK4a}$, is rivalling p53 for being the most frequently altered gene in human cancer. Regulation of p27$^{kip1}$ is complex and reports on transcriptional (Kolluri, S. K., C. Weiss, A. Koff and M. Gottlicher, 1999. Genes Dev. 13:1742-1753), translational (Hengst, L. and S. I. Reed, 1996. Science 271:1861-1864; Millard, S. S., J. S. Yan, H. Nguyen, M. Pagano, H. Kiyokawa and A. Koff, 1997. J. Biol. Chem. 272: 7093-7098), proteolytic (Catzavelos, C., N. Bhattacharya, Y. C. Ung, J. A. Wilson, L. Roncari, C. Sandhu, H. Yeger, I. Morava-Protzner, L. Kapusta, E. Franssen, K. I. Pritchard and J. M. Slingerland, 1997. Nature Med. 3:227-230; Loda, M., B. Cukor, S. W. Tam, P. Lavin, M. Fiorentino, G. F. Draetta, J. M. Jessup and M. Pagano, 1997. Nature Med. 3: 231-234; Nguyen, H., D. M. Gitig and A. Koff, 1999. Mol. Cell biol. 19:1190-1201; Pagano, M., S. W. Tam, A. M. Theodoras, P. B. Romero, G. D. Sal, V. Chau, P. R. Yew, G. F. Draetta and M. Rolfe, 1995. Science 269:682-685), and mis-localisation (Orend, G., T. Hunter and E. Ruoslanhti, 1998. Oncogene 16:2575-2583; Soucek, T., R. S. Yeung and M. Hengstschlager, 1998. PNAS 95:15653-15658), mechanisms are reported. However, it has been suggested that p27$^{kip1}$ may be a useful clinical tool even before the mechanisms of p27$^{kip1}$ inactivation are completely understood and the routine use of p27$^{kip1}$ as a prognostic indicator for cancer has been recommended (Moller, M. B., K. Skjodt, L. S. Mortensen and N. T. Pedersen, 1999. Br. J. Haematol. 105:730-736).

Immunohistochemical staining studies using the JC12 and P27$^{Kip1}$ antibodies (Figure 14) have shown that in normal tonsil both proteins have a very similar pattern of distribution with the germinal centre cells being largely negative and the surrounding mantle zone cells and interfollicular small lymphocytes having much higher levels of expression as has been previously described for p27$^{kip1}$ (Fredersdorf, S., J. Burns, A. M. Milne, G. Packham, L. Fallis, C. E. Gillett, J. A. Royds, D. Peston, P. A. Hall, A. M. Hanby, D. M. Barnes, S. Shousha, M. J. O-Hare andX. Lu, 1997. PNAS 94: 6380-6385; Sanchez-Beato, M., A. I. Sáez, J. C. Martinez-Montero, M. S. Mateo, L. Sánchez-Verde, R. Villuendas, G. Troncone and M. A. Piris, 1997. Am. J. Path. 151:151-160). Staining with the MIB-1 antibody confirms that it is quiescent cells which express high levels of both p27$^{kip1}$ and FOXP1 proteins. There was no apparent difference between the FOXP1 protein expression in either the light or dark zones of the germinal centre. The FOXP1 and p27$^{Kip1}$ proteins also have a similar distribution in other normal tissues; for example in normal kidney both proteins are localised in the cytoplasm of proximal tubule cells and are heterogeneously expressed in the nuclei of the distal tubules (Figure 14).

**In haematological malignancies poor prognosis is more generally associated with overexpression of p27$^{kip1}$**

The overexpression pattern of p27$^{kip1}$ in subsets of certain haematological malignancies correlates well with the staining pattern observed for the FOXP1 protein. This could provide an explanation for the lack of 3p deletions observed in haematological malignancies (Johansson, B., R. Billström, U. Kristoffersson, M. Åkerman, S. Garwicz, T. Ahlgren, C. Malm andF. Mitelman, 1997. Leukemia 11:1207-1213) with overexpression of the FOXP1 gene product being required to upregulate the expression of p27$^{kip1}$. However, subsequent experimental data provided by the inventors did not identify a significant correlation between the expression of the FOXP1 and p27$^{kip1}$ proteins in a study of diffuse large B-cell lymphoma. It has also been shown, in mice, that overexpression of *bmi-1* (which upregulates ink4a) induces lymphomas (Alkema, M. J., H. Jacobs, M. van Lohuizen and A. Berns, 1997. Oncogene 15:899-910; van der Lugt, N. M. T., J. Domen, K. Linders, M. van Roon, E. Robanus-Maandag, H. te Riele, M. van der Valk, J. Deschamps, M. Sofroniew, M. van Lohuizen and et. al, 1994. Genes Dev. 8:757-769).

Generally this pattern of p27$^{kip1}$ overexpression is associated with poor prognosis in a number of haematological malignancies, including diffuse large B-cell lymphoma (DLBCL) (Sáez, A., E. Sánchez, M. Sánchez-Beato, M. A. Cruz, I. Chacón, E. Muñoz, F. I. Camacho, J. C. Martinez-Montero, M. Mollejo, J. F. Garcia and M. A. Piris, 1999. Br. J. Cancer 80:1427-1434; Sanchez-Beato, M., F. I. Camacho, J. C. Martinez-Montero, A. I. Saez, R. Villuendas, L. Sanchez-Verde, J. F. Garcia and M. A. Piris, 1999. Blood 94:765-772), Burkitt's lymphoma (BL) (Sanchez-Beato, M., F. I. Camacho, J. C. Martinez-Montero, A. I. Saez, R. Villuendas, L. Sanchez-Verde, J. F. Garcia and M. A. Piris, 1999. Blood 94:765-772), and B-cell chronic lymphocytic leukaemia (B-CLL ) (Vrhorac, R., A. Delmer, R. Tang, J. P. Marie, R. Zittoun and F. Ajchenbaum-Cymbalista, 1998. Blood 91:4694-4700). Sequestration and inactivation of $p27^{kip1}$ by c-myc (Vlach, J., S. Hennecke, K. Alevizopoulos, D. Conti and B. Amati, 1996. EMBO J. 15:6595-6604) which is frequently overexpressed in aggressive lymphomas (Hernandez, S., L. Hernandez, S. Bea, M. Cazorla, P. L. Fernandez, A. Nadal, J. Muntane, C. Mallofre, E. Montserrat, A. Cardesa and E. Campo, 1998. Cancer Res. 58:1762-1767) has been proposed as a mechanism to explain a high proliferation rate despite concomitant elevated intranuclear $p27^{kip1}$ levels (Moller, M. B., K. Skjodt, L. S. Mortensen and N. T. Pedersen, 1999. Br. J. Haematol. 105:730-736).

There are also some reports of reduced $p27^{kip1}$ expression being related to a worse prognosis in DLBCL (Moller, M. B., K. Skjodt, L. S. Mortensen and N. T. Pedersen, 1999. Br. J. Haematol. 105:730-736) and acute myeloid leukaemia (AML) (Yokozawa, T., M. Towatari, H. Iida, K. Takeyama, M. Tanimoto, H. Kiyoi, T. Motoji, N. Asou, K. Saito, M. Takeuchi, Y. Kobayashi, S. Miyawaki, Y. Kodera, R. Ohno, H. Saito and T. Naoe, 2000. Leuk. 14:28-33).

**Allelic Loss at 3p and loss of $p27^{kip1}$ expression is an early event in the genesis of malignancy.**

A number of studies identify allelic loss at either 3p or 3p12-14 in premalignant lesions of head and neck cancers (Califano, J., P. van der Riet, W. Westra, H. Nawroz, G. Clayman, S. Piantadosi, R. Corio, D. Lee, B. Greenberg, W. Koch and D. Sidransky, 1996. Cancer Res. 56:2488-2492; Emilion, G., J. D. Langdon, P. Speight and M. Partridge, 1996. Br. J. Cancer 73:809-813; Roz, L., C. L. Wu, S. Porter, C. Scully, P. Speight, A. Read, P. Sloan and N. Thakker, 1996. Cancer Res. 56:1288-1231), cervical cancer (Fouret, P. J., D. Dabit, J.-L. Mergui and S. Uzan, 1998. Pathobiol. 66:306-310), breast cancer (Dietrich, C. U., N. Pandis, M. R. Teixeira, G. Bardi, A. M. Gerdes, J. A. Anderson and S. Heim, 1995. Int. J. Cancer 60:49-53; Euhus, D. M., A. Maitra, I. I. Wistuba, A. Alberts, J. Albores-Saavedra and A. F. Gazdar, 1999. J. Surg. Res. 83:13-18) and preneoplastic/preinvasive bronchial epithelium (Wistuba, I. I., C. Behrens, A. K. Virmani, G. Mele, S. Milchgrub, L. Girard, J. W. 3. Fondon, H. R. Garner, B. McKay, F. Latif, M. I. Lerman, S. Lam, A. F. Gazdar and J. D. Minna, 2000. Cancer Res. 60:1949-1960). In studies of oral, breast, and Barrett's-associated preinvasive and invasive cancers, reduction in the level of $p27^{kip1}$ is associated with increasing degree of malignancy (Catzavelos, C., N. Bhattacharya, Y. C. Ung, J. A. Wilson, L. Roncari, C. Sandhu, H. Yeger, I. Morava-Protzner, L. Kapusta, E. Franssen, K. I. Pritchard and J. M. Slingerland, 1997. Nature Med. 3:227-230; Jordan, R. C., G. Bradley and J. Slingerland, 1998. Am. J. Pathol. 152:585-590; Loda, M., B. Cukor, S. W. Tam, P. Lavin, M. Fiorentino, G. F. Draetta, J. M. Jessup and M. Pagano, 1997. Nature Med. 3:231-234; Porter, P. L., K. E. Malone, P. J. Heagerty, G. M. Alexander, L. A. Gatti, E. J. Firpo, J. R. Daling and J. M. Roberts, 1997. Nat. Med. 3:222-225; Tan, P., B. Cady, M. Wanner, P. Worland, B. Cukor, C. Magi-Galluzzi, P. Lavin, G. Draetta, M. Pagano and M. Loda, 1997. Cancer Res. 57: 1259-1263). It has been suggested that in cancers where both invasive and non invasive tumours co-exist, loss of $p27^{Kip1}$ protein is seen in both in situ and invasive components, suggesting that events leading to deregulation of $p27^{Kip1}$ may precede invasion, this is reviewed by (Tsihlias, J., L. Kapusta and J. Slingerland, 1999. Am. Res. Med. 50:401-423).

A possible explanation for this has been proposed (Vidal, A. and A. Koff, 2000. Gene 247:1-15), in which alterations that only cause a decrease in the $p27^{kip1}$ protein would not necessarily affect the pRb pathway thus, only if a second tumourigenic event occurred would loss of $p27^{Kip1}$ cause malignancy. The loss of *FOXP1* mRNA expression in some phenotypically normal tissue samples on the CLONTECH array (Figure 6) is consistent with the hypothesis that loss or reduction in the expression of this gene may be an early event in the development of malignancy. This hypothesis was further supported by the JC12 immunostaining of stomach and colon tumours where changes in FOXP1 protein expression were detected in preneoplastic cells.

**Correlation between the LOH at 3p12-14, and expression patterns of FOXP1 and $p27^{kip1}$.**

Examples of the immunohistochemical staining studies comparing the expression patterns of FOXP1 protein (using JC12) and $p27^{kip1}$ protein (using monoclonal antibodies from both DAKO and Transduction Laboratories) are presented in figures 15 and 16.

The *in situ* breast carcinoma case presented in figure 15A shows that most areas of the tumour strongly expressed nuclear FOXP1 and $p27^{kip1}$ proteins. However, some areas of the tumour show a loss of both FOXP1 and $p27^{Kip1}$ protein expression illustrating the heterogeneity of expression of both these proteins which often occurs within a tumour. The SCLC and NSCLC lung carcinoma cases demonstrate the loss of nuclear JC12 proteins in these tumour cells which was found in all cases we examined. This figure also shows that, although the DAKO $p27^{Kip1}$ antibody often exhibits a higher level of cytoplasmic staining than is observed with the antibody from Transduction Laboratories, similar nuclear staining patterns were observed with both antibodies. A previous report on the expression of $p27^{Kip1}$ in lung cancers reported that NSCLC tumours showed reduced expression of this protein while SCLC tumours showed increased expression of $p27^{kip1}$ protein (Yatabe, Y., et al., 1998. Cancer Res. 58:1042-1047). We found that in all the SCLC cases examined a variable proportion of tumour cells showed very high level expression of $p27^{Kip1}$ while other cells appeared to show almost no nuclear expression of $p27^{Kip1}$ protein.

The renal cell carcinoma cases presented in figure 16 show the range of staining patterns observed for both the FOXP1 and $p27^{Kip1}$ proteins confirming the relationship between tumours that have lost nuclear FOXP1 protein with their loss of nuclear $p27^{Kip1}$ protein and demonstrating the degree of heterogeneity in expression levels among the tumour cells. Although there are again some differences between the two $p27^{Kip1}$ monoclonal antibodies the cytoplasmic staining in some cases is observed with both antibodies (Figure 16 C and D) indicating that we have also observed the previously reported cytoplasmic mislocalisation of $p27^{Kip1}$ protein (Orend, G., T. Hunter and E. Ruoslanhti, 1998. Oncogene 16:2575-2583; Soucek, T., R. S. Yeung and M. Hengstschlager, 1998. PNAS 95:15653-15658). In both renal cases (Figure 16 C and D) where we show cytoplasmic $p27^{kip1}$ protein using both $p27^{Kip1}$ antibodies we also observe a cytoplasmic distribution of the FOXP1 protein.

The correlation between the LOH at 3p, and loss of nuclear expression of $p27^{Kip1}$ and FOXP1 proteins in solid tumours together with the reported lack of 3p LOH and overexpression of both $p27^{Kip1}$ and FOXP1 proteins in subsets of certain haematological neoplasms suggests that the three events are related. It is plausible that the LOH at 3p results in the disruption of the FOXP1 gene expression leading to a loss of nuclear FOXP1 protein and subsequent decrease in the nuclear expression of the $p27^{kip1}$ protein in cases which have lost the expression of both proteins.

**The FOXP1 protein contains a potential cyclin recognition motif and cdk phosphorylation sites.**

The FOXP1 protein, like $p27^{Kip1}$, contains potential cyclin binding motifs (or RXL motif) at aa 66-68, 325-7 and 484-6. Although the majority of these sites have a basic residue or cysteine before the arginine the FOXP1 RXL sequence has an alanine residue as does *C. elegans* predicted 2 (Vlach, J., S. Hennecke and B. Amati, 1997. EMBO J. 16: 5334-5344). This motif has been found in proteins other than CKIs including the retinoblastoma family proteins p107 and p130, as well as the E2F-1, -2 and -3 transcription factors (Adams, P. D., W. R. Sellers, S. K. Sharma, A. D. Wu, C. M. Nalin and W. G. Kaelin Jr, 1996. Mol. Cell. Biol. 16:6623-6633; Zhu, L., E. Harlow and B. D. Dynlacht, 1995. Genes Dev. 9:1740-1752) and it may function by targeting substrates to active cyclin-CDK complexes. The FOXP1 protein also contains a recognition site for the p70S6-kinase (RRYS) which is regulated by the PI3 kinase. The biological activity of the FOXP1 protein may therefore be affected by the PI3 kinase signalling pathway as are other members of the winged helix family (Kops, G.J., and Burgering, B.M., 1999. J. Mol. Med. 77 656-665). The FOXP1 protein also contains several versions of a preferred cdk phosphorylation site (S/T-P-X-Z where Z is typically a basic residue) (Nigg, E., 1991. Semin. Cell. Biol. 2:261-270; Songyang, Z., S. Blechner, N. Hoagland, M. F. Hoekstra, H. Piwnica-Worms and L. C. Cantley, 1994. Curr. Biol. 4:973-982; Srinivasan, J., M. Koszelak, M. Mendelow, Y.-G. Kwon and D. S. Lawrence, 1995. Biochem. J. 309:927-931; Zhang, J., R. J. Sanchez, S. Wang, C. Guarnaccia, A. Tossi, S. Zahariev and S. Pongor, 1994. Biophys. 315:415-424) indicating that it may be a substrate for the cyclin dependent protein kinase.

**Immunostaining mouse tissues with the JC12 antibody.**

The human FOXP1 protein has almost complete homology with the mouse Foxp1 protein within the published winged helix domain. To investigate the possibility that the FOXP1 specific antibody JC12 might also recognise the mouse protein we stained mouse spleen with this antibody (as described in materials and methods). We were able to weakly detect a nuclear protein in mouse spleen with our JC12 antibody (shown on figure 16 top right) demonstrating that the epitope recognised by this antibody is conserved between the mouse and human FOXP1 proteins.

It is therefore postulated that FOXP1 is a candidate tumour suppressor gene which is located on the short arm of chromosome 3 in a region that commonly shows loss of heterozygosity in a range of solid tumour types. Our immunostaining studies show that a number of solid tumour types, known to be associated with LOH at 3p, frequently show reduced or mis-localised expression of the FOXP1 protein. We show that the expression pattern of FOXP1 protein in normal tonsil and kidney has a similar distribution to that of the $p27^{Kip1}$ protein and that both have a reciprocal expression pattern with the MIB-1 antigen in tonsil. We have found that in the majority of cases where the solid tumour cells show reduced or cytoplasmic expression of FOXP1 protein they also show aberrant expression patterns for $p27^{Kip1}$. Equally in the majority of cases where the tumour cells show 'normal' nuclear expression of the FOXP1 protein they also show nuclear expression of the $p27^{Kip1}$ protein. Overexpression of the full length FOXP1 protein leads to a nuclear accumulation of $p27^{Kip1}$ protein in transfected COS cells, demonstrating that the expression level of the FOXP1 protein may directly affect the expression levels of the $p27^{Kip1}$ protein. Interestingly we have identified cases of Diffuse Large B-cell lymphoma (DLBCL) which overexpress nuclear FOXP1 protein but lack nuclear $p27^{Kip1}$ protein. A recent study of DLBCL using microarrays shows that the mRNA for $p27^{Kip1}$ is upregulated in an activated B-like subtype associated with a worse prognosis when compared to a germinal centre-like subgroup which shows less mRNA expression for $p27^{Kip1}$ (Alizadeh, A. A., M. B. Eisen, R. E. Davis, C. Ma, I. S. Lossos, A. Rosenwald, J. C. Boldrick, H. Sabet, T. Tran, X. Yu, J. I. Powell, L. Yang, G. E. Marti, T. Moore, J. Hudson Jr, L. Lu, D. B. Lewis, R. Tibshirani, 'G. Sherlock, W. C. Chan, T. C. Greiner, D. D. Weisenburger, J. O. Armitage, R. Warnke, R. Levy, W. Wilson, M. R. Grever, J. C. Byrd, D. Botstein, P. O. Brown and L. M. Staudt, 2000. Nature 403:503-511) indicating that transcriptional regulation of $p27^{Kip1}$ expression does occur in this malignancy. We propose that the FOXP1 protein may play an important role in the development of malignancy one possible mechanism being through it's effect on the expression of the $p27^{kip1}$ protein.

**Expression of FOXP1 in Pancreatic Tumours**

To provide experimental evidence concerning the expression of the FOXP1 protein in pancreatic tumours the present inventors immunostained paraffin embedded normal and pancreatic tumour tissues obtained from the John Radcliffe Hospital, Oxford with the JC12 monoclonal antibody. The data is presented in figure 17. Firstly, the expression of the FOXP1 protein in pancreas from a patient without tumours was investigated to confirm that the expression levels of the protein were similar to those in "normal" pancreas taken from cancer patients. In normal pancreas, about 80-90% of cells in the excreting duct show moderate to strong nuclear FOXP1 expression; exocrine glands show most cells with moderate nuclear FOXP1 expression and the majority of the remainder show some nuclear protein expression. Ducts show nuclear FOXP1 protein expression and more cytoplasmic protein expression than the endocrine cells, although these levels are quite variable. In case A the tumour cells do not show increased expression of the FOXP1 protein when compared to normal tissue and the tumour contains a proportion of negative nuclei. In cases B, C and E the tumour shows a loss of nuclear FOXP1 expression and very strong cytoplasmic expression of this protein. In case E the arrow shows an area of the tumour where the malignant cells show nuclear expression of the FOXP1 protein while other areas show cytoplasmic expression indicating that variation in the FOXP1 protein expression can occur within a tumour. In case D the tumour cells are indicated with arrows and these show very low levels of the nuclear FOXP1 protein. In summary, none of the five cases of pancreatic tumour showed over expression of the nuclear FOXP protein. While 3/5 cases showed a loss of nuclear FOXP1 protein and high levels of cytoplasmic protein expression the remaining two cases showed loss of nuclear protein expression without the high levels of cytoplasmic protein. FOXP1 over expression was not a characteristic of all pancreatic tumours and there was no evidence for increased expression of a nuclear FOXP1 protein in any of these five cases. The increased cytoplasmic FOXP1 protein expression in three of these tumours may be the result of increased mRNA levels or alternatively increased stability of the protein. However, the abnormal localisation of this protein does not correspond to functional over expression of this normally nuclear protein. In the cases which exhibited strong cytoplasmic staining this was a very good marker for pancreatic tumour cells and even scattered individual neoplastic cells were easy to visualise after JC12 immunostaining. In conclusion the present inventors did not find over expression of the nuclear FOXP1 protein in pancreatic tumours and loss of nuclear FOXP1 protein expression was the more general rule.

**Expression of FOXP1 in Stomach Tumours.**

Data obtained from Clontech's matched tumor/normal array probed with the FOXP1 cDNA indicated that stomach tumours expressed higher levels of *FOXP1* mRNA than the adjacent phenotypically normal tissue from the same patient. To investigate the expression of the FOXP1 protein in these tumours, cases were obtained from the routine files of the Histopathology Department of the Emek Medical Centre (Afula- Israel). To detect the expression of the FOXP1 protein paraffin embedded sections were immunostained using the JC12 monoclonal antibody. This study included 43 samples taken from surgically resected stomach including 20 adenocarcinomas, and 27 samples of normal gastric mucosa (14 cases from normal surgical margins of gastric carcinoma and 13 cases from gastric resections for benign conditions, mostly Peptic Ulcer Disease-PUD). Typical results are illustrated in figure 18 and described in the figure legend.

The nuclear expression of the FOXP1 protein in the different categories of carcinomas varies from those having no detectable nuclear FOXP1 protein to those with strong nuclear protein expression. The majority of the cases weakly express the FOXP1 protein in the nucleus. In most of the cases the nuclear FOXP1 expression is heterogeneous with different intensities of staining being observed in areas from the same tumour (examples are illustrated in F and comparisons between E&H or G&J). The most obvious difference between normal and tumour cells is in the cytoplasmic staining. There is obviously decreased expression of cytoplasmic FOXP1 in the malignant cells when compared to their normal counterpart (which typically have only low levels if any nuclear FOXP1 protein expression). Only in a few cases or in focal areas within a case was strong cytoplasmic expression observed. This change in cytoplasmic staining was also observed in metaplastic cells (D) indicating that this change occurs early and is detectable before the cells become malignant.

**Expression of FOXP1 in Diffuse Large B-cell Lymphomas (DLBCL).**

Diffuse large B-cell lymphoma (DLBCL) accounts for 30-40% of all adult non-Hodgkin's lymphomas and is heterogeneous in terms of its morphology and clinical features (Harris, et al., 1994. 84:1361-1392). Approximately 50% of patients relapse after treatment (The Non-Hodgkin's Lymphoma Classification Project.1997. Blood 89:3909-3918) and their tumours frequently become resistant to therapy. The genetic abnormalities underlying DLBCL remain poorly understood and in contrast to other lymphoma types (e.g. follicular lymphoma or Burkitt's lymphoma), no single characteristic genetic alteration has been found.

There have been studies suggesting that DLBCL can be divided into subtypes. The Kiel classification scheme, using morphological criteria, proposed that some DLBCL arise from germinal center B cells and others from extracellular B cells ("centroblastic" and "immunoblastic" lymphomas respectively) (Lennert, et al. 1975. Br. J. Haematol.,31(Suppl): 193-203). There is, however, controversy as to whether subtypes differ in term of their clinical behavior (Stein and Dallenbach. 1992. In D. M. Knowles (Eds.), Neoplastic Hematopathology. (pp. 675-714). Baltimore: Williams & Wilkins; Baars, et al. 1999. Br. J. Cancer 79:1770-1776, Kwak, et al. 1991. Cancer 68:1988-1993). The absence of objective criteria for these categories, (for example, immunophenotypic details) combined with the lack of reproducibility in their diagnosis means that few centres attempt to subdivide DLBCL morphologically and no such distinction is described in the "REAL" classification (Harris, et al. 1994. Blood, 84:1361-1392).

Recent genetic studies have provided evidence for the possibility of at least two sub-categories of DLBCL. The presence of the (14;18) translocation in a minority of cases suggests that some DLBCL represent transformed/diffuse follicular lymphomas (possibly associated with the acquisition of MYC and p53 abnormalities), in contrast to the commoner "*de novo"* cases (in which the BCL-6 gene may be involved (Dalla-Favera, et al. 1994. Ann. Oncol., 5 Suppl:S55-S60). A recent study using microarray-based techniques to study DLBCL gene expression also claims to have identified two subgroups of DLBCL, those with a germinal center-like profile having a better prognosis than those with an activated B-like profile (Alizadeh, et al. 2000. Nature 403:503-511). However, this approach is not yet practical in the routine clinical context and furthermore it provides no evidence for the expression of functional proteins. There is, therefore, a clear need to identify genetic changes associated with DLBCL, and to establish if these define clinically relevant subgroups.

**Expression of the FOXP1 protein in cases of DLBCL which have been sub-classified using genetic features.**

Cases of DLBCL which had been sub-classified on the basis of a common genetic background as being of either germinal center (CD10+, BCL-6 >70% cells positive, 14q32 translocation present, cIg negative) or post germinal centre (CD10-, BCL-6<50% cells positive, 14q32 negative, cIg positive) phenotype were provided by Dr German Ott, (Wuerzburg). Immunostaining of these cases with the JC12 antibody (without prior knowledge of the sub-grouping) identified either weak or no FOXP1 protein expression in 4/6 cases of germinal centre phenotype (GC top row, Fig.Z) and moderately high expression of the FOXP1 protein in 6/8 cases of post germinal centre phenotype (postGC botton row, Fig. 19). These subgroups are not necessarily the same as those in the microarray study but this method was not available for the analysis of these cases. These data do, however, indicate that the expression of FOXP1 protein may have a diagnostic use in subtyping this disease.

**Relationship between FOXP1 expression, cell-cycle regulatory proteins and the clinical outcome of DLBCL.**

Two previously published studies have investigated the expression of cell cycle regulatory proteins in DLBCL cases obtained from the "Virgin de la Salud" Hospital, Toledo, Spain (Sanchez E., et al. J Clin Oncol. 1998; 16(5):1931-9; Saez A., et al. Br. J. Cancer. 1999; 80(9):1427-34). Immunostaining of these cases enabled FOXP1 protein expression to be compared with the expression of other molecules such as $p27^{kip1}$, MIB-1, P53, BCL-2, $p21^{waf1}$, MDM2, Rb and clinical information such as overall survival, disease free survival, end of disease free survival, end of overall survival, stage, LDH, extranodal origin, patient age, and IPI score.

Immunostaining of 96 of these cases with the JC12 antibody was scored by a qualified pathologist who also confirmed the diagnosis of DLBCL. The immunohistochemical staining was scored for each case as:-

1. positive (68 cases) or negative (28 cases)
2. number of positive cells

| | | |
|---|---|---|
| 0 | None | 19 Cases |
| 1 | <10% | 10 cases |
| 2 | 10-50% | 15 cases |
| 3 | >50% | 52 cases |
| 4 | | |

3. intensity of staining

| | | |
|---|---|---|
| 0 | None | 19 Cases |
| 1 | weak | 25 cases |
| 2 | moderate | 31 cases |
| 3 | strong | 21 cases |

Preliminary statistical analyses of these data were performed using the program Statview. Nominal and continuous categories were compared using non parameteric tests (either Mann-Whitney or Kruskal-Wallis) and nominal categories were compared using contingency tables. JC12 staining results were treated as nominal categories in statistical analyses. Survival graphs were produced using Kaplan-Meier plots.

An interesting feature of the FOXP1 expression in DLBCL was that there was a significant correlation between the intensity of staining and the percentage of positive cells (P-value 0.0001). There were no cases which showed a strong intensity of staining where there were less than 10% JC12 positive cells.

When analysing the significance of positive or negative FOXP1 expression in univariate analyses we identified a relationship between the expression of FOXP1 and retinoblastoma (Rb) proteins with FOXP1 negative cases containing a lower percentage of Rb positive cells (P-value 0.0015).

Comparisons using the percentage of FOXP1 positive tumour cells again identified this relationship with expression of the Rb protein (P-value 0.0061) and a correlation with the age of the patients (P-value 0.0381). When cases having more than 50% cells positive (group 3) were compared to all the others this relationship with retinoblastoma expression (P-value 0.0064) was retained and there was an additional significant relationship with the MIB-1 positivity (P-value 0.0312) indicating a higher rate of cellular proliferation in this subset of cases. This was unexpected as the MIB-1 staining of normal tonsil had indicated that FOXP1 protein expression was normally higher in quiescent cells.

Comparisons using the intensity of FOXP1 expression again identified the correlation with increasing FOXP1 levels reflecting increased levels of the Rb protein (P-value 0.0508) and again there was a correlation with the age of the patients (P-value 0.0104). There was also an additional correlation between the intensity of FOXP1 expression and expression of the P53 tumour suppressor gene (P-value 0.0215) with the strongest FOXP1 staining group showing lower P53 expression than the others. When comparing the strongest staining group (group 3) with all the others there was still a significant correlation between the expression of Rb (P-value 0.0241), patients age (p-value 0.0169) and P53 (p-value 0.0037) proteins.

The loss of retinoblastoma protein has been proven to have an adverse effect on survival in tumours of different lineages (Cance WG, Brennan MF, Dudas ME, et al. N Engl. J. Med. 323: 1457-1462, 1990). This relationship was confirmed by the published study on this DLBCL case series in both univariate and multivariate analyses (Sanchez E., et al. J. Clin. Oncol. 1998; 16(5):1931-9). In our preliminary univariate analysis we have determined that there is a significant correlation between both the number of cells expressing FOXP1 and the intensity of this FOXP1 expression when compared to the expression of the retinoblastoma protein which indicates that FOXP1 protein expression may have prognostic value in DLBCL. This is particularly significant in view of our sequence analysis of the FOXP1 protein which has identified cdk phosphorylation sites and motifs which may be involved in cyclin binding in this protein. These domains are also present in the retinoblastoma protein which has important roles in control of the cell cycle and apoptosis protection.

In summary we have identified the differential expression of the FOXP1 protein in DLBCL and our preliminary data indicate that the expression of FOXP1 may have prognostic value in this malignancy. Although our univariate analyses did not identify a significant correlation between FOXP1 expression and clinical outcome the Kaplan Meier plots (Figure 20) do suggest that there may be a difference between survival in cases with no FOXP1 expression and those with either high intensity or high percentage positive FOXP1 expression.

**Expression of FOXP1 in Colon Tumours**

The study comprised 40 paraffin embedded sections taken from 28 surgically resected large bowel samples. These included 20 tumours taken from 19 patients, together with 11 sections from normal surgical margins and 6 sections taken from colectomy performed for benign conditions (diverticulosis, volvulus, traumatic perforation, ischemic bowel disease and Crohn's disease) as normal controls. The cases were obtained from the files of the Histopathology Department of the Emek Medical Centre (Afula-Israel). The routinely processed paraffin sections were immunostained using the JC12 monoclonal antibody to detect the expression of the FOXP1 protein. The results are illustrated in Figure 21.

In summary, the nuclear and to a lesser extent the cytoplasmic staining with the JC12 monoclonal antibody (corresponding to the expression of the FOXP1 protein) is heterogeneous, both when comparing different tumours and within areas of the same tumour. There is a definite trend towards decreased cytoplasmic FOXP1 expression in the carcinomas (18/20) in comparison with the non-neoplastic mucosa (controls and surgical margins of tumours). The majority of the colon carcinomas (15/20) also show weak to absent nuclear FOXP1 expression. These JC12 immunostaining data support our results obtained from analysing the *FOXP1* mRNA expression in colon tumours using Clontech's matched tumor/normal expression array. Our data show that the majority of the colon tumours, which we have studied, show a loss of FOXP1 expression at the levels of both mRNA and protein expression.

**FOXP1 expression in Mantle Cell Lymphomas (MCL)**

Mantle cell lymphoma cases were provided by Dr Elias Campo from Barcelona, Spain who has published a study concluding that the blastic variant of mantle cell lymphoma tended to express higher levels of the $p27^{Kip1}$ protein despite it's higher rate of proliferation (Quintanilla-Martinez, L. et al., Am. J. Path. 1998;153:175-182). We immunostained both ordinary and blastic variants of mantle cell lymphoma with the JC12 antibody to assess the expression of the FOXP1 protein in these neoplasms.

These data are illustrated in Figure 22. The top row of the figure illustrates 7 cases of mantle cell lymphoma while the bottom row illustrates 5 cases of the blastic variant of mantle cell lymphoma. We have found that 4/5 cases of the blastic variant of MCL show very high expression of the nuclear FOXP1 protein compared to only 2/7 cases of typical MCL. The blastic variant of MCL, in contrast to the typical cases has been shown to have frequent occurrence of mutations and deletions in negative cell cycle-regulatory genes *(p53, $p16^{INK4a}$* and *$p21^{waf1}$*), which may contribute to the development of more aggressive disease with higher proliferative activity (Pinyol M. et al., Blood 1997, 89:272-280; Hernandez, L. et al., Blood 1996, 87:3351-3359).

Mantle cell lymphomas are characterised by the t(11;14) translocation which leads to overexpression of cyclin D1. In itself cyclin D1 has little transforming activity and a number of other proteins have been shown to interact with this protein and increase its oncogenic activity (summarised in Quintanilla-Martinez, L. et al., Am. J. Path. 1998;153: 175-182). Because the FOXP1 protein contains potential cyclin binding sites this protein may have a role which affects the transforming activity of cyclin D1. The overexpression of the FOXP1 protein may also be useful to help distinguish the more aggressive blastic variant of mantle cell lymphoma.

Quintanilla-Martinez, L. et al., Mantle Cell Lymphomas Lack Expression of $p27^{kip1}$, a Cyclin-Dependent Kinase Inhibitor Am. J. Path. 1998;153:175-182 Pinyol M. et al., Deletions and loss of expression of $p16^{INK4a}$ and $P21^{Waf1}$ genes are associated with aggressive variants of Mantle cell lymphomas. Blood 1997, 89:272-280; Hernandez, L. et al., *p53* gene mutations and protein overexpression are associated with aggressive variants of mantle cell lymphomas. Blood 1996, 87:3351-3359

**Expression of FOXP1 in prostate tumours**

Slides of multi-tissue blocks containing cases of prostate tumours together with some normal and premalignant tissue were provided by Dr Elias Campo and Dr Pedro Fernàndez from Barcelona, Spain. The normal prostate tissue showed either scattered cytoplasmic or scattered cytoplasmic and nuclear expression (not generally in the same cell) of the FOXP1 protein. Of the 27 tumours stained with the JC12 antibody, 9 cases did not express the FOXP1 protein, while 7 cases showed cytoplasmic expression and 10 cases showed nuclear FOXP1 protein expression. The expression of FOXP1 was very complex in this tissue and there was no overall common pattern. However, in several cases there was a distinct difference between the expression of the FOXP1 protein in the normal tissue and in the tumour from the same patient. We have certainly also observed tumours which express higher levels of the FOXP1 protein than the normal tissue be it localised to the nucleus or the cytoplasm. This FOXP1 overexpression combined with its loss of expression in other prostate tumours indicates that this differential expression of the FOXP1 protein may have clinical implications in this malignancy.

**Therapeutic/Diagnostic Potential**

1. Use of FOXP1 in early detection of malignancy and as a prognostic indicator.

Both the loss of LOH on 3p and decreased $p27^{kip1}$ protein expression are known to be early events in the development of solid tumours and are associated with a poor prognosis. As it has been demonstrated that loss of FOXP1 protein expression in the nucleus is linked to these observations, then analysis of the *FOXP1* gene sequence or aberrant patterns of mRNA or protein expression could have a wide range of applications in screening programmes for the early detection of premalignant lesions in a range of tumour types e.g. cervical, breast, prostate, stomach, colon, renal, head and neck and lung. The JC12 antibody staining combined with staining for $p27^{Kip1}$ might lead to a more reliable prognosis in both haematological and non-haematological malignancies.

**2. Genetic screening**

As increased information becomes available it is possible that inherited mutations in the *FOXP1* gene will be detected. The identification of inherited mutations could lead to screening for families with a predisposition to a range of cancers.

**3. Development of effective treatment plans.**

As down regulation of $p27^{Kip1}$ has been reported to increase sensitivity to anti cancer agents, (St Croix, B. et al. 1996. Nat. Med. 2 : 1204-1210) it has been suggested that patients with weak $p27^{Kip1}$ expression in their tumours may be the best candidates for adjuvant chemoradiation therapy, considering their poor prognosis (Shamma, A., Y. Doki, T. Tsujinaka, H. Shiozaki, M. Inoue, M. Yano, K. Kawanishi and M. Monden, 2000. Oncol. 58:152-158). Assessment of the FOXP1 status of these patients may also help this process.

**4. Gene therapy**

As the technique of gene therapy becomes better established the introduction of the *FOXP1* gene into tumours could be used to treat patients which have no functional copy of this gene.

**5. Veterinary use**

The FOXP1 antibody JC12 was able to detect the mouse Foxp1 protein in mouse spleen and the African Green Monkey protein in COS cells. This cross reactivity between species raises the possibility that detection of the FOXP1 protein may be of veterinary use in assessing the prognosis/treatment of tumours from other animal species.

<u>**References**</u>

Aburent, H., Wang, Y., Shibata, Y., Noda, T., Schwartz, D., & D., H. (1994). *Am. J. Hum. Genet.,* **55 Suppl.,** 269.

Adams, P. D., Sellers, W. R., Sharma, S. K., Wu, A. D., Nalin, C. M., & Kaelin Jr, W. G. (1996). Identification of a cyclin-cdk2 recognition motif present in substrates and p21-like cyclin-dependent kinase inhibitors. *Mol. Cell. Biol.,* **16,** 6623-6633.

Alizadeh, A. A., Eisen, M. B., Davis, R. E., Ma, C., Lossos, I. S., Rosenwald, A., Boldrick, J. C., Sabet, H., Tran, T., Yu, X., Powell, J. I., Yang, L., Marti, G. E., Moore, T., Hudson Jr, J., Lu, L., Lewis, D. B., Tibshirani, R., Sherlock, G., Chan, W. C., Greiner, T. C., Weisenburger, D. D., Armitage, J. O., Warnke, R., Levy, R., Wilson, W., Grever, M. R., Byrd, J. C., Botstein, D., Brown, P. O., & Staudt, L. M. (2000). Distinct types of diffuse large B-cell lymphoma identified by gene expression profiling. Nature, **403,** 503-511.

Asante-Owusu, R. N., Banham, A.H., Böhnert, H.U., Mellor, E.J.C. & Casselton, L.A. 1996. Heterodimerization between two classes of homeodomain proteins in the mushroom *Coprinus cinereus* brings together potential DNA-binding and activation domains. Gene 172: 25-31.

Alkema, M. J., Jacobs, H., van Lohuizen, M., & Berns, A. (1997). Peturbation of B and T cell development and predisposition to lymphomagenesis in *E$\mu$-Bmi1* transgenic mice require the Bmi1 RING finger. *Oncogene,* **15**, 899-910.

Baars, J. W., de Jong, D., Willemse, E. M., Gras, L., Dalesio, O., v. Heerde, P., Huygens, P. C., v. d. Lelie, H., & Kr v.d. Borne, A. E. G. (1999). Diffuse large B-cell non-Hodgkin lymphomas: the clinical relevance of histological subclassification. Br. J. Cancer, 79, 1770-1776.

Bergthorsson, J. T., Johannsdottir, J., Jonasdottir, A., Eiriksdottir, G., Egilsson, V., Ingvarsson, S., Barkardottir, R. B., & Arason, A. (1998). Chromosome imbalance at the 3p14 region in human breast tumors: High frequency in patients with inherited predisposition due to BRCA2. *Eur. J. Cancer,* **34,** 142-147.

Béroud, C., Joly, D., Gallou, C., Staroz, F., Orfanelli, M. T., & Junien, C. (1998). Software and database for the analysis of mutations in the VHL gene. *Nucl. Acids Res.,* **26,** 256-258.

Bockmühl, U., Wolf, G., Schmidt, S., Schwendel, A., Jahnke, V., Dietel, M., & Petersen, I. (1998). Genomic alterations associated with malignancy in head and neck cancer. *Head & Neck,* **20,** 145-151.

Brauch, H., Johnson, B., Hovis, J., Yano, T., Gazdar, A., Pettengill, O. S., Graziano, S., Sorenson, G. D., Poiesz, B. J., Minna, J., & al, e. (1987). Molecular analysis of the short arm of chromosome 3 in small-cell and non-small-cell carcinoma of the lung. *N. Engl. J. Med.,* **317,** 1109-1113.

Brauch, H., Tory, K., Kotler, F., Gazdar, A. F., Pettengill, O. S., Johnson, B., Graziano, S., Wintin, T., Buys, C. H., Sorenson, G. D., Poiesz, B. J., Minna, J. D., & Zbar, B. (1990). Molecular mapping of deletion sites in the short arm of chromosome 3 in human lung cancer. *Genes Chrm. Cancer,* **1***,* 240-246.

Buchhagen, D. L. (1996). Frequent involvement of chromosome 3p alterations in lung carcinogenesis: allelotypes of 215 established cell lines at six chromosome 3p loci. *J. Cell. Biochem. Suppl.,* **24,** 198-209. '

Buchhagen, D. L., Worsham, M. J., Dyke, D. L., & Carey, T. E. (1996). Two regions of homozygosity on chromosome 3p in squamous cell carcinoma of the head and neck: comparison with cytogenetic analysis. *Head Neck,* **18**, 529-537.

Bugert, P., Kenck, C., Wilhelm, M., & Kovacs, G. (1996). Refining a proximal breakpoint cluster at chromosome 3p11.2 in non-papillary renal cell carcinomas. *Int. J. Cancer,* **68**, 723-726.

Bugert, P., Wilhelm, M., & Kovacs, G. (1997). FHIT gene and the FRA3B region are not involved in the genetics of renal cell carcinomas. *Genes Chrm. Cancer,* **20**, 9-15.

Cairns, P., Polascik, T. J., Eby, Y., Tokino, K., Califano, J., Merlo, A., Mao, L., Herath, J., Jenkins, R., Westra, W., Rutter, J. L., Buckler, A., Gabrielson, E., Tockman, M., Cho, K. R., Hedrick, L., Bova, G. S., Isaacs, W., Koch, W., Schwab, D., & Sidransky, D. (1995). Frequency of homozygous deletion of p16/CDKN2 in primary human tumours. *Nature Genet.,* **11**, 210-212.

Califano, J., van der Riet, P., Westra, W., Nawroz, H., Clayman, G., Piantadosi, S., Corio, R., Lee, D., Greenberg, B., Koch, W., & Sidransky, D. (1996). Genetic progression model for head and neck cancer: implications for field cancerization. *Cancer Res.,* **56***,* 2488-2492.

Cance WG, Brennan MF, Dudas ME, et al. Altered expression of the retinoblastoma gene product in human sarcomas. N Engl. J. Med. 323: 1457-1462, 1990.

Casaccia-Bonnefil, P., Tikoo, R., Kiyokawa, H., & al., e. (1997). Oligodendrocyte precursor differentiation is perturbed in the absence of the cyclin-dependent kinase inhibitor $p27^{Kip1}$. *Genes Dev.,* **11,** 2335-2346.

Catzavelos, C., Bhattacharya, N., Ung, Y. C., Wilson, J. A., Roncari, L., Sandhu, C., Yeger, H., Morava-Protzner, I., Kapusta, L., Franssen, E., Pritchard, K. I., & Slingerland, J. M. (1997). Decreased levels of the cell cycle inhibitor $p27^{kipl}$ protein: Prognostic implication in primary breast cancer. *Nature Med.,* **3***,* 227-230.

Catzavelos, C., Tsao, M. S., DeBoer, G., Bhattacharya, N., Shepherd, F. A., & Slingerland, J. M. (1999). Reduced expression of the cell cycle inhibitor $p27^{kip1}$ in non-small cell lung carcinoma: a prognostic factor independent of Ras. *Cancer Res.,* **59**, 684-688.

Cebon, J. MacGregor, D, Scott, A. and DeBoer, R. 1997. Australas J. Dermatol. 38; S66-72.

Chen, L. C., Matsumura, K., Deng, G., Kurisu, W., Ljung, B. M., Lerman, M. I., Waldman, F. M., & Smith, H. S. (1994). Deletion of two separate regions on chromosome 3p in breast cancers. *Cancer Res.,* **54***,* 3021-3024.

Chino, K., Esumi, M., Ishida, H., & Okada, K. (1999). Charactertic loss of heterozygosity in chromosome 3p and low frequency of replication errors in sporadic renal cell carcinoma. J. *Urol.,* **162,** 614-618.

Clurman, B. E., & Porter, P. (1998). New insights into the tumor suppression function of P27(kip1). *Proc. Natl. Acad. Sci. USA,* **95**, 15158-15160.

Cote, R. J., Shi, Y., Groshen, S., Feng, A. C., Cordon-Cardo, C., Skinner, D., & Lieskovosky, G. (1998). Association of $p27^{kipl}$ levels with recurrence and survival in patients with stage C prostate cancer. *J. Natl. Cancer Inst.,* **90**, 916-920.

Dalla-Favera, R., Yye, B. H., Le Coco, F., G., G., Lista, F., Knowles, D. M., Louie, D. C., Offit, K., & Chaganti, R. S. K. (1994). Identification of genetic lesions associated with diffuse large-cell lymphoma. Ann. Oncol., 5 Suppl, S55-S60.

Decker, H. J., Weidt, E. J., & Brieger, J. (1997). The von Hippel-Lindau tumor suppressor gene. A rare and intriguing disease opening new insight into basic mechanisms of carcinogenesis. *Cancer Genet. Cytogenet.,* **93**, 74-83.

Devilee, P., van den Broek, M., Mannens, M., Slater, R., Cornelisse, C. J., Westerveld, A., & Khan, P. M. (1991). Differences in patterns of allelic loss between two common types of adult cancer, breast and colon carcinoma, and Wilms' tumor of childhood. *Int. J. Cancer,* **47**, 817-821.

Devillee, P., van den Broek, M., Kuipers-Dijkshoorn, N., Kolluri, R., Meera Khan, P., Pearson, P. L., & Cornelisse, C. J. (1989). At least four different chromosomal regions are involved in loss of heterozygosity in human breast carcinoma. *Genomics,* **5***,* 554-560.

Dietrich, C. U., Pandis, N., Teixeira, M. R., Bardi, G., Gerdes, A. M., Anderson, J. A., & Heim, S. (1995). Chromosome abnormalities in benign hyperproliferative disorders of epithelial and stromal breast tissue. *Int. J. Cancer,* **60**, 49-53.

Durand, B., Gao, F. B., & Raff, M. (1997). Accumulation of the cyclin-dependent kinase inhibitor $P27^{Kip1}$ and the timing of oligodendrocyte differentiation. *EMBO J.,* **16**, 306-317.

Eiriksdottir, G., Barkardottir, R. B., Agnarsson, B. A., Johannesdottir, G., Olafsdottir, K., Egilsson, V., & Ingvarsson, S. (1998). High incidence of loss of heperozygosity at chromosome 17p13 in breast tumors from BRCA2 mutation carriers. *Oncogene,* **16**, 21-26.

Emilion, G., Langdon, J. D., Speight, P., & Partridge, M. (1996). Frequent gene deletions in potentially malignant oral lesions. *Br. J. Cancer,* **73**, 809-813.

Esposito, V., Baldi, A., De-Luca, A., Groger, A. M., Loda, M., Giordano, G. G., Caputi, M., Baldi, F., Pagano, M., & Giordano, A. (1997). Prognostic role of the cyclin-dependent kinase inhibitor p27 in non-small cell lung cancer. *Cancer Res.,* **57**, 3381-3385.

Euhus, D. M., Maitra, A., Wistuba, I. I., Alberts, A., Albores-Saavedra, J., & Gazdar, A. F. (1999). Loss of heterozygosity at 3p in benign lesions preceding invasive breast cancer. *J. Surg. Res.,* **83**, 13-18.

Eyfjord, J. E., Thorlacius, S., Steinarsdottir, M., Valgardsdottir, R., Ogmundsdottir, H. M., & Anamthawat-Jonsson, K. (1995). p53 abnormalities and genomic instability in primary human breast carcinoma. *Cancer Res.,* **55**, 646-651.

Florenes, V. A., Maelandsmo, G. M., Kerbel, R. S., Slingerland, J. M., Nesland, J. M., & Holm, R. (1998). Protein expression of the cell-cycle inhibitor $p27^{Kip1}$ in malignant melanoma. *Am. J. Path.,* **153**, 305-312.

Foster, K., Crossey, P. A., Cairns, P., Hetherington, J. W., Richards, F. M., Jones, M. H., Bentley, E., Affara, N. A., Ferguson-Smith, M. A., & Maher, E. R. (1994). Molecular genetic investigation of sporadic renal cell carcinoma: Analysis of allele loss on chromosomes 3p, 5q, 11p, 17 and 22. *Br. J. Cancer, 69,* 230-234.

Fouret, P. J., Dabit, D., Mergui, J.-L., & Uzan, S. (1998). Loss of heterozygosity on the short arm of chromosome 3 in cervical intro-epithelial neoplasia without concomitant cervical carcinoma. *Pathobiol.,* **66**, 306-310.

Fredersdorf, S., Burns, J., Milne, A. M., Packham, G., Fallis, L., Gillett, C. E., Royds, J. A., Peston, D., Hall, P. A., Hanby, A. M., Barnes, D. M., Shousha, S., O-Hare, M. J., & Lu, X. (1997). High level expression of $p27^{kip1}$ and cyclin D1 in some human breast cancer cells: Inverse correltation between the expression of $p27^{kip1}$ and degree of malignancy in human breast and colorectal cancer. *Proc. Natl. Acad. Sci. USA,* **94,** 6380-6385.

Fullwood, P., Marchini, S., Rader, J. S., Martinez, A., Macartney, D., Broggini, M., Morelli, C., Barbanti-Brodano, G., Maher, E. R., & Latif, F. (1999). Detailed genetic and physical mapping of tumor suppressor loci on chromosome 3p in ovarian cancer. *Cancer Research,* **59**, 4662-4667.

Gazdar, A., Bader, S., Hung, J., Kishimoto, Y., Sekido, Y., Sugio, K., Virmani, A., Fleming, J., Carbone, D., & Minno, J. D. (1994). Molecular genetic changes found in human lung cancer and its precursor lesions. *Cold Spring Harb. Symp. Quant. Biol.,* **59**, 565-572.

Gazdar, A. F., Kurvari, V., Virmani, A., Gollahon, L., Sakaguchi, M., Westerfield, M., Kodagoda, D., Stasny, V., Cunningham, H. T., Wistuba, I. I., Tomlinson, G., Tonk, V., Ashfaq, R., Leitch, A. M., Minna, J. D., & Shay, J. W. (1998). Characterization of paired tumor and non-tumor cell lines established from patients with breast cancer. *Int. J. Cancer,* **78**, 766-774.

Ginsberg, R. J., Kris, M. G., & Armstrong, J. G. (1993). Non-small cell lung cancer. In V. T. De Vita Jr., S. Hellman, & S. A. Rosenberg (Eds.), Cancer: Principles and Practise of Oncology. (pp. 673-723). Philadelphia: Lippincott, J.B.

Gorunova, L., Hoglund, M., Andren-Sandberg, A., Dawiskiba, S., Jin, Y., Mitelman, F., & Johansson, B. (1998). Cytogenetic analysis of pancreatic carcinomas: intratumor heterogeneity and nonrandom pattern of chromosome aberrations. *Genes Chrm. Cancer,* **23**, 81-99.

Grati, F. R., Sirchia, S. M., Garagiola, I., Sironi, E., Galioto, S., Rossella, F., Serafini, P., Dulcetti, F., Bozzetti, A., Brusati, R., & Simoni, G. (2000). Losses of heterozygosity in oral and oropharyngeal epithelial carcinomas. *Cancer Genet. Cytogenet.,* **118***,* 57-61.

Hahn, S. A., Schutte, M., Hoque, A. T., Moskaluk, C. A., da Costa, L. T., Rozenblum, E., Weinstein, C. L., Fischer, A., Yeo, C. J., Hruban, R. H., & Kern, S. E. (1996). DPC4, a candidate tumor suppressor gene at human chromosome 18q21.1. *Science,* **271**, 350-353.

Harris, N. L., Jaffe, E. S., Stein, H., Banks, P. M., Chan, J., Cleary, M. L., Delsol, G., Dewolfpeeters, C., Falini, B., Gatter, K. C., Grogan, T. M., Isaacson, P. G., Knowles, D. M., Mason, D. Y., Müller-Hermelink, H. K., Pileri, S. A., Piris, M. A., Ralfkiaer, E., & Warnke, R. A. (1994). A revised european-american classification of lymphoid neoplasms - a proposal from the international lymphoma study-group. Blood, 84, 1361-1392.

Hazaczek, P., Podolski, J., Toloczko, A., Kurzawski, G., Sikorski, A., Rabbitts, P., Huebner, K., & Lubinski, J. (1996). Losses at 3p common deletion sites in subtypes of kidney tumours: histopathological correlations. *Virchows Arch.,* **429,** 37-42.

Hengst, L., Dulic, V., Slingerland, J., & al., e. (1994). A cell cycle regulated inhibitor of cyclin dependent kinases,. *Proc. Natl. Acad. Sci. USA,* **91***,* 5291-5294. ,

Hengst, L., & Reed, S. I. (1996). Translational control of $p27^{KIP1}$ accumulation during the cell cycle. *Science,* 271, 1861-1864.

Hernandez, S., Hernandez, L., Bea, S., Cazorla, M., Fernandez, P. L., Nadal, A., Muntane, J., Mallofre, C., Montserrat, E., Cardesa, A., & Campo, E. (1998). *cdc25* cell cycle-activating phosphatases and *c-myc* expression in human non-Hodgkin's lymphomas. *Cancer Res.,* **58,** 1762-1767.

Hibi, K., Takahashi, T., Yamakawa, K., Ueda, R., Sekido, Y., Ariyoshi, Y., Suyama, M., Takagi, H., Nakamura, Y., & Takahashi, T. (1992). Three distinct regions involved in 3p deletion in human lung cancer. *Oncogene,* **7**, 445-449.

Hughson, M. D., Meloni, A., Dougherty, S., Silva, F. G., & Sandberg, A. (1996). Analysis of 3p allelic loss in papillary and nonpapillary renal cell carcinoma. Correlation with tumor karyotypes. *Cancer Genet. Cytogenet.,* **87,** 133-139.

Hunter, T., & Pines, J. (1994). Cyclins and cancerII: cyclin D and cdk inhibitors come of age. *Cell,* **79,** 573-582.

Ito, R., Yasui, W., Ogawa, Y., Toyosawa, S., Tahara, E., & Ijuhin, N. (1999). Reduced expression of cyclin-dependent kinase inhibitor p27(Kip1) in oral malignant tumors. *Pathobiol.,* **67,** 169-173.

Jiang, G. L., Liu, L. M., Buyse, I. M., Simon, D., & Huang, S. (1999). Decreased RIZ1 expression but not RIZ2 in hepatoma and suppression of hepatoma tumorigenicity by RIZ1. *Int. J. Cancer,* **83,** 541-546.

Johansson, B., Billström, R., Kristoffersson, U., Åkerman, M., Garwicz, S., Ahlgren, T., Malm, C., & Mitelman, F. (1997). Deletion of chromosome arm 3p in hematologic malignancies. *Leukemia,* **11**, 1207-1213.

Jones, M. H., & Nakamura, Y. (1992). Deletion mapping of chromosome 3p in female genital tract malignancies using microsatellite polymorphism. *Oncogene*, **7**, 1631-1634.

Jordan, R. C., Bradley, G., & Slingerland, J. (1998). Reduced levels of the cell-cycle inhibitor p27$^{kip1}$ in epitheial dysplasia and carcinoma of the oral cavity. *Am. J. Pathol.,* **152**, 585-590.

Kaestner, K. H., Knüchel, W., & Martinez, D. E. (2000). Unified nomenclature for the winged helix/forkhead transcription factors. *Genes & Development,* **14**, 142-146.

Katayose, Y., Kim, M., Rakkar, A. N., Li, Z., Cowan, K. H., & Seth, P. (1997). Promoting apoptosis: A novel activity associated with the cyclin-dependent kinase inhibitor p27. *Cancer Res.,* **57**, 5441-5445.

Kato, J.-Y., Matsuoka, M., Polyak, K., Massague, J., & Sherr, C. J. (1994). Cyclic AMP-induced G1 phase arrest mediated by an inhibitor (p27$^{Kip1}$) of cyclin-dependent kinase 4 activation. *Cell,* **79**, 487-496.

Kawamata, N., Morosettie, R., Miller, C. W., Park, D., Spirin, K. S., Nakamaki, T., Takeuchi, S., Hatta, Y., Simpson, J., & Wilczynski, S. (1995). Molecular analysis of the cyclin-dependent kinase inhibitor p27/Kip1 in human malignancies. *Cancer Res.,* **55,** 2266-2269.

Kiyokawa, H., Kineman, R. D., Manova-Todorova, K. O., Soares, V. C., Hoffman, E. S., Ono, M., Khanam, D., Hayday, A. C., Frohman, L. A., & Koff, A. (1996). Enhancer growth of mice lacking the cyclin-dependent kinase inhibitor function of p27 (Kip1). *Cell,* **85,** 721-732.

Knudson, A. G. (1971). Mutation and cancer: statistical study of retinoblastoma. *Proc. Natl. Acad. Sci. USA,* **68,** 820-823.

Koff, A., Ohtsuki, M., Polyak, K., Roberts, J. M., & Massague, J. (1993). Negative regulation of G1 in mammalian cells: inhibition of cyclin E-dependent kinase by TGF-b. *Science,* **260**, 536-539.

Kok, K., Naylor, S. L., & Buys, C. H. (1997). Deletions of the short arm of chromosome 3 in solid tumors and the search for suppressor genes. *Adv. Cancer Res.,* **71**, 27-92.

Kolluri, S. K., Weiss, C., Koff, A., & Gottlicher, M. (1999). p27(Kip1) induction and inhibition of proliferation by the intracellular Ah receptor in developing thymus and hepatoma cells [In Process Citation]. *Genes Dev.,* **13**, 1742-1753.

Kops, G.J. and Burgering, B.M. (1999). Forkhead Transcription Factors: new insights into protein kinase B (c-akt) signalling. *J. Mol. Med.* 77 656-665.

Kovacs, G. (1993). Molecular cytogenetics of renal cell tumors. *Adv. Cancer Res.,* **62**, 89-124.

Kovacs, G. (1993). Molecular differential pathology of renal cell tumours. *Histpath.,* **22,** 1-8.

Koyama, H., Raines, E. W., Bornfeldt, K. E., Roberts, J. M., & Ross, R. (1996). Fibrillar collagen inhibits arterial smooth muscle proliferation through regulation of cdk2 inhibitors. *Cell,* **87**, 1069-1078.

Le Beau, M. M., Drabkin, H., Glover, T. W., Gemmill, R., Rassool, F. V., McKeithan, T. W., & Smith, D. I. (1998). An FHIT tumor suppressor gene? *Genes Chrm. Cancer,* **21**, 281-289.

Kwak, L., Wilson, M., Weiss, L. M., Horning, S. J., Warnke, R. A., & Dorfman, R. F. (1991). Clinical significance of morphological subdivision in diffuse large B cell lymphoma. Cancer, 68, 1988-1993.

Lennert, K., Mohri, N., Stein, H., & Kaiserling, F. (1975). The histopathology of malignant lymphoma. Br. J. Haematol., 31(Suppl), 193-203.

Li, J., Yen, C., Liaw, D., Podsypanina, K., Bose, S., Wang, S. I., Pue, J., Miliaresis, C., Rodgers, L., McCombie, R., Bigner, S. H., Giovanella, B. C., Ittman, M., Tycko, B., Hibshoosh, H., Wigler, M. H., & Parsons, R. (1997). Pten, a putative protein tyrosine phosphatase gene mutated in human, brain, breast and prostate cancer. *Science,* **275**, 1943-1947.

Li, X., Lee, N. K., Waber, P. G., Schweitzer, C., Cheng, Q. C., & Nisen, P. D. (1994). Allelic loss at chromosomes 3p, 8p, 13q and 17p associated with poor prognosis in head and neck cancer. J. *Natl. Cancer Inst.,* **86**, 1524-1529.

Loda, M., Cukor, B., Tam, S. W., Lavin, P., Fiorentino, M., Draetta, G. F., Jessup, J. M., & Pagano, M. (1997). Increased proteasome-dependent degradation of the cyclin-dependent kinase inhibitor p27 in aggressive colorectal carcinomas. *Nature Med.,* 3, 231-234.

Lott, S. T., Lovell, M., Naylor, S. L., & Killary, A. M. (1998). Physical and functional mapping of a tumor suppressor locus for renal cell carcinoma within chromosome 3p12. *Cancer Res.,* **58,** 3533-3537.

Lovell, M., Lott, S. T., Wong, P., El-Naggar, A., Tucker, S., & Killary, A. M. (1999). The genetic locus NRC-1 within chromosome 3p12 mediates tumor suppression in renal cell carcinoma independently of histological type, tumor microenvironment, and VHL mutation. *Cancer Res.,* **59**, 2182-2189.

Lubinski, J., Hadaczek, P., Podolski, J., Toloczko, A., Sikorski, A., McCue, P., Druck, T., & Huebner, K. (1994). Common regions of deletion in chromosome regions 3p12 and 3p14.2 in primary clear cell renal carcinomas. *Cancer Res.,* **54**, 3710-3713.

Maestro, R., Gasparotto, D., Vukosavljevic, T., Barzan, L., Sulfaro, S., & Boiocchi, M. (1993). Three discrete regions of deletion at 3p in head and neck cancers. *Cancer Res,* **53,** 5775-5779.

Medema, R. H., Kops, G. J. P. L., Bos, J. L., & Burgering, B. M. T. (2000). AFX-like Forkhead. transcription factors mediate cell-cycle regulation by Ras and PKB through $p27^{kipl}$. *Nature,* **404,** 782-787.

Mehle, C., Lindblom, A., Ljunberg, B., Stenling, R., & Roos, G. (1998). Loss of heterozygosity at chromosome 3p correletes with telomerase activity in renal cell carcinoma. *Int. J. Oncol.,* **13**, 289-295.

Millard, S. S., Yan, J. S., Nguyen, H., Pagano, M., Kiyokawa, H., & Koff, A. (1997). Enhanced ribosomal association of p27(Kip1) mRNA is a mechanism contributing to accumulation during growth arrest. J. *Biol. Chem.,* **272,** 7093-7098.

Mineta, H., Miura, K., Suzuki, I., Takebayashi, S., Misawa, K., Ueda, Y., & Ichimura, K. (1999). p27 expression correlates with prognosis in patients with hypopharyngeal carcinoma. *Anticancer Res.,* **19,** 4407-4412.

Mitelman, F., Mertens, F., & Johansson, B. (1997). A breakpoint map of recurrent chromosomal rearrangements in human neoplasia. *Nat. Genet.,* **15**, 417-474.

Moller, M. B., Skjodt, K., Mortensen, L. S., & Pedersen, N. T. (1999). Clinical significance of cyclin-dependent kinase inhibitor $p27^{kip1}$ expression and proliferation in non-Hodgkin's lymphoma:independent prognostic value of $p27^{kip1}$. *Br. J. Haematol.,* **105**, 730-736.

Mori, M., Mimori, K., Shiraishi, T., Tanaka, S., Ueo, H., Sugimachi, K., & Akiyoshi, T. (1997). p27 expression and gastric carcinoma. *Nature Med.,* 3, 593.

Morosetti, R., Kawamata, N., Gombart, A. F., Miller, C. W., Hatta, Y., Hirama, T., Said, J. W., Tomonaga, M., & Koeffler, H. P. (1995). Alterations of the $p27^{kip1}$ gene in non-Hodgkin's lymphomas and adult T-cell leukemia/ lymphoma. *Blood,* **86**, 1924-1930.

Nguyen, H., Gitig, D. M., & Koff, A. (1999). Cell-free degradation of p27(kip1), a G1 cyclin-dependent kinase inhibitor, is dependent on CDK2 activity and the proteasome. *Mol. Cell. Biol.,* **19**, 1190-1201.

Nigg, E. (1991). The substrates of the cdc2 kinase. *Semin. Cell. Biol.,* **2**, 261-270.

Nourse, J., Firpo, E., Flanagan, W. M., Coats, S., Polyak, K., Lee, M.-H., Massague, J., Crabtree, G. R., & Roberts, J. M. (1994). Interleukin-2-mediated elimination of the $p27^{KIP1}$ cyclin-dependent kinase inhibitor prevented by rapamycin. *Nature*, **372**, 570-573.

The Non-Hodgkin's Lymphoma Classification Project. (1997). A clinical evaluation of the international lymphoma study group classification of non-Hodgkin's lymphoma. Blood, 89, 3909-3918.

Orend, G., Hunter, T., & Ruoslanhti, E. (1998). Cytoplasmic displacement of cyclin E-cdk2 inhibitors p21Cip1 and $p27^{kip1}$ in anchorage-independent cells. *Oncogene,* 16, 2575-2583.

Pagano, M., Tam, S. W., Theodoras, A. M., Romero, P. B., Sal, G. D., Chau, V., Yew, P. R., Draetta, G. F., & Rolfe, M. (1995). Role of the ubiquitin-proteasome in regulating abundance of the cyclin-dependent kinase inhibitor p27. *Science,* **269**, 682-685.

Pandis, N., Jim, Y., Limon, J., Bardi, G., Idvall, I., Mandahl, N., Mitelman, F., & Heim, S. (1993). Interstitial deletion of the short arm of chromosome 3 as a primary chromosome abnormality in carcinomas of the breast. *Genes Chrm. Cancer,* **6**, 151-155.

Parry, D., Bates, S., Mann, D. J., & Peters, G. (1995). Lack of cyclinD-cdk complexes in Rb-negative cells correlates with high levels of p16INK4/MTS1 tumour suppressor gene product. *EMBO J.,* **14,** 503-511.

Partridge, M., Emilion, G., Pateromichelakis, S., A'Hern, R., Lee, G., Phillips, E., & Langdon, J. (1999). The prognostic significance of allelic imbalance at key chromosomal loci in oral cancer. *Br. J. Cancer,* **79,** 1821-1827.

Pietenpol, J. A., Bohlander, S. K., Sato, Y., Papadopoulos, N., Liu, B., Friedman, C., Trask, B. J., Roberts, J. M., Kinzler, K. W., Rowly, J. D., & Vogelstein, B. (1995). Assignment of the human $p27^{kip1}$ gene to 12p13 and its analysis in leukemias. *Cancer Res.,* **55,** 1206-1210.

Polyak, C., Lee, M.-H., Erdjument-Bromage, H., Koff, A., Roberts, J. M., Tempst, P., & Massague, J. (1994). Cloning of $p27^{kip1}$, a cyclin-dependent kinase inhibitor and a potential mediator of extracellular antimitogenic signals. *Cell,* **78,** 59-66.

Polyak, K., Kato, J. Y., Solomon, M. J., Sherr, C. J., Massague, J., Roberts, J. M., & Koff, A. (1994). $p27^{Kip1}$, a cyclin-Cdk inhibitor, links transforming growth factor-b and contact inhibition to cell cycle arrest. *Genes Dev.,* **8**, 9-22.

Porter, P. L., Malone, K. E., Heagerty, P. J., Alexander, G. M., Gatti, L. A., Firpo, E. J., Daling, J. R., & Roberts, J. M. (1997). Expression of cell cycle regulators p27 Kip1 and cyclin E, alone and in combination, correlate with survival in young breast cancer patients. *Nat. Med.,* **3**, 222-225.

Presti Jr., J. C., Reuter, V. E., Cordon-Cardo, C., Mazumdar, M., Fair, W. R., & Jhanwar, S. C. (1993). Allelic deletions in renal tumors: histopathological correlations. *Cancer Res.,* **53**, 5780-5783.

Quintanilla-Martinez, L., Thieblemont, C., Fend, F., Kumar, S., Pinyol, M., Campo, E., Jaffe, E., & Raffeld, M. (1998). Mantle cell lymphomas lack expression of $p27^{kip1}$ a cyclin-dependent kinase inhibitor. Am. J. *Pathol.,* **153**, 175-182.

Rabbitts, P., Bergh, J., Douglas, J., Collins, F., & Waters, J. (1990). A submicroscopic homozygous deletion at the D3S3 locus in a cell line isolated from a small cell lung carcinoma. *Genes Chrm. Cancer,* **2**, 231-238.

Roz, L., Wu, C. L., Porter, S., Scully, C., Speight, P., Read, A., Sloan, P., & Thakker, N. (1996). Allelic imbalance on chromosome 3p in oral dysplastic lesions: an early event in oral carcinogenesis. *Cancer Res.,* **56,** 1288-1231.

Ruas, M., & Peters, G. (1998). The p16INK4a/CDKN2A tumor suppressor and its relatives. *Biochim. Biophys. Acta,* **1378,** F115-177.

Sáez, A., Sánchez, E., Sánchez-Beato, M., Cruz, M. A., Chacón, I., Munoz, E., Camacho, F. I., Martinez-Montero, J. C., Mollejo, M., Garcia, J. F., & Piris, M. A. (1999) . $p27^{KIP1}$ is abnormally expressed in Diffuse Large B-cell Lymphomas and is associated with an adverse clinical outcome. *Br. J. Cancer,* **80**(9), 1427-1434.

Sambrook, J., Fritsch, E. F., & Maniatis, T. (1989). Molecular cloning. A laboratory manual. Cold Spring Harbor: Cold Spring Harbor Laboratory Press.

Stein, H., & Dallenbach, F. (1992). Diffuse large cell lymphomas of B and T cell type. In D. M. Knowles (Eds.), Neoplastic Hematopathology. (pp. 675-714). Baltimore: Williams & Wilkins.

Sanchez, Y., El-Naggar, A., Pathak, S., & Killary, A. M. (1994). A tumor suppressor locus within 3p14-p12 mediates rapid cell death of renal cell carcinomas in vivo. *Proc. Natl. Acad. Sci. USA,* **91,** 3383-3387.

Sanchez-Beato, M., Camacho, F. I., Martinez-Montero, J. C., Saez, A. I., Villuendas, R., Sanchez-Verde, L., Garcia, J. F., & Piris, M. A. (1999). Anomalous high $p27^{kip1}$ expression in a subset of aggressive B-cell lymphomas is associated with cyclin D3 overexpression. p27/KIP1-cyclin D3 colocalization in tumor cells. *Blood,* **94**, 765-772.

Sanchez-Beato, M., Sáez, A. I., Martinez-Montero, J. C., Mateo, M. S., Sánchez-Verde, L., Villuendas, R., Troncone, G., & Piris, M. A. (1997). Cyclin-dependent kinase inhibitor $p27^{KIP1}$ in lymphoid tissue: $p27^{KIP1}$ expression is inversely proportional to the proliferative index. *Am. J. Pathol.,* **151,** 151-160.

Sandhu, C., Garbe, J., Daksis, J., & al, e. (1997). TGF-b stabilizes p15INK4B protein, increases p15INK4B/cdk4 complexes and inhibits cyclin D1/cdk4 association in human mammary epithelial cells. *Mol. Cell Biol.,* **17**, 2458-2467.

Sato, T., Saito, H., Morita, R., Koi, S., Lee, J. H., & Nakamura, Y. (1991). Allelotype of human ovarian cancer. *Cancer Res.,* **51**, 5118-5122.

Sekido, Y., Ahmadian, M., Wistuba, I. I., Latif, F., Bader, S., Wei, M.-H., Duh, F.-M., Gazdar, A. F., Lerman, M. I., & Minna, J. D. (1998). Cloning of a breast cancer homozygous deletion junction narrows the region of search for a 3p21.3 tumor suppressor gene. *Oncogene,* **16**, 3151-3157.

Sezinger, B. R., Klinger, H. P., Junien, C., Nakamura, Y., Le Beau, M., Cavence, W., Emanuel, B., Ponder, B., Naylor, S., Mitelman, F., Louis, D., Menon, A., Newsham, I., Decker, J., Kaelbling, M., Henry, I., & von Deimling, A. (1991). Report of the committee on chromosome and gene loss in human neoplasia. *Cytogenet. Cell. Genet.,* **58**, 1080-1096.

Sgambata, A., Zhang, Y., Arber, N., Hibshoosh, H., Doki, Y., Ciaparrone, M., Santella, R. M., Cittadini, A., & Weinstein, I. B. (1997). Deregulated expression of $p27^{kip1}$ in human breast cancers. *Clin. Cancer Res.,* **3**, 1879-1887.

Shamma, A., Doki, Y., Tsujinaka, T., Shiozaki, H., Inoue, M., Yano, M., Kawanishi, K., & Monden, M. (2000). Loss of $p27^{kip1}$ expression predicts poor prognosis in patients with esophageal squamous cell carcinoma. *Oncol.,* **58,** 152-158.

Sherr, C. J., & Roberts, J. M. (1995). Inhibitors of mammalian G1 cyclin-dependent kinases. *Genes Dev.,* ***9,*** 1149-1163. '

Sherr, J. S. (1996). Cancer cell cycles. *Science,* **274**, 1672-1677.

Singh, S. P., Lipman, J., Goldman, H., Ellis, F. H., Aiseman, L., Cangi, M. G., Signoretti, S., Chiaur, D. S., Pagano, M., & Loda, M. (1998). Loss or altered subcellular localization of p27 in Barrett's associated adenocarcinoma. *Cancer Res.,* **58,** 1730-1735.

Slingerland, J. M., Hengst, L., Pan, C.-H., &'al, e. (1994). A novel inhibitor of cyclin-cdk activity detected in Transforming Growth Factor b-arrested epithelial cells. *Mol. Cell Biol.,* **14**, 3683-3694.

Songyang, Z., Blechner, S., Hoagland, N., Hoekstra, M. F., Piwnica-Worms, H., & Cantley, L. C. (1994). Use of an orientated peptide library to determine the optimal substrates of protein kinases. *Curr. Biol.,* **4,** 973-982.

Soucek, T., Yeung, R. S., & Hengstschlager, M. (1998). Inactivation of the cyclin-dependent kinase inhibitor p27 upon loss of the tuberous sclerosis complex gene-*2. Proc. Natl. Acad. Sci. USA,* **95,** 15653-15658.

Srinivasan, J., Koszelak, M., Mendelow, M., Kwon, Y.-G., & Lawrence, D. S. (1995). The design of peptide based substrates for the cdc2 protein kinase. *Biochem.* J., 309, 927-931.

St Croix, B., Florenes, V. A., Rak, J. W., Flanagan, M., Bhattacharya, N., Slingerland, J. M., & Kerbel, R. S. (1996). Impact of the cyclin-dependent kinase inhibitor p27$^{kip1}$ on resistance of tumor cells to anticancer agents. *Nat. Med.,* **2**, 1204-1210.

Steck, P. A., Perhouse, M. A., Jasser, S. A., 'Yung, W. K. A., Lin, H., Ligon, A. H., Langford, L. A., Baumgard, M. L., Hattier, T., Davis, T., Frye, C., Hu, R., Swedlund, B., Teng, D. H. F., & Tavtigian, S. V. (1997). Identification of a candidate tumour suppressor gene, Mmac1, at chromosome 10q23.3 that is mutated in some advanced cancers. *Nature Genet.,* **15***,* 356-362.

Stein, H., & Dallenbach, F. (1992). Diffuse large cell lymphomas of B and T cell type. In D.M. Knowles (Eds.), Neoplastic Hematopathology. (pp 675-714). Baltimore: Williams & Wilkins.

Sundaresan, V., Heppell-Parton, A., Coleman, N., Miozzo, M., Sozzi, G., Ball, R., Cary, N., Hasleton, P., Fowler, W., & Rabbitts, P. (1995). Somatic genetic changes in lung cancer and precancerous lesions. *Ann. Oncol.,* **6 (Suppl. 1),** S27-S32.

Tan, P., Cady, B., Wanner, M., Worland, P., Cukor, B., Magi-Galluzzi, C., Lavin, P., Draetta, G., Pagano, M., & Loda, M. (1997). The cell cycle inhibitor p27 is an independent prognostic marker in small (T1a, b) invasive breast carcinomas. *Cancer Res.,* **57,** 1259-1263.

Tenjo, T., Toyoda, M., Okuda, J., Watanabe, I., Yamamoto, T., Tanaka, K., Ohtani, M., Nohara, T., & Kawasaki, H. (2000). Prognostic significance pf p27(kip1) protein expression and spontaneous apoptosis in patients with colorectal adenocarcinomas. *Oncol.,* **58**, 45-51.

Texeira, L. T., Kiyokawa, H., Peng, X. D., Christov, K. T., Frohman, L. A., & Kineman, R. D. (2000). p27$^{kip1}$-deficient mice exhibit accelerated growth hormone-releasing hormone (GHRH)-induced somatotrope proliferation and adenoma formation. *Oncogene, 19,* 1875-1884.

Timmer, T., Terpstra, P., vandenBerg, A., Veldhuis, P. M. J. F., TerElst, A., Voutsinas, G., Hulsbeek, M. M. F., Draaijers, T. G., Looman, M. W. G., Kok, K., Naylor, S. L., & Buys, C. H. C. M. (1999). A comparison of genomic structures and expression patterns of two closely related flanking genes in a critial lung cancer region at 3p21.3. *Eur. J. Human Genet.,* **7**, 478-486.

Todd, S., Franklin, W. A., Varella-Garcia, M., Kennedy, T., Hilliker Jr., C. E., Hahner, L., Anderson, M., Wiest, J. S., Drabkin, H. A., & Gemmill, R. M. (1997). Homozygous deletions of human chromosome 3p in lung tumors. *Cancer Res.,* 57, 1344-1352.

Tseng, S.-L., Yu, J.-C., Yue, C.-T., Chang, S.-F., Chang, T.-M., Wu, C.-W., & Shen, C.-Y. (1997). Allelic loss of BRCA1, and BRCA2, and adjacent loci in relation to TP53 abnormality in breast cancer. *Genes Chrm. Cancer,* **20**, 377-382.

Tsihlias, J., Kapusta, L., & Slingerland, J. (1999). The prognostic significance of altered cyclin-dependent kinase inhibitors in human cancer. *Ann. Rev. Med.,* **50**, 401-423.

Tsihlias, J., Kapusta, L. R., DeBoer, G., Morava-Protzner, I., Zbieranowski, I., Bhattacharya, N., Catzavelos, G. C., Klotz, L. H., & Slingerland, J. M. (1998). Loss of cyclin dependent kinase inhibitor p27$^{Kip}$1 is a novel prognostic factor in localized human prostate adenocarcinoma. *Cancer Res.,* **58,** 542-548.

Uzawa, N., Yoshida, M. A., Hosoe, S., Oshimura, M., Amagasa, T., & Ikeuchi, T. (1998). Functional evidence for involvement of multiple putative tumor suppressor genes on the short arm of chromosome 3 in human oral squamous cell carcinogenesis. *Cancer Genet. Cytogenet.,* **107**, 125-131.

van der Lugt, N. M. T., Domen, J., Linders, K., van Roon, M., Robanus-Maandag, E., te Riele, H., van der Valk, M., Deschamps, J., Sofroniew, M., van Lohuizen, M., & al, e. (1994). Posterior transformation, neurological abnormalities, and severe hematopoietic defects in mice with a targeted deletion of the proto-oncogene. *Genes Dev.,* **8,** 757-769.

Vidal, A., & Koff, A. (2000). Cell-cycle inhibitor: three families united by a common cause. *Gene,* **247,** 1-15.

Virmani, A. K., Fong, K. M., Kodagoda, D., McIntire, D., Hung, J., Tonk, V., Minna, J. D., & Gazdar, A. F. (1998). Allelotyping demonstrates common and distinct patterns of chromosomal loss in human lung cancer types. *Genes Chrm. Cancer,* **21,** 308-319.

Vlach, J., Hennecke, S., Alevizopoulos, K., Conti, D., & Amati, B. (1996). Growth arrest by the cyclin-dependent kinase inhibitor $p27^{Kip1}$ is abrogated by c-myc. *EMBO J.,* **15**, 6595-6604.

Vlach, J., Hennecke, S., & Amati, B. (1997). Phosphorylation-dependent degradation of the cyclin-dependent kinase inhibitor $p27^{Kip1}$. *EMBO J.,* **16,** 5334-5344.

Vrhorac, R., Delmer, A., Tang, R., Marie, J. P., Zittoun, R., & Ajchenbaum-Cymbalista, F. (1998). Prognostic significance of the cell cycle inhibitor $p27^{kip1}$ in chronic lymphocytic leukemia. *Blood*, **91**, 4694-4700.

Wang, X., Gorospe, M., Huang, Y., & Holbrook, N. J. (1997). $p27^{kip1}$ overexpression causes apoptotic death of mammalian cells. *Oncogene,* **15**, 2991-2997.

Willers, C. P., Siebert, R., Bardenheuer, W., Lux, A., Michaelis, S., Seeber, S., Luboldt, H.-J., Opalka, B., & Schütte, J. (1996). Genetic instability of 3p12-p21-specific microsatellite sequences in renal cell carcinoma. *Br. J. Urol.,* **77**, 524-529.

Wistuba, I. I., Behrens, C., Virmani, A. K., Mele, G., Milchgrub, S., Girard, L., Fondon, J. W. 3., Garner, H. R., McKay, B., Latif, F., Lerman, M. I., Lam, S., Gazdar, A. F., & Minna, J. D. (2000). High resolution chromosome 3p allelotyping of human lung cancer and preneoplastic/preinvasive bronchial epithelium reveals multiple, discontinuous sites of 3p allele loss and three regions of frequent breakpoints. *Cancer Res.,* **60**, 1949-1960.

Yamasaki, L. (1998). Growth regulation by the E2F and DP transcription factor families. *Results Probl. Cell Differ. ,* **22,** 199-227.

Yang, R. M., Naitoh, J., Murphy, M., Wang, H. J., Phillipson, J., Dekernion, J. B., Loda, M., & Reiter, R. E. (1998). Low p27 expression predicts poor prognosis in patients with prostate cancer. *J. Urol.*, **159**, 941-945.

Yatabe, Y., Masuda, A., Koshikawa, T., Nakamura, S., Kuroishi, T., Osada, H., Takahashi, T., Mitsudomi, T., & Takahashi, T. (1998). $P27^{KIP1}$ in human lung cancers: differential changes in small-cell and non-small-cell carcinomas. *Cancer Res.,* **58,** 1042-1047.

Yokata, J., Tsukada, Y., Nakajima, T., Gotoh, M., Shimosato, Y., Mori, N., Tsunokawa, Y., Sugimura, T., & Terada, M. (1989). Loss of heterozygosity on the short arm of chromosome 3 in carcinoma of the uterine cervix. *Cancer Res.,* **49,** 3598-3601.

Yokozawa, T., Towatari, M., Iida, H., Takeyama, K., Tanimoto, M., Kiyoi, H., Motoji, T., Asou, N., Saito, K., Takeuchi, M., Kobayashi, Y., Miyawaki, S., Kodera, Y., Ohno, R., Saito, H., & Naoe, T. (2000). Prognostic significance of the cell cycle inhibitor $p27^{kip1}$ in acute myeloid leukemia. *Leuk.,* **14,** 28-33.

Zhang, J., Sanchez, R. J., Wang, S., Guarnaccia, C., Tossi, A., Zahariev, S., & Pongor, S. (1994). Substrate specificity of cdc2 kinase from human HeLa cells as determined with synthetic peptides and molecular modelling. *Biophys.,* **315,** 415-424.

Zhu, L., Harlow, E., & Dynlacht, B. D. (1995). p107 uses a p21CIP1-related domain to bind cyclin/cdk2 and regulate interactions with E2F. *Genes Dev.,* **9,** 1740-1752.

Zhu, X., Ohtsubo, M., Bohmer, R. M., Roberts, J. M., & Assoian, R. K. (1996). Adhesion-dependent cell cycle progression linked to the expression of cyclin D1, activation of cyclinE-cdk2, and phosphorylation of the retinoblastoma protein. *J. Cell. Biol.,* **133**, 391-403.

**Table 1. Reactivity of the JC12 Monoclonal Antibody on Normal Human Tissues.**

| Tissue Sample | Staining |
|---|---|
| Tonsil | Stains some nuclei in the germinal centre, much stronger nuclear staining of mantle zone cells. Nuclear and some cytoplasmic staining of epithelium. |
| Spleen | Mainly nuclear staining of some cells in germinal centre. Mantle zone mainly nuclear. Marginal zone negative. Weak cytoplasmic and occasional nuclear staining of cells in red pulp |
| Blood | The majority of white cells are positive |
| Thymus | Nuclear staining of most cells in the medulla and some cells in the cortex. |
| Lung | Macrophages show cytoplasmic staining |
| Testis | Strong cytoplasmic staining of spermatogonia and heterogeneous nuclear labelling of spermatozytes with some capping. |
| Kidney | Cytoplasmic staining of tubules and some glomeruli. Nuclear staining of occasional glomeruli. |
| Liver | Cytoplasmic staining of Kupfer cells and weak cytoplasmic staining of hepatocytes. |
| Large Bowel | Nuclear staining of crypt epithelium. |
| Cerebellum | Cytoplasmic staining of non neuronal cells in both grey and white matter. |
| Skin | Cytoplasmic and nuclear staining of epidermis and nuclear staining of sweat glands. |
| Ovary | Nuclear staining of stromal and plasma cells. |

**Table 2. Expression of QRF-2 protein in cell lines.**

| Cell Line | Disease | Staining |
|---|---|---|
| Erythroid | | |
| K562 | CML | Nuclear + some cytoplasmic |
| HEL | Erythroleukaemia | Punctate nuclear + cytoplasmic |
| T cell | | |
| HUT78 | Lymphoma | Nuclear with strong dots |
| Jurkat | Leukaemia | Punctate nuclear + nucleoli and weak cytoplasmic |
| SU-DHL-1 | T cell (ALCL t2;5) | Nuclear with strong dots + weak cytoplasmic |
| B cell | | |
| Nalm-1 | CML | Punctate nuclear |
| Reh | Leukaemia | Nuclear (but not nucleoli) + cytoplasmic |
| Daudi | Burkitt's lymphoma | Nuclear |
| Namalwa | Burkitt's lymphoma | Nuclear + very weak cytoplasmic |
| Thiel | Myeloma | Punctate nuclear + cytoplasmic |
| JOK1 | Hairy cell leukaemia | Punctate nuclear + nucleoli and weak cytoplasmic |
| Hodgkin's disease | | |
| L540 | | Nuclear + nucleoli and cytoplasmic |
| KM-H2 | | Punctate nuclear + cytoplasmic |
| Myeloid | | |
| HL60 | Acute leukaemia | Punctate nuclear + nucleoli and cytoplasmic |
| U937 | Histiocytic | Punctate nuclear |
| Carcinoma | | |

(continued)

| Cell Line | Disease | Staining |
|---|---|---|
| A431 | | Nuclear with dots + cytoplasmic |
| HeLa | Vulval | Punctate nuclear + cytoplasmic very strong |
| HT29 | Colon | Nuclear + cytoplasmic very strong |
| Other | | |
| Rh30 | Rhabdomyosarcoma | Nuclear with very strong dots + cytoplasmic |

**Table 3. Expression of QRF-2 protein in human neoplastic cells.**

| Tumour | Cases stained Positive | |
|---|---|---|
| **Carcinomas** | | |
| Basal carcinoma | 2 | 2 |
| Squamous cell carcinoma | 2 | 2 |
| Ductal carcinoma | 1 | 1 |
| Rhabdomyosarcoma | 2 | 2 |
| Neuroblastoma | 2 | 2 |
| **T Cell Lymphomas** | | |
| T cell lymphoma | 8 | 8 |
| Anaplastic large cell lymphoma | 9 | 9 |
| **B Cell Lymphomas** | | |
| Diffuse large B cell lymphoma | 25 | 25 |
| Classical Hodgkin's disease (HD) | 12 | 12 |
| HD lymphocyte predominance | 5 | 2 |
| Follicular lymphoma | 10 | 9 |
| Marginal zone B-cell lymphoma | 1 | 1 |
| Lymphoplasmacytoid lymphoma | 3 | 2 |
| Chronic lymphocytic leukaemia | 8 | - 8 |
| Burkitt's lymphoma | 6 | 6 |
| Mantle cell lymphoma | 4 | 4 |
| Hairy cell leukaemia | 5 | 5 |

## Claims

1. An isolated transcription factor protein comprising (i) a winged helix motif which has the potential capability of binding to nucleic acid and (ii) a $Cys_2$-$His_2$ zinc finger motif which also has potential nucleic acid binding capability.

2. A protein as claimed in claim 1 which further comprises at least one transcriptional activation domain.

3. A protein as claimed in claim 2, which protein comprises the amino acid sequence set forth in Figure 2 or an amino acid sequence which has an identity of at least 70% with the amino acid sequence set forth in Figure 2.

4. A protein as claimed in claim 1, which protein comprises the amino acid sequence set forth in Figure 3C or an amino acid sequence which has an identity of at least 80% with the amino acid sequence set forth in Figure 3C.

5. A protein as claimed in claim 1, which protein comprises the amino acid sequence set forth in Figure 4B or an amino acid sequence which has an identity of at least 90% with the amino acid sequence set forth in Figure 4B.

6. A protein as claimed in claim 1, which protein comprises the amino acid sequence set forth in Figure 4D or an amino acid sequence which has an identity of at least 95% with the amino acid sequence set forth in Figure 4D.

7. A protein as claimed in claim 3 which lacks the extreme N-terminal methionine.

8. An isolated nucleic acid molecule comprising a sequence of nucleotides which encodes the protein of any one of claims 1 to 7.

**9.** An isolated nucleic acid molecule comprising the sequence of nucleotides shown from position 264 or position 267 to position 2294 of the nucleic acid sequence set forth in Figure 2, or the sequence of nucleotides from position 16 to 2352 of the nucleic acid sequence set forth in Figure 2.

**10.** An isolated nucleic acid molecule comprising the sequence of nucleotides set forth in Figure 3A or 3B.

**11.** An isolated nucleic acid molecule comprising the sequence of nucleotides set forth in Figure 4A.

**12.** An isolated nucleic acid molecule comprising the sequence of nucleotides set forth in Figure 4C.

**13.** An expression vector comprising the nucleic acid of any one of claims 8 to 11.

**14.** A host cell transformed or transfected with the expression vector of claim 13.

**15.** An antibody which is capable of binding to the protein claimed in any one of claims 1 to 7 or an epitope thereof which is a monoclonal antibody JC12 obtainable from the hybridoma deposited with the European Collection of Cell Cultures under accession No. 99041425.

**16.** An antibody according to claim 15 for use in treatment of the human or animal body.

**17.** Use of an antibody according to claim 15 in the manufacture of a medicament for treating a disease or condition mediated or associated with expression or function of a protein according to any of claims 1 to 7.

**18.** A hybridoma deposited with the European Collection of Cell Cultures under accession No. 99041425.

**19.** An in vitro method of detecting expression of a protein comprising a winged helix motif and a $Cys_2$-$His_2$ zinc finger motif in a mammalian subject, which method comprises contacting a sample of tissue, cells or cell lysates removed from the mammalian subject with the antibody according to claim 18 and detecting specific binding of the antibody to its target protein in the said tissue.

**20.** A method as claimed in claim 19 wherein the antibody is a monoclonal antibody obtainable from the hybridoma deposited with the European Collection of Cell Cultures under accession No. 99041425.

**21.** A nucleic acid molecule according to any of claims 8 to 12, or a nucleic acid molecule capable of hybridising thereto under conditions of high stringency or a fragment thereof, for use in the treatment of a human or animal body.

**22.** Use of a nucleic acid molecule according to any of claims 8 to 12, or a nucleic acid molecule capable of hybridising thereto under conditions of high stringency or a fragment thereof, in the manufacture of a medicament for treating cancer.

**23.** A protein according to any of claims 1 to 7 or a derivative or fragment thereof for use in treating a human or animal body.

**24.** Use of a protein according to any of claims 1 to 7 or a derivative or fragment thereof in the manufacture of a medicament for treating cancer.

**25.** A pharmaceutical composition comprising any of an isolated protein according to any of claims 1 to 7, a nucleic acid molecule according to any of claims 8 to 12, or a nucleic acid capable of hybridising to said nucleic acid molecules under conditions of high stringency, an antibody according to claim 16, together with a pharmaceutically acceptable carrier, diluent or excipient therefor.

**26.** Use of an antibody according to claim 16 in the manufacture of a medicament for treating cancer.

**27.** An in vitro method of assessing the prognosis of premalignant lesions in a patient which method comprises detecting a change of expression pattern or function of a protein according to any of claims 1 to 7 in said patient, wherein a change of expression pattern or function of said protein is indicative of the likelihood of said patient's premalignant lesions developing into a malignant tumour.

**28.** A method according to claim 27 wherein said change of expression pattern or function of said protein occurs because of changes in the levels of expression or subcellular localisation of the protein according to any of claims 1 to 7 in said patient.

**29.** A method according to claim 27 or 28 wherein said premalignant lesions occur in non-haematological malignancies.

**30.** A method according to any of claims 27 to 29 wherein said tumour type is any of cervical, breast, renal, head and neck, pancreatic, prostate, stomach, colon or lung.

**31.** An in vitro method of assessing the prognosis of a condition or cancer in a patient which method comprises detecting varying expression patterns or function of a protein according to any of claims 1 to 7.

**32.** An in vitro method of assessing the prognosis of a condition or cancer in a patient which method comprises detecting for abnormal mRNA transcripts or abnormal levels of mRNA expression, nucleotide sequences or gene copy numbers encoding a protein according to any of claims 1 to 7.

**33.** An in vitro method of screening for predisposition to cancer in an individual which comprises screening for an inherited genetic mutation in a nucleic acid sequence from said individual encoding a protein according to any of claims 1 to 7.

**34.** An in vitro method of identifying a cancer patient with increased sensitivity to anticancer agents, which method comprises determining levels of a protein according to any of claims 1 to 7 in the tumours of said patients, wherein varying expression patterns of said protein in the tumour of said patient is indicative of increased susceptibility to anticancer agents.

**35.** Use of a protein according to any of claims 1 to 7 in the manufacture of a medicament for controlling T-cell mediated immune responses in an individual, wherein high levels of said protein induce or mediate a reduction in antigen-specific T-cell mediated immune responses in said individual.

**36.** Use of a protein according to any of claims 1 to 7 in the manufacture of a medicament for alleviating graft-versus-host diseases in an individual.

**37.** The use according to claim 36 wherein said graft tissue is a transplanted organ.

**38.** An in vitro method of detecting or diagnosing cancer in an individual which is associated with reduced levels of expression of a protein according to any of claims 1 to 7 which method comprises testing in a cell of said individual for increased levels of methylation of a regulatory region of a nucleic acid sequence from said individual encoding a protein according to any of claims 1 to 7.

**39.** Use of a methylation inhibitor in the manufacture of a medicament for treating cancer associated with reduced levels of expression of a FOXP1 protein according to any of claims 1 to 7.

**40.** An in vitro method of detecting or diagnosing cancer in an individual which is associated with increased levels of expression of a protein according to any of claims 1 to 7, which method comprises testing in a cell of said individual for decreased levels of methylation of a regulatory region of a nucleic acid molecule encoding a protein according to any of claims 1 to 7.

**41.** Use of an antibody according to claim 16 or a blocking peptide, or a nucleic acid molecule capable of hybridising to a nucleic acid molecule according to any of claims 8 to 12 under conditions of high stringency in the manufacture of a medicament for treating a disease or condition in a patient associated with overexpression of a FOXP1 protein according to any of claims 1 to 7.

**42.** An in vitro method of identifying minimal residual disease in a cancer patient which method comprises detecting for the presence of neoplastic cells in an individual by identifying a change of expression pattern or function of a protein according to any of claims 1 to 7.

**43.** An in vitro method of identifying minimal residual disease in a cancer patient which method comprises detecting for the presence of neoplastic cells in an individual by identifying abnormal levels of mRNA expression or nucleotide sequences or gene copy number encoding a protein according to any of claims 1 to 7.

**Patentansprüche**

1. Isoliertes Transkriptionsfaktorprotein, umfassend (i) ein flügelförmiges Helixmotiv, welches die potenzielle Fähigkeit aufweist, Nukleinsäure zu binden, und (ii) ein $Cys_2$-$His_2$-Zinkfingermotiv, welches ebenso eine potenzielle Nukleinsäurebindefähigkeit aufweist.

2. Protein nach Anspruch 1, weiterhin umfassend mindestens eine transkriptionelle Aktivierungsdomäne.

3. Protein nach Anspruch 2, wobei das Protein die Aminosäuresequenz gemäß Figur 2 oder eine Aminosäuresequenz umfasst, welche eine Identität von mindestens 70 % mit der Aminosäuresequenz gemäß Figur 2 aufweist.

4. Protein nach Anspruch 1, wobei das Protein die Aminosäuresequenz gemäß Figur 3C oder eine Aminosäuresequenz umfasst, welche eine Identität von mindestens 80 % mit der Aminosäuresequenz gemäß Figur 3C aufweist.

5. Protein nach Anspruch 1, wobei das Protein die Aminosäuresequenz gemäß Figur 4B oder eine Aminosäuresequenz umfasst, welche eine Identität von mindestens 90 % mit der Aminosäuresequenz gemäß Figur 4B aufweist.

6. Protein nach Anspruch 1, wobei das Protein die Aminosäuresequenz gemäß Figur 4D oder eine Aminosäuresequenz umfasst, welche eine Identität von mindestens 95 % mit der Aminosäuresequenz gemäß Figur 4D aufweist.

7. Protein nach Anspruch 3, welches kein äußeres N-terminales Methionin aufweist.

8. Isoliertes Nukleinsäuremolekül, welches eine Nukleotidsequenz umfasst, die für das Protein nach einem der Ansprüche 1 bis 7 codiert.

9. Isoliertes Nukleinsäuremolekül, welches die Nukleotidsequenz umfasst, die von Position 264 oder Position 267 bis Position 2294 der Nukleinsäuresequenz gemäß Figur 2 gezeigt ist oder welches die Nukleotidsequenz umfasst, die von Position 16 bis 2352 der Nukleinsäuresequenz gemäß Figur 2 gezeigt ist.

10. Isoliertes Nukleinsäuremolekül, welches die Nukleotidsequenz gemäß Figur 3A oder 3B umfasst.

11. Isoliertes Nukleinsäuremolekül, welches die Nukleotidsequenz gemäß Figur 4A umfasst.

12. Isoliertes Nukleinsäuremolekül, welches die Nukleotidsequenz gemäß Figur 4C umfasst.

13. Expressionsvektor, welcher die Nukleinsäure nach einem der Ansprüche 8 bis 11 umfasst.

14. Wirtszelle, die mit dem Expressionsvektor von Anspruch 13 transformiert oder transfiziert ist.

15. Antikörper, welcher in der Lage ist, das Protein nach einem der Ansprüche 1 bis 7 oder ein Epitop davon zu binden, welches ein monoklonaler Antikörper JC12 ist, der von dem Hybridom, der bei der Europäischen Zellkultursammlung unter der Accession Nr. 99041425 hinterlegt ist, erhältlich ist.

16. Antikörper nach Anspruch 15 zur Verwendung zur Behandlung des menschlichen oder tierischen Körpers.

17. Verwendung eines Antikörpers nach Anspruch 15 zur Herstellung eines Arzneimittels zur Behandlung einer Erkrankung oder eines Zustandes, der durch die Expression oder Funktion eines Proteins nach einem der Ansprüche 1 bis 7 vermittelt wird oder damit in Zusammenhang steht.

18. Hybridom, das bei der Europäischen Zellkultursammlung unter der Accession Nr. 99041425 hinterlegt ist.

19. *In vitro*-Verfahren zum Nachweis der Expression eines Proteins, welches ein flügelförmiges Helixmotiv und ein $Cys_2$-$His_2$-Zinkfingermotiv umfasst, in einem Säugersubjekt, wobei das Verfahren das Inkontaktbringen einer Probe von Gewebe, Zellen oder Zelllysaten, die aus dem Säugersubjekt entnommen werden, mit dem Antikörper gemäß Anspruch 18 umfasst, und Nachweisen von spezifischer Bindung des Antikörpers an dessen Zielprotein in dem Gewebe.

20. Verfahren nach Anspruch 19, wobei der Antikörper ein monoklonaler Antikörper ist, der von dem Hybridom erhältlich ist, das bei der Europäischen Zellkultursammlung unter der Accession Nr. 99041425 hinterlegt ist.

**21.** Nukleinsäuremolekül nach einem der Ansprüche 8 bis 12 oder ein Nukleinsäuremolekül, das in der Lage ist, unter Bedingungen von hoher Stringenz daran zu hybridisieren, oder ein Fragment davon zur Verwendung zur Behandlung eines menschlichen oder tierischen Körpers.

**22.** Verwendung eines Nukleinsäuremoleküls nach einem der Ansprüche 8 bis 12 oder eines Nukleinsäuremoleküls, das in der Lage ist, daran unter Bedingungen von hoher Stringenz zu hybridisieren, oder eines Fragmentes davon zur Herstellung eines Arzneimittels zur Behandlung von Krebs.

**23.** Protein nach einem der Ansprüche 1 bis 7 oder eines Derivates oder Fragmentes davon zur Verwendung zur Behandlung eines menschlichen oder tierischen Körpers.

**24.** Verwendung eines Proteins nach einem der Ansprüche 1 bis 7 oder ein Derivat oder Fragment davon zur Herstellung eines Arzneimittels zur Behandlung von Krebs.

**25.** Pharmazeutische Zusammensetzung, umfassend ein isoliertes Protein nach einem der Ansprüche 1 bis 7, ein Nukleinsäuremolekül nach einem der Ansprüche 8 bis 12 oder eine Nukleinsäure, die in der Lage ist, an die Nukleinsäuremoleküle unter Bedingungen von hoher Stringenz zu hybridisieren oder einen Antikörper nach Anspruch 16, zusammen mit einem pharmazeutisch annehmbaren Träger, Verdünnungsmittel oder Hilfsstoff dafür.

**26.** Verwendung eines Antikörpers nach Anspruch 16 zur Herstellung eines Arzneimittels zur Behandlung von Krebs.

**27.** *In vitro*-Verfahren zur Beurteilung der Prognose von prämalignen Läsionen bei einem Patienten, wobei das Verfahren das Nachweisen einer Veränderung des Expressionsmusters oder der Funktion eines Proteins nach einem der Ansprüche 1 bis 7 in dem Patienten umfasst, wobei eine Veränderung des Expressionsmusters oder der Funktion des Proteins auf die Wahrscheinlichkeit hindeutet, dass sich die prämalignen Läsionen des Patienten zu einem malignen Tumor entwickeln.

**28.** Verfahren nach Anspruch 27, wobei die Veränderung des Expressionsmusters oder der Funktion des Proteins aufgrund von Veränderungen der Expressionsmengen oder der subzellulären Lokalisation des Proteins nach einem der Ansprüche 1 bis 7 in dem Patienten auftritt.

**29.** Verfahren nach Anspruch 27 oder 28, wobei die prämalignen Läsionen bei nicht-hämatologischen Malignomen auftreten.

**30.** Verfahren nach einem der Ansprüche 27 bis 29, wobei die Tumorart eine beliebige Tumorart von Gebärmutterhals-, Brust-, Nieren-, Kopf- und Nacken-, Bauchspeicheldrüsen-, Prostata-, Magen-, Dickdarm- oder Lungentumor ist.

**31.** *In vitro*-Verfahren zur Beurteilung der Prognose eines Zustandes oder von Krebs bei einem Patienten, wobei das Verfahren das Nachweisen von variierenden Expressionsmustem oder variierender Funktion eines Proteins nach einem der Ansprüche 1 bis 7 umfasst.

**32.** *In vitro*-Verfahren zur Beurteilung der Prognose eines Zustandes oder von Krebs bei einem Patienten, wobei das Verfahren das Nachweisen von abnormen mRNA-Transkripten oder abnormen Mengen an mRNA-Expression, Nukleotidsequenzen oder einer abnormen Anzahl von Genkopien, die für ein Protein nach einem der Ansprüche 1 bis 7 codieren, umfasst.

**33.** *In vitro*-Verfahren zum Durchmustern nach einer Veranlagung für Krebs in einem Subjekt, wobei das Verfahren das Durchmustern nach einer vererbten genetischen Mutation in einer Nukleinsäuresequenz umfasst, die für ein Protein nach einem der Ansprüche 1 bis 7 codiert.

**34.** *In vitro*-Verfahren zur Identifizierung eines Krebspatienten mit erhöhter Empfindlichkeit gegenüber Antikrebsmitteln, wobei das Verfahren die Bestimmung von Proteinmengen nach einem der Ansprüche 1 bis 7 in den Tumoren der Patienten umfasst, wobei das Variieren von Expressionsmustern des Proteins in dem Tumor des Patienten auf eine erhöhte Ansprechbarkeit für Antikrebsmittel hindeutet.

**35.** Verwendung eines Proteins nach einem der Ansprüche 1 bis 7 zur Herstellung eines Arzneimittels zur Steuerung von T-Zell-vermittelten Immunantworten in einem Subjekt, wobei hohe Mengen des Proteins eine Abnahme von Antigen-spezifischen T-Zell-vermittelten Immunantworten bei dem Subjekt auslösen oder vermitteln.

**36.** Verwendung eines Proteins nach einem der Ansprüche 1 bis 7 zur Herstellung eines Arzneimittels zur Linderung von Transplantat-Wirt-Reaktionen bei einem Subjekt.

**37.** Verwendung nach Anspruch 36, wobei das transplantierte Gewebe ein transplantiertes Organ ist.

**38.** *In vitro*-Verfahren zum Nachweisen oder Diagnostizieren von Krebs bei einem Subjekt, welches mit verminderten Proteinexpressionsmengen nach einem der Ansprüche 1 bis 7 in Zusammenhang steht, wobei das Verfahren das Untersuchen von erhöhten Methylierungsmengen einer regulatorischen Region einer Nukleinsäuresequenz des Subjektes, welche für ein Protein nach einem der Ansprüche 1 bis 7 codiert, in einer Zelle des Subjektes umfasst.

**39.** Verwendung eines Methylierungs-Inhibitors zur Herstellung eines Arzneimittels zur Behandlung von Krebs, der mit verminderten Expressionsmengen eines FOXP1-Proteins nach einem der Ansprüche 1 bis 7 in Zusammenhang steht.

**40.** *In vitro*-Verfahren zum Nachweisen oder Diagnostizieren von Krebs in einem Subjekt, welcher mit erhöhten Expressionsmengen eines Proteins nach einem der Ansprüche 1 bis 7 im Zusammenhang steht, wobei das Verfahren das Untersuchen von verminderten Methylierungsmengen einer regulatorischen Region eines Nukleinsäuremoleküls, das für ein Protein nach einem der Ansprüche 1 bis 7 codiert, in einer Zelle des Subjektes umfasst.

**41.** Verwendung eines Antikörpers nach Anspruch 16 oder eines Blockierungspeptids oder eines Nukleinsäuremoleküls, das in der Lage ist, an ein Nukleinsäuremolekül nach einem der Ansprüche 8 bis 12 unter Bedingungen von hoher Stringenz zu hybridisieren, zur Herstellung eines Arzneimittels zur Behandlung einer Erkrankung oder eines Zustandes bei einem Patienten, der mit Überexpression eines FOXP1-Proteins nach einem der Ansprüche 1 bis 7 in Zusammenhang steht.

**42.** *In. vitro*-Verfahren zur Identifizierung von minimalen Resten einer Erkrankung bei einem Krebspatienten, wobei das Verfahren das Nachweisen des Vorliegens von neoplastischen Zellen bei einem Subjekt durch Identifizieren einer Veränderung des Expressionsmusters oder der Funktion eines Proteins nach einem der Ansprüche 1 bis 7 umfasst.

**43.** *In vitro*-Verfahren zur Identifizierung von minimalen Resten einer Erkrankung bei einem Krebspatienten, wobei das Verfahren das Nachweisen des Vorliegens von neoplastischen Zellen bei einem Subjekt durch Identifizieren von abnormen Mengen an mRNA-Expression oder Nukleotidsequenzen oder Anzahl von Genkopien, die für ein Protein nach einem der Ansprüche 1 bis 7 codieren, umfasst.

## Revendications

**1.** Protéine isolée de facteur de transcription, comprenant (1) un motif « winged helix », qui a la capacité potentielle de lier l'acide nucléique et (ii) un motif en doigt de zinc $Cys_2$-$His_2$, qui a également la capacité potentielle de lier l'acide nucléique.

**2.** Protéine selon la revendication 1, qui comprend en outre, au moins un domaine d'activation transcriptionnelle.

**3.** Protéine selon la revendication 2, qui comprend la séquence des acides aminés représentée à la figure 2 ou une séquence d'acides aminés, qui a une identité d'au moins 70% avec la séquence d'acides aminés représentée à la figure 2.

**4.** Protéine selon la revendication 1, qui comprend la séquence d'acides aminés représentée à la figure 3C ou une séquence d'acides aminés, qui a une identité d'au moins 80% avec la séquence d'acides aminés représentée à la figure 3C.

**5.** Protéine selon la revendication 1, qui comprend la séquence d'acides aminés représentée à la figure 4B ou une séquence d'acides aminés, qui a une identité d'au moins 90% avec la séquence d'acides aminés représentée à la figure 4B.

6. Protéine selon la revendication 1, qui comprend la séquence d'acides aminés représentée à la figure 4D ou une séquence d'acides aminés, qui a une identité d'au moins 95% avec la séquence d'acides aminés représentée à la figure 4D.

7. Protéine selon la revendication 3, qui ne présente pas de méthionine à l'extrémité N-terminale.

8. Molécule d'acide nucléique isolée, comprenant une séquence des nucléotides, qui code la protéine selon l'une quelconque des revendications 1 à 7.

9. Molécule d'acide nucléique isolée, comprenant la séquence des nucléotides représentée depuis la position 264 ou la position 267 à la position 2294 de la séquence d'acides nucléiques représentée à la figure 2, ou la séquence de nucléotides depuis la position 16 à 2352 de la séquence d'acides nucléiques représentée à la figure 2.

10. Molécule d'acide nucléique isolée, comprenant la séquence de nucléotides représentée à la figure 3A ou 3B.

11. Molécule d'acide nucléique isolée, comprenant la séquence de nucléotides représentée à la figure 4A.

12. Molécule d'acide nucléique isolée, comprenant la séquence de nucléotides représentée à la figure 4C.

13. Vecteur d'expression comprenant la séquence d'acide nucléique selon l'une quelconque des revendications 8 à 11.

14. Cellule hôte transformée ou transfectée avec le vecteur d'expression de la revendication 13.

15. Anticorps qui est capable de lier la protéine selon l'une quelconque des revendications 1 à 7 ou un épitope de celle-ci, qui est un anticorps monoclonal JC12, pouvant être obtenu à partir de l'hybridome déposé au « European Collection of Cell Cultures » sous le n° d'accès 99041425.

16. Anticorps selon la revendication 15, pour une utilisation dans le traitement du corps humain ou animal.

17. Utilisation d'un anticorps selon la revendication 15, pour la préparation d'un médicament pour traiter une maladie ou un état à médiation par ou associé à l'expression ou la fonction d'une protéine selon l'une quelconque des revendications 1 à 7.

18. Hybridome déposé au « European Collection of Cell Cultures » sous le n° d'accès 99041425.

19. Procédé in vitro de détection de l'expression d'une protéine, comprenant un motif « winged hélix » et un motif en doigt de zinc $Cys_2$-$His_2$, chez un sujet mammifère, lequel procédé comprend la mise en contact d'un échantillon de tissu, de cellules ou de lysats cellulaires, prélevés du sujet mammifère, avec l'anticorps selon la revendication 18 et la détection de la liaison spécifique de l'anticorps à sa protéine cible dans ledit tissu.

20. Procédé selon la revendication 19, dans lequel l'anticorps est un anticorps monoclonal susceptible d'être obtenu à partir de l'hybridome déposé au « European Collection of Cell Cultures » sous le n° d'accès 99041425.

21. Molécule d'acide nucléique selon l'une quelconque des revendications 8 à 12, ou molécule d'acide nucléique capable de s'hybrider à celle-ci dans des conditions de haute stringence ou un fragment de celle-ci, pour l'utilisation dans le traitement du corps humain ou animal.

22. Utilisation d'une molécule d'acide nucléique selon l'une quelconque des revendications 8 à 12, ou d'une molécule d'acide nucléique capable de s'hybrider à celle-ci dans des conditions de haute stringence ou un fragment de celle-ci, pour la préparation d'un médicament pour traiter le cancer.

23. Protéine selon l'une quelconque des revendications 1 à 7 ou un dérivé ou fragment de celle-ci, pour l'utilisation dans le traitement du corps humain ou animal.

24. Utilisation d'une protéine selon l'une quelconque des revendications 1 à 7 ou un dérivé ou fragment de celle-ci, dans la préparation d'un médicament pour traiter le cancer.

25. Composition pharmaceutique comprenant l'une quelconque d'une protéine isolée selon l'une quelconque des revendications 1 à 7, d'une molécule d'acide nucléique selon l'une quelconque des revendications 8 à 12, ou d'une molécule d'acide nucléique capable de s'hybrider à celle-ci dans des conditions de haute stringence, d'un anticorps selon la revendication 16, avec un support, diluant ou excipient pharmaceutiquement acceptable.

**26.** Utilisation d'un anticorps selon la revendication 16, pour la préparation d'un médicament pour traiter le cancer.

**27.** Procédé in vitro pour établir le pronostic de lésions prémalignes chez un patient, lequel procédé comprend la détection d'un changement du profil d'expression ou de la fonction d'une protéine selon l'une quelconque des revendications 1 à 7 chez ledit patient, où un changement du profil d'expression ou de la fonction de ladite protéine est une indication de la probabilité des lésions prémalignes du patient à se développer en tumeur maligne.

**28.** Procédé selon la revendication 27, dans lequel ledit changement du profil d'expression ou de la fonction de ladite protéine se produit à cause de changements des taux d'expression ou de la localisation subcellulaire de la protéine selon l'une quelconque des revendications 1 à 7 chez ledit patient.

**29.** Procédé selon la revendication 27 ou 28, dans lequel lesdites lésions prémalignes se produisent dans les malignités non hématologiques.

**30.** Procédé selon l'une quelconque des revendications 27 à 29, dans lequel le type de tumeur est une tumeur du col, du sein, du rein, de la tête et du cou, du pancréas, de la prostate, de l'estomac, du colon ou des poumons.

**31.** Procédé in vitro pour établir le pronostic d'un état ou d'un cancer chez un patient, lequel procédé comprend la détection d'un changement des profils d'expression ou de la fonction d'une protéine selon l'une quelconque des revendications 1 à 7.

**32.** Procédé in vitro pour établir le pronostic d'un état ou d'un cancer chez un patient, lequel procédé comprend la détection des produits de transcription ARNm anormaux, ou de taux anormaux de l'expression d'ARNm, de séquences de nucléotides ou de nombres de copies de gène, codant une protéine selon l'une quelconque des revendications 1 à 7.

**33.** Procédé in vitro pour cribler la prédisposition à un cancer chez un individu, qui comprend le criblage d'une mutation génétique héritée dans une séquence d'acide nucléique dudit individu, codant une protéine selon l'une quelconque des revendications 1 à 7.

**34.** Procédé in vitro pour identifier un cancer chez un patient avec une sensibilité accrue aux agents anticancéreux, lequel procédé comprend la détermination des taux d'une protéine selon l'une quelconque des revendications 1 à 7 dans les tumeurs dudit patient, où des profils d'expression variables de la dite protéine dans la tumeur dudit patient est une indication d'une susceptibilité accrue aux agents anticancéreux.

**35.** Utilisation d'une protéine selon l'une quelconque des revendications 1 à 7, pour la préparation d'un médicament pour contrôler les réponses immunes à médiation par les cellules T chez un individu, où des taux élevés de ladite protéine induisent ou interviennent dans la réduction des réponses immunes à médiation par les cellules T spécifiques d'antigène chez ledit individu.

**36.** Utilisation d'une protéine selon l'une quelconque des revendications 1 à 7, pour la préparation d'un médicament pour soulager les maladies du greffon contre l'hôte chez un individu.

**37.** Utilisation selon la revendication 36, dans laquelle ledit tissu greffé est un organe transplanté.

**38.** Procédé in vitro pour détecter ou diagnostiquer un cancer chez un individu, qui est associé à des taux réduits d'expression d'une protéine selon l'une quelconque des revendications 1 à 7, lequel procédé comprend le test dans une cellule dudit individu, de taux accrus de méthylation d'une région de régulation d'une séquence d'acide nucléique dudit individu, codant une protéine selon l'une quelconque des revendications 1 à 7.

**39.** Utilisation d'un inhibiteur de méthylation dans la préparation d'un médicament pour traiter un cancer associé à des taux réduits d'expression d'une protéine FOXP1 selon l'une quelconque des revendications 1 à 7.

**40.** Procédé in vitro pour détecter ou diagnostiquer un cancer chez un individu, qui est associé à des taux accrus d'expression d'une protéine selon l'une quelconque des revendications 1 à 7, lequel procédé comprend le test dans une cellule dudit individu, de taux diminués de méthylation d'une région de régulation d'une séquence d'acide nucléique, codant une protéine selon l'une quelconque des revendications 1 à 7.

**41.** Utilisation d'un anticorps selon la revendication 16 ou d'un peptide de blocage ou d'une molécule d'acide nucléique capable d'hybrider une molécule d'acide nucléique selon l'une quelconque des revendications 8 à 12, dans des conditions de haute stringence, pour la préparation d'un médicament pour traiter une maladie ou un état chez un patient, associé à la surexpression d'une protéine FOXP1 selon l'une quelconque des revendications 1 à 7.

**42.** Procédé in vitro d'identification d'une maladie résiduelle minimale chez un patient cancéreux, lequel procédé comprend la détection de la présence de cellules néoplasiques chez un individu par identification du changement du profil d'expression ou de la fonction d'une protéine selon l'une quelconque des revendications 1 à 7.

**43.** Procédé in vitro d'identification d'une maladie résiduelle minimale chez un patient cancéreux, lequel procédé comprend la détection de la présence de cellules néoplasiques chez un individu par identification de taux anormaux de l'expression d'ARNm ou de séquences de nucléotides ou de nombre de copies d'un gène codant une protéine selon l'une quelconque des revendications 1 à 7.

FIG. 1.

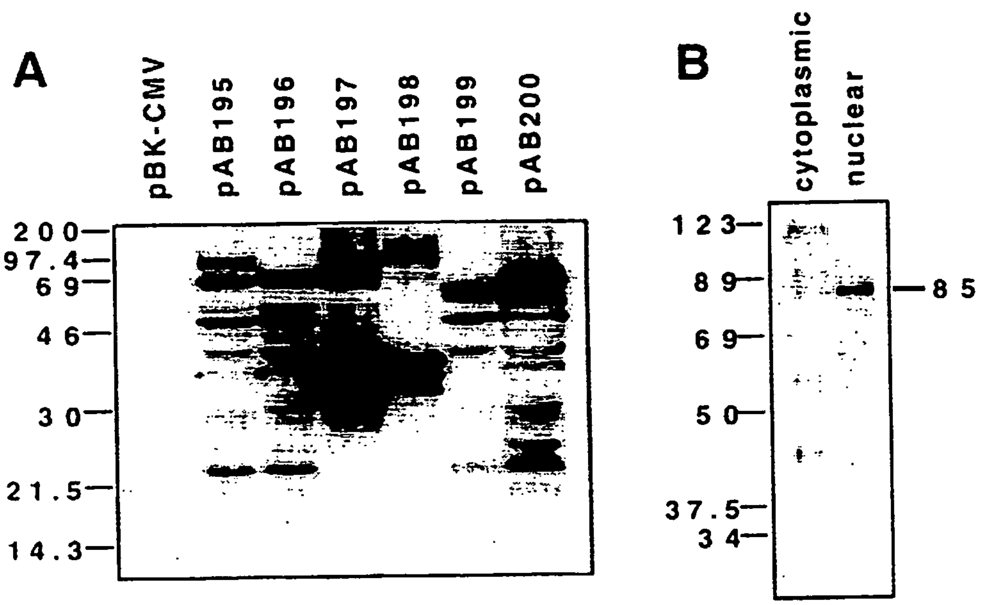
A
pBK-CMV
pAB195
pAB196
pAB197
pAB198
pAB199
pAB200
200
97.4
69
46
30
21.5
14.3
B
cytoplasmic
nuclear
123
89
69
50
37.5
34
85

FIG. 2.

```
1    GAATTCGGCACGAGCGGCAATGGTGAGGGCTTCGATCCCTTCTCTGATTTGCTGTCAGCCATGAACGGATGGATGTGATGCCTGCTAGCCAAAAGGCTTC
linker from library
101  CCTCTGTGTGTTGCAGTCCTGTGGCATTATGCATGCCCCCTCCCAGTGACCCCAGGCTTTTTATGGCTGTGAGACACGTTAAAATTTCAGGGGTAAGACG

201  TGACCTTTTGAGGTGACTATAACTGAAGATTGCTTTACAGAAGCCAAAAAAGGTTTTTGAGTCATGATGCAAGAATCTGGGACTGAGACAAAAAGTAACG
                                                                M  M  Q  E  S  G  T  E  T  K  S  N  G
                                                                                 CK2
301  GTTCAGCCATCCAGAATGGGTCGGGCGGCAGCAACCACTTACTAGAGTGCGGCGGTCTTCGGGAGGGGCGGTCCAACGGAGAGACGCCGGCCGTGGACAT
       S  A  I  Q  N  G  S  G  G  S  N  H  L  L  E  C  G  G  L  R  E  G  R  S  N  G  E  T  P  A  V  D  I
                                                                                  CK2
401  CGGGGCAGCTGACCTCGCCCACGCCCAGCAGCAGCAGCAACAGGCACTTCAGGTGGCAAGACAGCTCCTTCTTCAGCAGCAACAGCAGCAGCAAGTTAGT
      G  A  A  D  L  A  H  A  Q  Q  Q  Q  Q  Q  A  L  Q  V  A  R  Q  L  L  L  Q  Q  Q  Q  Q  Q  Q  V  S

501  GGATTAAAATCTCCCAAGAGGAATGACAAACAACCAGCTCTTCAGGTTCCCGTGTCAGTGGCTATGATGACACCTCAAGTTATCACTCCCCAGCAAATGC
     G  L  K  S  P  K  R  N  D  K  Q  P  A  L  Q  V  P  V  S  V  A  M  M  T  P  Q  V  I  T  P  Q  Q  M  Q
                 PKC
601  AGCAGATCCTCCAGCAACAAGTGCTGAGCCCTCAGCAGCTCCAGGTTCTCCTCCAGCAGCAGCAGGCCCTCATGCTTCAACAGCAGCAGCTTCAAGAGTT
       Q  I  L  Q  Q  Q  V  L  S  P  Q  Q  L  Q  V  L  L  Q  Q  Q  Q  A  L  M  L  Q  Q  Q  Q  L  Q  E  F
                                                                   Coiled Coil
701  TTATAAAAAACAACAGGAACAGTTGCAGCTTCAACTTTTACAACAACAACATGCTGGAAAACAGCCTAAAGAGCAACAGCAGGTGGCTACCCAGCAGTTG
      Y  K  K  Q  Q  E  Q  L  Q  L  Q  L  L  Q  Q  Q  H  A  G  K  Q  P  K  E  Q  Q  Q  V  A  T  Q  Q  L

801  GCTTTTCAGCAGCAGCTTTTACAGATGCAGCAGTTACAGCAGCAGCACCTCCTGTCTTTGCAGCGCCAAGGCCTTCTGACAATTCAGCCCGGGCAGCCTG
      A  F  Q  Q  Q  L  L  Q  M  Q  Q  L  Q  Q  Q  H  L  L  S  L  Q  R  Q  G  L  L  T  I  Q  P  G  Q  P  A

901  CCCTTCCCCTTCAACCTCTTGCTCAAGGCATGATTCCAACAGAACTGCAGCAGCTCTGGAAAGAAGTGACAAGTGCTCATACTGCAGAAGAAACCACAGG
       L  P  L  Q  P  L  A  Q  G  M  I  P  T  E  L  Q  Q  L  W  K  E  V  T  S  A  H  T  A  E  E  T  T  G
                                                                                           CK2
1001 CAACAATCACAGCAGTTTGGATCTGACCACGACATGTGTCTCCTCCTCTGCACCTTCCAAGACCTCCTTAATAATGAACCCACATGCCTCTACCAATGGA
      N  N  H  S  S  L  D  L  T  T  T  C  V  S  S  S  A  P  S  K  T  S  L  I  M  N  P  H  A  S  T  N  G
                 CK2 ST rich
1101 CAGCTCTCAGTCCACACTCCCAAAAGGGAAAGTTTGTCCCATGAGGAGCACCCCCATAGCCATCCTCTCTATGGACATGGTGTATGCAAGTGGCCAGGCT
      Q  L  S  V  H  T  P  K  R  E  S  L  S  H  E  E  H  P  H  S  H  P  L  Y  G  H  G  V  C  K  W  P  G  C
```

FIG. 2 (CONTINUED).

```
                     PKC      cAMP          CK2
1201 GTGAAGCAGTGTGCGAAGATTTCCAATCATTTCTAAAACATCTCAACAGTGAGCATGCGCTGGACGATAGAAGTACAGCCCAATGTAGAGTACAAATGCA
      E  A  V  C  E  D  F  Q  S  F  L  K  H  L  N  S  E  H  A  L  D  D  R  S  T  A  Q  C  R  V  Q  M  Q
                    Zn Finger
1301 GGTTGTACAGCAGTTAGAGCTACAGCTTGCAAAAGACAAAGAACGCCTGCAAGCCATGATGACCCACCTGCATGTGAAGTCTACAGAACCCAAAGCCGCC
      V  V  Q  Q  L  E  L  Q  L  A  K  D  K  E  R  L  Q  A  M  M  T  H  L  H  V  K  S  T  E  P  K  A  A
                    Coiled Coil
1401 CCTCAGCCCTTGAATCTGGTATCAAGTGTCACTCTCTCCAAGTCCGCATCGGAGGCTTCTCCACAGAGCTTACCTCATACTCCAACGACCCCAACCGCCC
     P  Q  P  L  N  L  V  S  S  V  T  L  S  K  S  A  S  E  A  S  P  Q  S  L  P  H  T  P  T  T  P  T  A  P
                                              CK2                           STP rich
1501 CCCTGACTCCCGTCACCCAAGGCCCCTCTGTCATCACAACCACCAGCATGCACACGGTGGGACCCATCCGCAGGCGGTACTCAGACAAATACAACGTGCC
      L  T  P  V  T  Q  G  P  S  V  I  T  T  T  S  M  H  T  V  G  P  I  R  R  R  Y  S, D  K  Y  N  V  P
                                                               ---NLS------cAMP   PKC
1601 CATTTCGTCAGCAGATATTGCGCAGAACCAAGAATTTTATAAGAACGCAGAAGTTAGACCACCATTTACATATGCATCTTTAATTAGGCAGGCCATTCTC
      I  S  S  A  D  I  A  Q  N  Q  E  F  Y  K  N  A  E  V  R  P  P  F  T  Y  A  S  L  I  R  Q  A  I  L
        CK2
1701 GAATCTCCAGAAAAGCAGCTAACACTAAATGAGATCTATAACTGGTTCACACGAATGTTTGCTTACTTCCGACGCAACGCGGCCACGTGGAAGAATGCAG
     E  S  P  E  K  Q  L  T  L  N  E  I  Y  N  W  F  T  R  M  F  A  Y  F  R  R  N  A  A  T  W  K  N  A  V
                         CK2          Winged Helix Domain                  PKC
1801 TGCGTCATAATCTTAGTCTTCACAAGTGTTTTGTGCGAGTAGAAAACGTTAAAGGGGCAGTATGGACAGTGGATGAAGTAGAATTCCAAAAACGAAGGCC
      R  H  N  L  S  L  H  K  C  F  V  R  V  E  N  V  K  G  A  V  W  T  V  D  E  V  E  F  Q  K  R  R  P
                                                                  CK2              ---NLS-----
1901 ACAAAAGATCAGTGGTAACCCTTCCCTTATTAAAAACATGCAGAGCAGCCACGCCTACTGCACACCTCTCAATGCAGCTTTACAGGCTTCAATGGCTGAG
      Q  K  I  S  G  N  P  S  L  I  K  N  M  Q  S  S  H  A  Y  C  T  P  L  N  A  A  L  Q  A  S  M  A  E
                                                                                          CK2
2001 AATAGTATACCTCTATACACTACCGCTTCCATGGGAAATCCCACTCTGGGCAACTTAGCCAGCGCAATACGGGAAGAGCTGAACGGGGCAATGGAGCATA
     N  S  I  P  L  Y  T  T  A  S  M  G  N  P  T  L  G  N  L  A  S  A  I  R  E  E  L  N  G  A  M  E  H  T

2101 CCAACAGCAACGAGAGTGACAGCAGTCCAGGCAGATCTCCTATGCAAGCCGTGCATCCTGTACACGTCAAAGAAGAGCCCCTCGATCCAGAGGAAGCTGA
      N  S  N  E  S  D  S  S  P  G  R  S  P  M  Q  A  V  H  P  V  H  V  K  E  E  P  L  D  P  E  E  A  E
                    STP rich                                                      Acidic
2201 AGGGCCCCTGTCCTTAGTGACAACAGCCAACCACAGTCCAGATTTTGACCATGACAGAGATTACGAAGATGAACCAGTAAACGAGGACATGGAGTGACTA
      G  P  L  S  L  V  T  T  A  N  H  S  P  D  F  D  H  D  R  D  Y  E  D  E  P  V  N  E  D  M  E  *
                                                                   Acidic
2301 TCGGGGCGGGCCAACCCCGAGAATGAAGATTGGAAAAAAAAAAAAAAAAAAA 2352
```

FIG. 3A.

## cDNA Sequences FOXP1 clones

### FOXP1 cDNA (pAB195)

GGCAATGGTG AGGGCTTCGA TCCCTTCTCT GATTTGCTGT CAGCCATGAA
CGGATGGATG TGATGCCTGC TAGCCAAAAG GCTTCCCTCT GTGTGTTGCA
GTCCTGTGGC ATTATGCATG CCCCCTCCCA GTGACCCCAG GCTTTTTATG
GCTGTGAGAC ACGTTAAAAT TTCAGGGGTA AGACGTGACC TTTTGAGGTG
ACTATAACTG AAGATTGCTT TACAGAAGCC AAAAAAGGTT TTTGAGTCAT
GATGCAAGAA TCTGGGACTG AGACAAAAAG TAACGGTTCA GCCATCCAGA
ATGGGTCGGG CGGCAGCAAC CACTTACTAG AGTGCGGCGG TCTTCGGGAG
GGGCGGTCCA ACGGAGAGAC GCCGGCCGTG GACATCGGGG CAGCTGACCT
CGCCCACGCC CAGCAGCAGC AGCAACAGGC ACTTCAGGTG GCAAGACAGC
TCCTTCTTCA GCAGCAACAG CAGCAGCAAG TTAGTGGATT AAAATCTCCC
AAGAGGAATG ACAAACAACC AGCTCTTCAG GTTCCCGTGT CAGTGGCTAT
GATGACACCT CAAGTTATCA CTCCCCAGCA AATGCAGCAG ATCCTCCAGC
AACAAGTGCT GAGCCCTCAG CAGCTCCAGG TTCTCCTCCA GCAGCAGCAG
GCCCTCATGC TTCAACAGCA GCAGCTTCAA GAGTTTTATA AAAAACAACA
GGAACAGTTG CAGCTTCAAC TTTTACAACA ACAACATGCT GGAAAACAGC
CTAAAGAGCA ACAGCAGGTG GCTACCCAGC AGTTGGCTTT TCAGCAGCAG
CTTTTACAGA TGCAGCAGTT ACAGCAGCAG CACCTCCTGT CTTTGCAGCG
CCAAGGCCTT CTGACAATTC AGCCCGGGCA GCCTGCCCTT CCCCTTCAAC
CTCTTGCTCA AGGCATGATT CCAACAGAAC TGCAGCAGCT CTGGAAAGAA
GTGACAAGTG CTCATACTGC AGAAGAAACC ACAGGCAACA ATCACAGCAG
TTTGGATCTG ACCACGACAT GTGTCTCCTC CTCTGCACCT TCCAAGACCT
CCTTAATAAT GAACCCACAT GCCTCTACCA ATGGACAGCT CTCAGTCCAC
ACTCCCAAAA GGGAAAGTTT GTCCCATGAG GAGCACCCCC ATAGCCATCC
TCTCTATGGA CATGGTGTAT GCAAGTGGCC AGGCTGTGAA GCAGTGTGCG
AAGATTTCCA ATCATTTCTA AAACATCTCA ACAGTGAGCA TGCGCTGGAC
GATAGAAGTA CAGCCCAATG TAGAGTACAA ATGCAGGTTG TACAGCAGTT
AGAGCTACAG CTTGCAAAAG ACAAAGAACG CCTGCAAGCC ATGATGACCC
ACCTGCATGT GAAGTCTACA GAACCCAAAG CCGCCCCTCA GCCCTTGAAT
CTGGTATCAA GTGTCACTCT CTCCAAGTCC GCATCGGAGG CTTCTCCACA
GAGCTTACCT CATACTCCAA CGACCCCAAC CGCCCCCCTG ACTCCCGTCA
CCCAAGGCCC CTCTGTCATC ACAACCACCA GCATGCACAC GGTGGGACCC
ATCCGCAGGC GGTACTCAGA CAAATACAAC GTGCCCATTT CGTCAGCAGA
TATTGCGCAG AACCAAGAAT TTTATAAGAA CGCAGAAGTT AGACCACCAT
TTACATATGC ATCTTTAATT AGGCAGGCCA TTCTCGAATC TCCAGAAAAG
CAGCTAACAC TAAATGAGAT CTATAACTGG TTCACACGAA TGTTTGCTTA
CTTCCGACGC AACGCGGCCA CGTGGAAGAA TGCAGTGCGT CATAATCTTA
GTCTTCACAA GTGTTTTGTG CGAGTAGAAA ACGTTAAAGG GGCAGTATGG
ACAGTGGATG AAGTAGAATT CCAAAAACGA AGGCCACAAA AGATCAGTGG
TAACCCTTCC CTTATTAAAA ACATGCAGAG CAGCCACGCC TACTGCACAC
CTCTCAATGC AGCTTTACAG GCTTCAATGG CTGAGAATAG TATACCTCTA
TACACTACCG CTTCCATGGG AAATCCCACT CTGGGCAACT TAGCCAGCGC
AATACGGGAA GAGCTGAACG GGGCAATGGA GCATACCAAC AGCAACGAGA
GTGACAGCAG TCCAGGCAGA TCTCCTATGC AAGCCGTGCA TCCTGTACAC
GTCAAAGAAG AGCCCCTCGA TCCAGAGGAA GCTGAAGGGC CCCTGTCCTT
AGTGACAACA GCCAACCACA GTCCAGATTT TGACCATGAC AGAGATTACG
AAGATGAACC AGTAAACGAG GACATGGAGT GACTATCGGG GCGGGCCAAC
CCCGAGAATG AAGATTGGAA AAAAAAAAAA AAAAAAA

FIG. 3B.

**pAB196 cDNA Sequence**

TCGCAGAAAC AGCCAGAACC CATCTACAGC AAGAAGACGG AAATCCAAAG
GCAGACAGTA CGGGCTCCCT TCGCCAAACT CTTCATTTTC TCTGCACTTC
AGGTGGCAAG ACAGCTCCTT CTTCAGCAGC AACAGCAGCA GCAAGTTAGT
GGATTAAAAT CTCCCAAGAG GAATGACAAA CAACCAGCTC TTCAGCAACA
GCAGGTGGCT ACCCAGCAGT TGGCTTTTCA GCAGCAGCTT TTACAGATGC
AGCAGTTACA GCAGCAGCAC CTCCTGTCTT TGCAGCGCCA AGGCCTTCTG
ACAATTCAGC CCGGGCAGCC TGCCCTTCCC CTTCAACCTC TTGCTCAAGG
CATGATTCCA ACAGAACTGC AGCAGCTCTG GAAAGAAGTG ACAAGTGCTC
ATACTGCAGA AGAAACCACA GGCAACAATC ACAGCAGTTT GGATCTGACC
ACGACATGTG TCTCCTCCTC TGCACCTTCC AAGACCTCCT TAATAATGAA
CCCACATGCC TCTACCAATG GACAGCTCTC AGTCCACACT CCCAAAAGGG
AAAGTTTGTC CCATGAGGAG CACCCCCATA GCCATCCTCT CTATGGACAT
GGTGTATGCA AGTGGCCAGG CTGTGAAGCA GTGTGCGAAG ATTTCCAATC
ATTTCTAAAA CATCTCAACA GTGAGCATGC GCTGGACGAT AGAAGTACAG
CCCAATGTAG AGTACAAATG CAGGTTGTAC AGCAGTTAGA GCTACAGCTT
GCAAAAGACA AAGAACGCCT GCAAGCCATG ATGACCCACC TGCATGTGAA
GTCTACAGAA CCCAAAGCCG CCCCTCAGCC CTTGAATCTG GTATCAAGTG
TCACTCTCTC CAAGTCCGCA TCGGAGGCTT CTCCACAGAG CTTACCTCAT
ACTCCAACGA CCCCAACCGC CCCCCTGACT CCCGTCACCC AAGGCCCCTC
TGTCATCACA ACCACCAGCA TGCACACGGT GGGACCCATC CGCAGGCGGT
ACTCAGACAA ATACAACGTG CCCATTTCGT CAGCAGATAT TGCGCAGAAC
CAAGAATTTT ATAAGAACGC AGAAGTTAGA CCACCATTTA CATATGCATC
TTTAATTAGG CAGGCCATTC TCGAATCTCC AGAAAAGCAG CTAACACTAA
ATGAGATCTA TAACTGGTTC ACACGAATGT TTGCTTACTT CCGACGCAAC
GCGGCCACGT GGAAGAATGC AGTGCGTCAT AATCTTAGTC TTCACAAGTG
TTTTGTGCGA GTAGAAAACG TTAAAGGGGC AGTATGGACA GTGGATGAAG
TAGAATTCCA AAAACGAAGG CCACAAAAGA TCAGTGGTAA CCCTTCCCTT
ATTAAAAACA TGCAGAGCAG CCACGCCTAC TGCACACCTC TCAATGCAGC
TTTACAGGCT TCAATGGCTG AGAATAGTAT ACCTCTATAC ACTACCGCTT
CCATGGGAAA TCCCACTCTG GGCAACTTAG CCAGCGCAAT ACGGGAAGAG
CTGAACGGGG CAATGGAGCA TACCAACAGC AACGAGAGTG ACAGCAGTCC
AGGCAGATCT CCTATGCAAG CCGTGCATCC TGTACACGTC AAAGAAGAGC
CCCTCGATCC AGAGGAAGCT GAAGGGCCCC TGTCCTTAGT GACAACAGCC
AACCACAGTC CAGATTTTGA CCATGACAGA GATTACGAAG ATGAACCAGT
AAACGAGGAC ATGGAGTGAC TATCGGGGCG GGCCAACCCC GAGAATGAAG
ATTGGAAAAA GGAAAAAAAA AAAAAAAAAC AAAAA

FIG. 3C.

**pAB196 protein sequence**

SQKQPEPIYS KKTEIQRQTV RAPFAKLFIF SALQVARQLL LQQQQQQVS
GLKSPKRNDK QPALQQQVA TQQLAFQQQL LQMQQLQQQH LLSLQRQGLL
TIQPGQPALP LQPLAQGMIP TELQQLWKEV TSAHTAEETT GNNHSSLDLT
TTCVSSSAPS KTSLIMNPHA STNGQLSVHT PKRESLSHEE HPHSHPLYGH
GVCKWPGCEA VCEDFQSFLK HLNSEHALDD RSTAQCRVQM QVVQQLELQL
AKDKERLQAM MTHLHVKSTE PKAAPQPLNL VSSVTLSKSA SEASPQSLPH
TPTTPTAPLT PVTQGPSVIT TTSMHTVGPI RRRYSDKYNV PISSADIAQN
QEFYKNAEVR PPFTYASLIR QAILESPEKQ LTLNEIYNWF TRMFAYFRRN
AATWKNAVRH NLSLHKCFVR VENVKGAVWT VDEVEFQKRR PQKISGNPSL
IKNMQSSHAY CTPLNAALQA SMAENSIPLY TTASMGNPTL GNLASAIREE
LNGAMEHTNS NESDSSPGRS PMQAVHPVHV KEEPLDPEEA EGPLSLVTTA
NHSPDFDHDR DYEDEPVNED ME

FIG. 4A.

pAB199 cDNA Sequence

ACGGGGTACT CCCAGCTGAA CCGGCTCTGA ATGTAGCTAA CTCAACTGTC
AGAACTGCAT GAAGGACGGT TCCCGTGTCA GTGGCTATGA TGACACCTCA
AGTTATCACT CCCCAGCAAA TGCAGCAGAT CCTCCAGCAA CAAGTGCTGA
GCCCTCAGCA GCTCCAGGTT CTCCTCCAGC AGCAGCAGGC CCTCATGCTT
CAACTGCAGC AGCTCTGGAA AGAAGTGACA AGTGCTCATA CTGCAGAAGA
AACCACAGGC AACAATCACA GCAGTTTGGA TCTGACCACG ACATGTGTCT
CCTCCTCTGC ACCTTCCAAG ACCTCCTTAA TAATGAACCC ACATGCCTCT
ACCAATGGAC AGCTCTCAGT CCACACTCCC AAAAGGGAAA GTTTGTCCCA
TGAGGAGCAC CCCCATAGCC ATCCTCTCTA TGGACATGGT GTATGCAAGT
GGCCAGGCTG TGAAGCAGTG TGCGAAGATT TCCAATCATT TCTAAAACAT
CTCAACAGTG AGCATGCGCT GGACGATAGA AGTACAGCCC AATGTAGAGT
ACAAATGCAG GTTGTACAGC AGTTAGAGCT ACAGCTTGCA AAAGACAAAG
AACGCCTGCA AGCCATGATG ACCCACCTGC ATGTGAAGTC TACAGAACCC
AAAGCCGCCC CTCAGCCCTT GAATCTGGTA TCAAGTGTCA CTCTCTCCAA
GTCCGCATCG GAGGCTTCTC CACAGAGCTT ACCTCATACT CCAACGACCC
CAACCGCCCC CCTGACTCCC GTCACCCAAG GCCCCTCTGT CATCACAACC
ACCAGCATGC ACACGGTGGG ACCCATCCGC AGGCGGTACT CAGACAAATA
CAACGTGCCC ATTTCGTCAG CAGATATTGC GCAGAACCAA GAATTTTATA
AGAACGCAGA AGTTAGACCA CCATTTACAT ATGCATCTTT AATTAGGCAG
GCCATTCTCG AATCTCCAGA AAAGCAGCTA ACACTAAATG AGATCTATAA
CTGGTTCACA CGAATGTTTG CTTACTTCCG ACGCAACGCG GCCACGTGGA
AGAATGCAGT GCGTCATAAT CTTAGTCTTC ACAAGTGTTT TGTGCGAGTA
GAAAACGTTA AAGGGGCAGT ATGGACAGTG GATGAAGTAG AATTCCAAAA
ACGAAGGCCA CAAAAGATCA GTGGTAACCC TTCCCTTATT AAAAACATGC
AGAGCAGCCA CGCCTACTGC ACACCTCTCA ATGCAGCTTT ACAGGCTTCA
ATGGCTGAGA ATAGTATACC TCTATACACT ACCGCTTCCA TGGGAAATCC
CACTCTGGGC AACTTAGCCA GCGCAATACG GGAAGAGCTG AACGGGGCAA
TGGAGCATAC CAACAGCAAC GAGAGTGACA GCAGTCCAGG CAGATCTCCT
ATGCAAGCCG TGCATCCTGT ACACGTCAAA GAAGAGCCCC TCGATCCAGA
GGAAGCTGAA GGGCCCCTGT CCTTAGTGAC AACAGCCAAC CACAGTCCAG
ATTTTGACCA TGACAGAGAT TACGAAGATG AACCAGTAAA CGAGGACATG
GAGTGACTAT CGGGGCGGGC CAACCCCGAG AATGAAGATT GGAAAAAGGA
AAAAAAAAAA AACACGTCAA AAGTTAAAAA AAAAAAAAAA AAA

FIG. 4B.

pAB199 protein sequence

MMTPQVITPQQ MQQILQQQVL SPQQLQVLLQ QQQALMLQLQ QLWKEVTSA
HTAEETTGNNH SSLDLTTTCV SSSAPSKTSL IMNPHASTNG QLSVHTPKR
ESLSHEEHPHS HPLYGHGVCK WPGCEAVCED FQSFLKHLNS EHALDDRST
AQCRVQMQVVQ QLELQLAKDK ERLQAMMTHL HVKSTEPKAA PQPLNLVSS
VTLSKSASEAS PQSLPHTPTT PTAPLTPVTQ GPSVITTTSM HTVGPIRRR
YSDKYNVPISS ADIAQNQEFY KNAEVRPPFT YASLIRQAIL ESPEKQLTL
NEIYNWFTRMF AYFRRNAATW KNAVRHNLSL HKCFVRVENV KGAVWTVDE
VEFQKRRPQKI SGNPSLIKNM QSSHAYCTPL NAALQASMAE NSIPLYTTA
SMGNPTLGNLA SAIREELNGA MEHTNSNESD SSPGRSPMQA VHPVHVKEE
PLDPEEAEGPL SLVTTANHSP DFDHDRDYED EPVNEDME

FIG. 4C.

**pAB200 5' cDNA sequence**

TGCCCTTCCC CTTCAACCTC TTGCTCAAGG CATGATTCCA ACAGAACTGC
AGCAGCTCTG GAAAGAAGTG ACAAGTGCTC ATACTGCAGA AGAAACCACA
GGCAACAATC ACAGCAGTTT GGATCTGACC ACGACATGTG TCTCCTCCTC
TGCACCTTCC AAGACCTCCT TAATAATGAA CCCACATGCC TCTACCAATG
GACAGCTCTC AGTCCACACT CCCAAAAGGG AAAGTTTGTC CCATGAGGAG
CACCCCCATA GCCATCCTCT CTATGGACAT GGTGTATGCA AGTGGCCAGG
CTGTGAAGCA GTGTGCGAAG ATTTCCAATC ATTTCTAAAA CATCTCAACA
GTGAGCATGC GCTGGACGAT AGAAGTACAG CCCAATGTAG AGTACAAATG
CAGGTTGTAC AGCAGTTAGA GCTACAGCTT GCAAAAGACA AAGAACGCCT
GCAAGCCATG ATGACCCACC TGCATGTGAA GTCTACAGAA CCCAAAGCCG
CCCCTCAGCC CTTGAATCTG GTATCAAGTG TCACTCTCTC CAAGTCCGCA
TCGGAGGCTT CTCCACAGAG CTTACCTCAT ACTCCAACGA CCCCAACCGC
CCCCCTGACT CCCGTCACCC AAGGCCCCTC TGTCATCACA ACCACCAGCA
TGCACACGGT GGGACCCATC CGCAGGCGGT ACTCAGACAA ATACAACGTG
CCCATTTCGT CAGATATTGC GCAGAACCAA GAATTTTATA AGAACGCAGA
AGTTAGACCA CCATTTACAT ATGCATCTTT AATTAGGCAG GCCATTCTCG
AATCTCCAGA AAAGCAGCTA ACACTAAATG AGATCTATAA CTGGTTCACA
CGAATGTTTG CTTACTTCCG ACGCAACGCG GCCACGTGGA AGAATGCAGT
GCGTCATAAT CTTAGTCTTC ACAAGTGTTT TGTGCGAGTA GAAAACGTTA
AAGGGGCAGT ATGGACAGTG GATGAAGTAG AATTCCAAAA ACGAAGGCCA
CAAAAGATCA GTGGTAACCC TTCCCTTATT AAAAACATGC AGAGCAGCCA
CGCCTACTGC ACACCTCTCA ATGCAGCTTT ACAGGCTTCA ATGGCTGAGA
ATAGTATACC TCTATACACT ACCGCTTCCA TGGGAAATCC CACTCTGGGC
AACTTAGCCA GCGCAATACG GGAAGAGCTG AACGGGGCAA TGGAGCATAC
CAACAGCAAC GAGAGTGACA GCAGTCCAGG CAGATCTCCT ATGCAAGCCG
TGCATCCTGT ACACGTCAAA GAAGAGCCCC TCGATCCAGA GGAAGCTGAA
GGGCCCCTGT CCTTAGTGAC AACAGCCAAC CACAGTCCAG ATTTTGACCA
TGACAGAGAT TACGAAGATG AACCAGTAAA CGAGGACATG GAGTGACTAT
CGGGGCGGGC CAACCCCGAG AATGAAGATT GGAAAAA

FIG. 4D.

**pAB200 protein sequence**

ALPLQPLAQG MIPTELQQLW KEVTSAHTAE ETTGNNHSSL DLTTTCVSSS
APSKTSLIMN PHASTNGQLS VHTPKRESLS HEEHPHSHPL YGHGVCKWPG
CEAVCEDFQS FLKHLNSEHA LDDRSTAQCR VQMQVVQQLE LQLAKDKERL
QAMMTHLHVK STEPKAAPQP LNLVSSVTLS KSASEASPQS LPHTPTTPTA
PLTPVTQGPS VITTTSMHTV GPIRRRYSDK YNVPISSDIA QNQEFYKNAE
VRPPFTYASL IRQAILESPE KQLTLNEIYN WFTRMFAYFR RNAATWKNAV
RHNLSLHKCF VRVENVKGAV WTVDEVEFQK RRPQKISGNP SLIKNMQSSH
AYCTPLNAAL QASMAENSIP LYTTASMGNP TLGNLASAIR EELNGAMEHT
NSNESDSSPG RSPMQAVHPV HVKEEPLDPE EAEGPLSLVT TANHSPDFDH
DRDYEDEPVN EDME

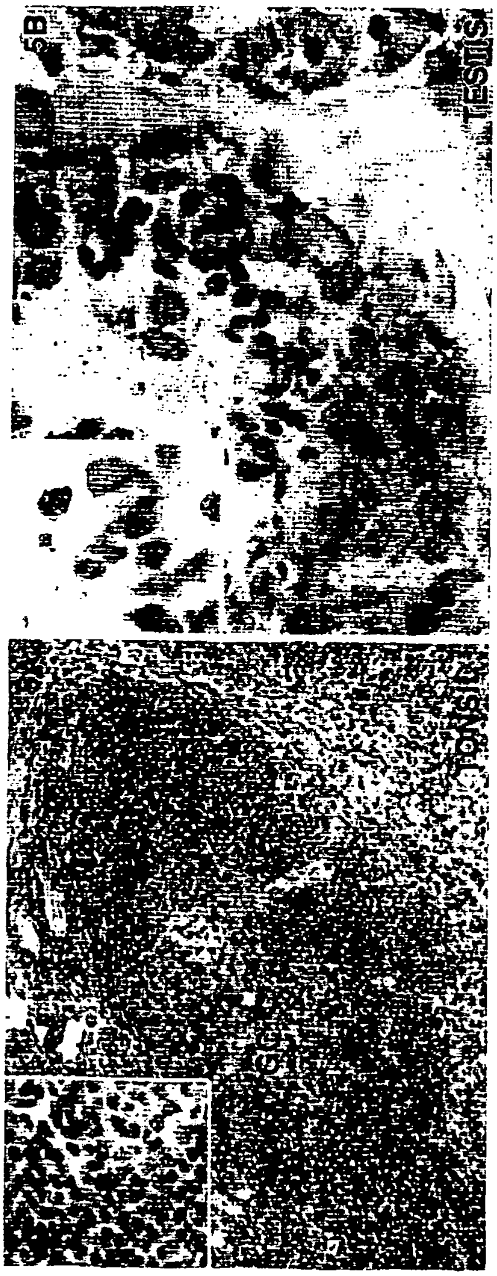


FIG. 5.

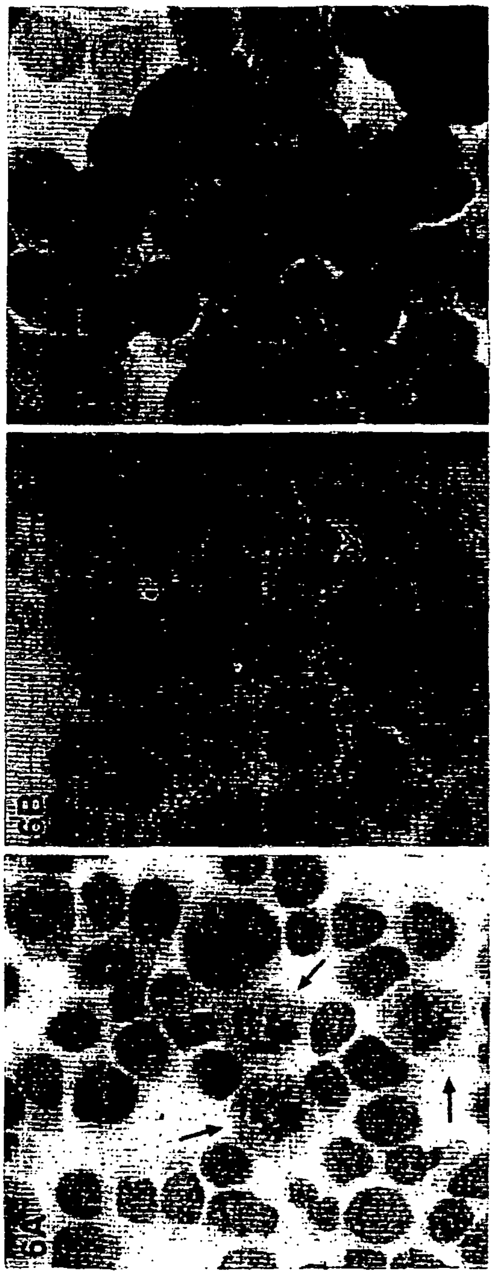


FIG. 6.

FIG. 7.

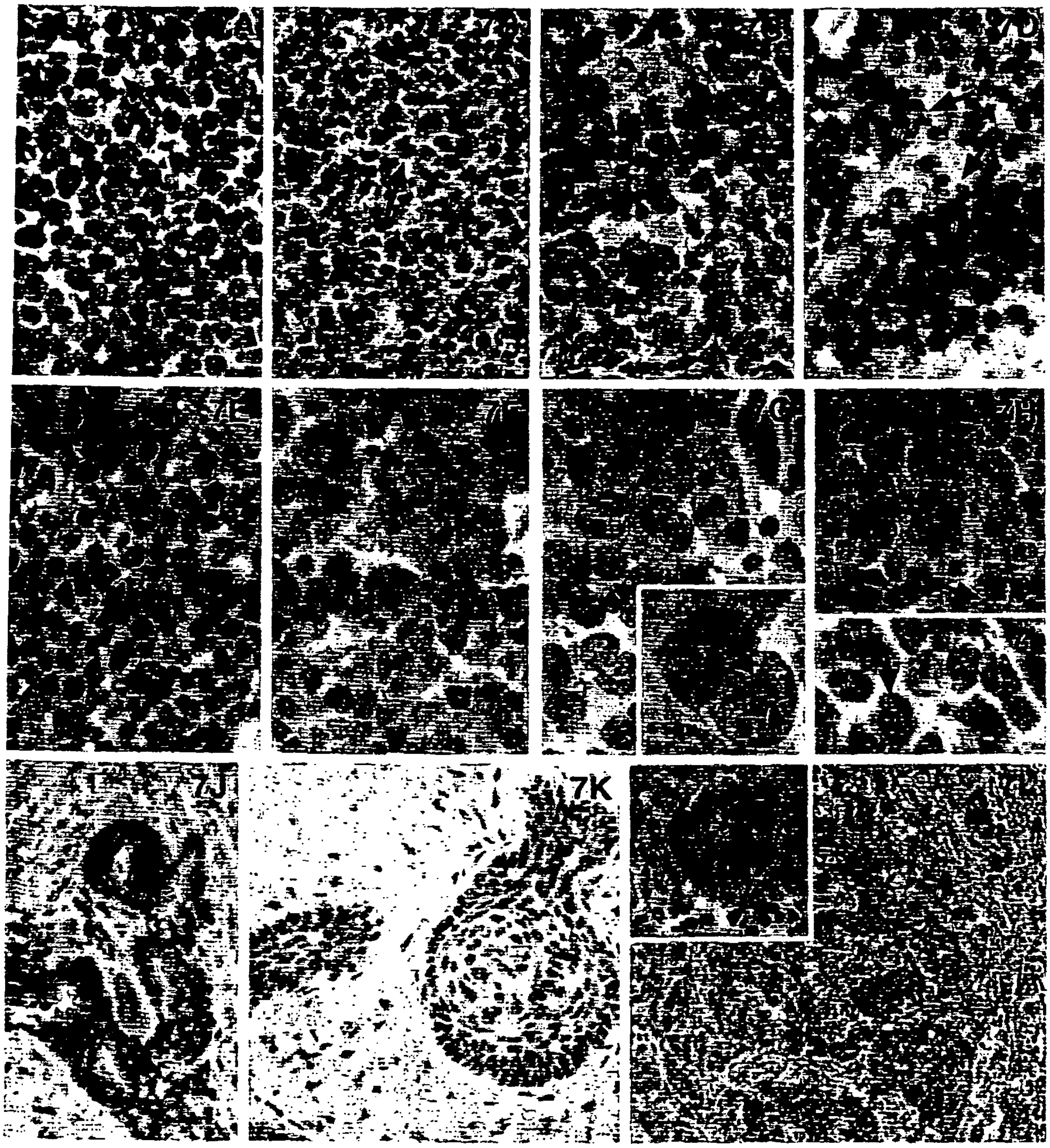

FIG. 8.

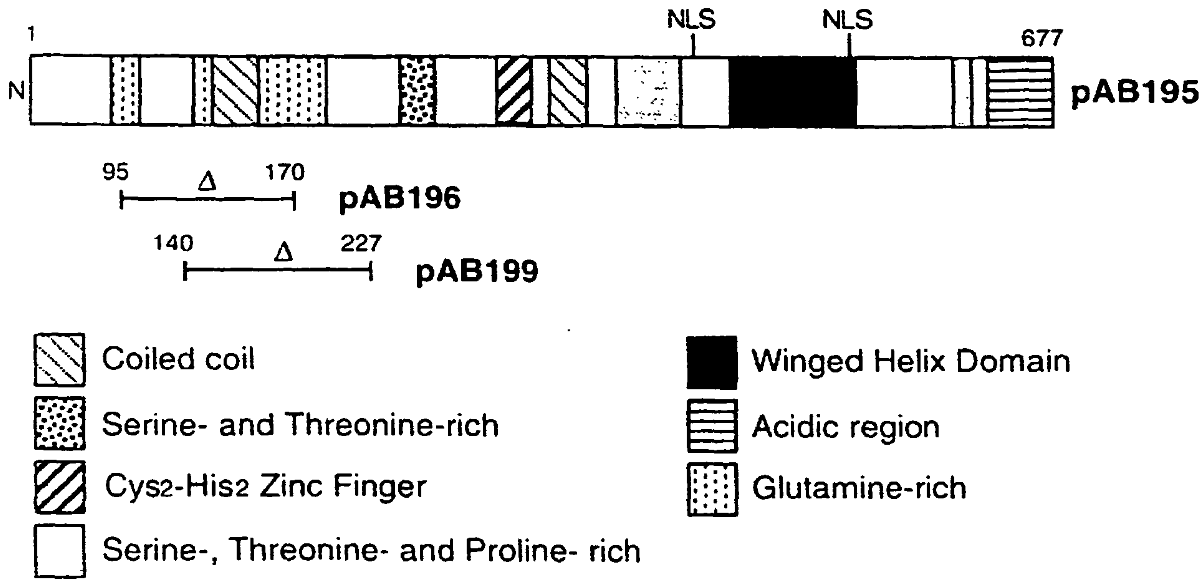
1
N
NLS
NLS
677
pAB195
95
Δ
170
pAB196
140
Δ
227
pAB199
Coiled coil
Serine- and Threonine-rich
Cys2-His2 Zinc Finger
Serine-, Threonine- and Proline- rich
Winged Helix Domain
Acidic region
Glutamine-rich

FIG. 9.

**Multiple Alignment of FOXP1 Proteins**

```
         1                                                                           80
pAB200
pAB196                                 S QKQPEPIYSK KTEIQRQTVR APFAKLFIFS ALQVARQLLL QQQQQQQVSG
pAB199
FOXP1    MMQESGTETK SNGSAIQNGS GGSNHLLECG GLREGRSNGE TPAVDIGAAD LAHAQQQQQQ ALQVARQLLL QQQQQQQVSG

         81                                                                         160
pAB200
pAB196   LKSPKRNDKQ PALQ------ ---------- ---------- ---------- ---------- ---------- ----------
pAB199                         MMTPQVITPQ QMQQILQQQV LSPQQLQVLL QQQQALMLQ- ---------- ----------
FOXP1    LKSPKRNDKQ PALQVPVSVA MMTPQVITPQ QMQQILQQQV LSPQQLQVLL QQQQALMLQQ QQLQEFYKKQ QEQLQLQLLQ
                                                                   Coiled Coil

         161                                                                        240
pAB200                                                         ALPLQPLA QGMIPTELQQ LWKEVTSAHT
pAB196   ---------- QQQVATQQLA FQQQLLQMQQ LQQQHLLSLQ RQGLLTIQPG QPALPLQPLA QGMIPTELQQ LWKEVTSAHT
pAB199   ---------- ---------- ---------- ---------- ---------- ---------- -------LQQ LWKEVTSAHT
FOXP1    QQHAGKQPKE QQQVATQQLA FQQQLLQMQQ LQQQHLLSLQ RQGLLTIQPG QPALPLQPLA QGMIPTELQQ LWKEVTSAHT

         241                                                                        320
pAB200   AEETTGNNHS SLDLTTTCVS SSAPSKTSLI MNPHASTNGQ LSVHTPKRES LSHEEHPHSH PLYGHGVCKW PGCEAVCEDF
pAB196   AEETTGNNHS SLDLTTTCVS SSAPSKTSLI MNPHASTNGQ LSVHTPKRES LSHEEHPHSH PLYGHGVCKW PGCEAVCEDF
pAB199   AEETTGNNHS SLDLTTTCVS SSAPSKTSLI MNPHASTNGQ LSVHTPKRES LSHEEHPHSH PLYGHGVCKW PGCEAVCEDF
FOXP1    AEETTGNNHS SLDLTTTCVS SSAPSKTSLI MNPHASTNGQ LSVHTPKRES LSHEEHPHSH PLYGHGVCKW PGCEAVCEDF
                                                                                  Zn Finger
```

FIG. 9 (CONTINUED).

```
                                                                             Zn Finger
        321                                                                         400
pAB200  QSFLKHLNSE HALDDRSTAQ CRVQMQVVQQ LELQLAKDKE RLQAMMTHLH VKSTEPKAAP QPLNLVSSVT LSKSASEASP
pAB196  QSFLKHLNSE HALDDRSTAQ CRVQMQVVQQ LELQLAKDKE RLQAMMTHLH VKSTEPKAAP QPLNLVSSVT LSKSASEASP
pAB199  QSFLKHLNSE HALDDRSTAQ CRVQMQVVQQ LELQLAKDKE RLQAMMTHLH VKSTEPKAAP QPLNLVSSVT LSKSASEASP
 FOXP1  QSFLKHLNSE HALDDRSTAQ CRVQMQVVQQ LELQLAKDKE RLQAMMTHLH VKSTEPKAAP QPLNLVSSVT LSKSASEASP
                                     Coiled Coil
        401                                                                         480
pAB200  QSLPHTPTTP TAPLTPVTQG PSVITTTSMH TVGPIRRRYS DKYNVPISS. DIAQNQEFYK NAEVRPPFTY ASLIRQAILE
pAB196  QSLPHTPTTP TAPLTPVTQG PSVITTTSMH TVGPIRRRYS DKYNVPISSA DIAQNQEFYK NAEVRPPFTY ASLIRQAILE
pAB199  QSLPHTPTTP TAPLTPVTQG PSVITTTSMH TVGPIRRRYS DKYNVPISSA DIAQNQEFYK NAEVRPPFTY ASLIRQAILE
 FOXP1  QSLPHTPTTP TAPLTPVTQG PSVITTTSMH TVGPIRRRYS DKYNVPISSA DIAQNQEFYK NAEVRPPFTY ASLIRQAILE
                                        NLS
        481                                                                         560
pAB200  SPEKQLTLNE IYNWFTRMFA YFRRNAATWK NAVRHNLSLH KCFVRVENVK GAVWTVDEVE FQKRRPQKIS GNPSLIKNMQ
pAB196  SPEKQLTLNE IYNWFTRMFA YFRRNAATWK NAVRHNLSLH KCFVRVENVK GAVWTVDEVE FQKRRPQKIS GNPSLIKNMQ
pAB199  SPEKQLTLNE IYNWFTRMFA YFRRNAATWK NAVRHNLSLH KCFVRVENVK GAVWTVDEVE FQKRRPQKIS GNPSLIKNMQ
 FOXP1  SPEKQLTLNE IYNWFTRMFA YFRRNAATWK NAVRHNLSLH KCFVRVENVK GAVWTVDEVE FQKRRPQKIS GNPSLIKNMQ
                              Winged Helix Domain                   NLS
        561                                                                         640
pAB200  SSHAYCTPLN AALQASMAEN SIPLYTTASM GNPTLGNLAS AIREELNGAM EHTNSNESDS SPGRSPMQAV HPVHVKEEPL
pAB196  SSHAYCTPLN AALQASMAEN SIPLYTTASM GNPTLGNLAS AIREELNGAM EHTNSNESDS SPGRSPMQAV HPVHVKEEPL
pAB199  SSHAYCTPLN AALQASMAEN SIPLYTTASM GNPTLGNLAS AIREELNGAM EHTNSNESDS SPGRSPMQAV HPVHVKEEPL
 FOXP1  SSHAYCTPLN AALQASMAEN SIPLYTTASM GNPTLGNLAS AIREELNGAM EHTNSNESDS SPGRSPMQAV HPVHVKEEPL

        641                              677
pAB200  DPEEAEGPLS LVTTANHSPD FDHDRDYEDE PVNEDME
pAB196  DPEEAEGPLS LVTTANHSPD FDHDRDYEDE PVNEDME
pAB199  DPEEAEGPLS LVTTANHSPD FDHDRDYEDE PVNEDME
 FOXP1  DPEEAEGPLS LVTTANHSPD FDHDRDYEDE PVNEDME
```

FIG. 10.

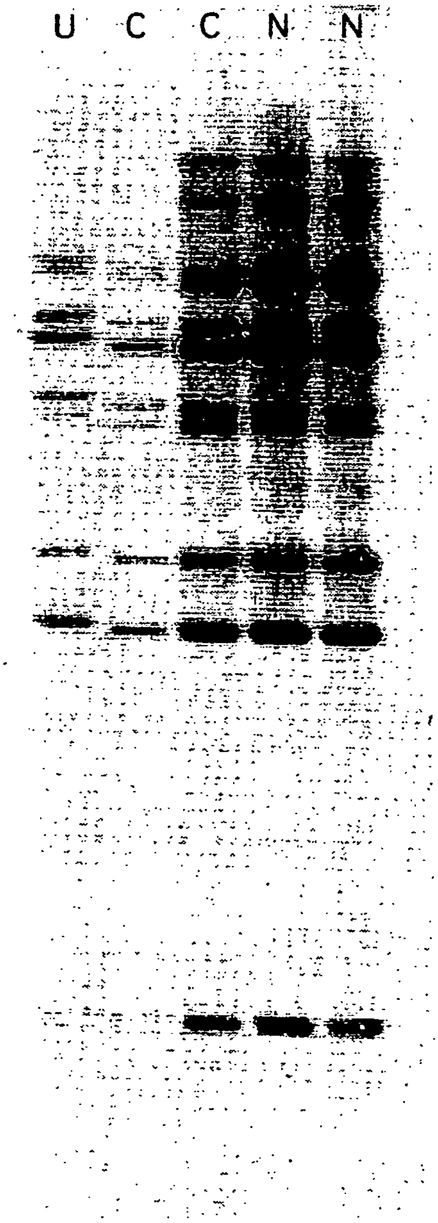

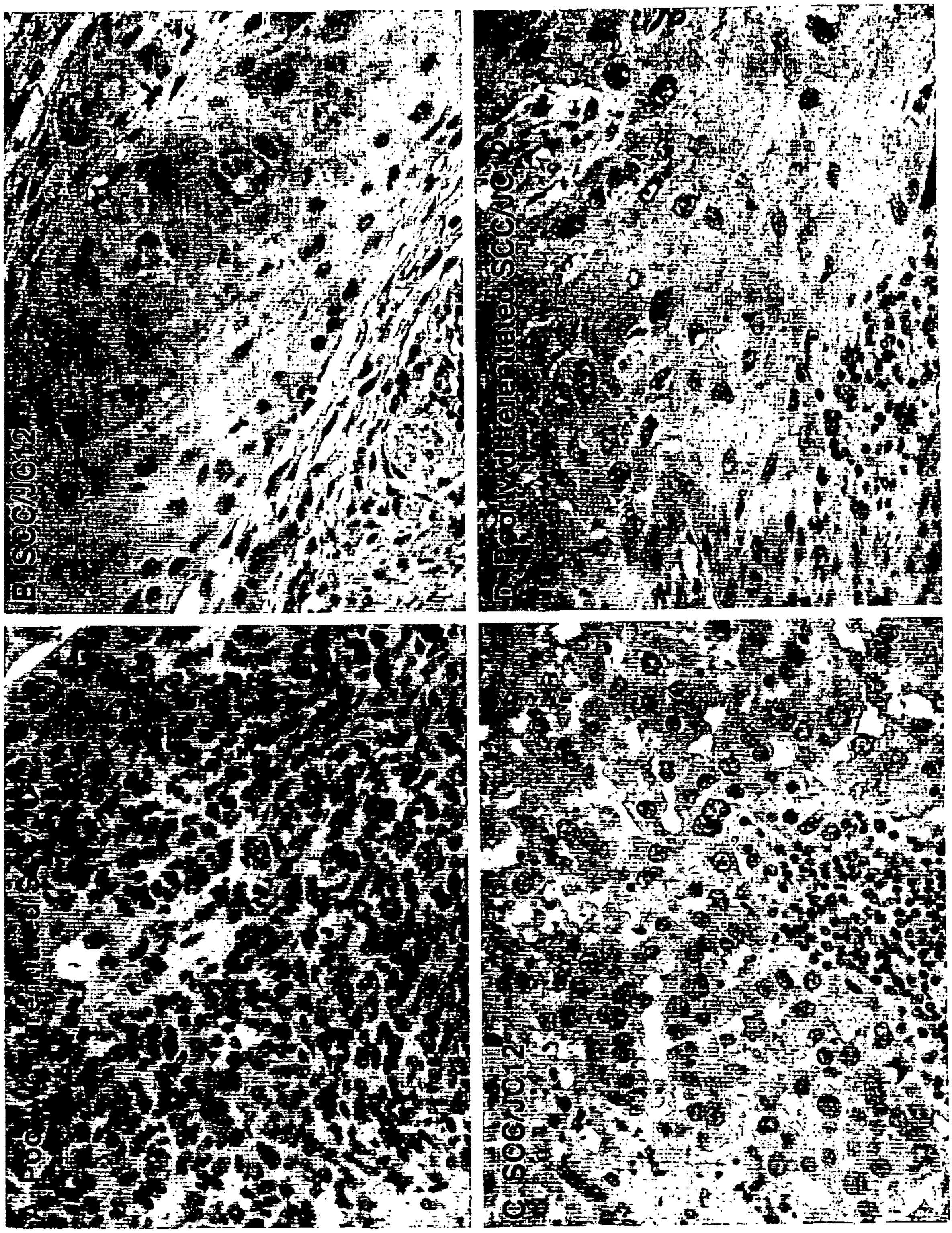


FIG. 11.

FIG. 12A.

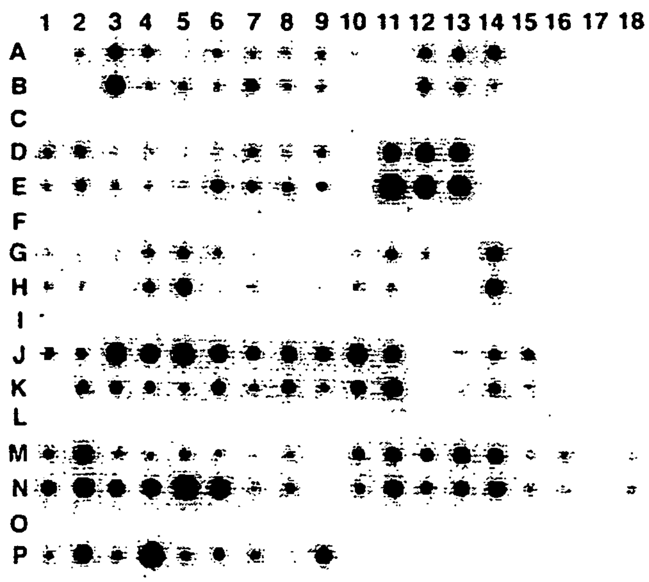

FIG. 12B.

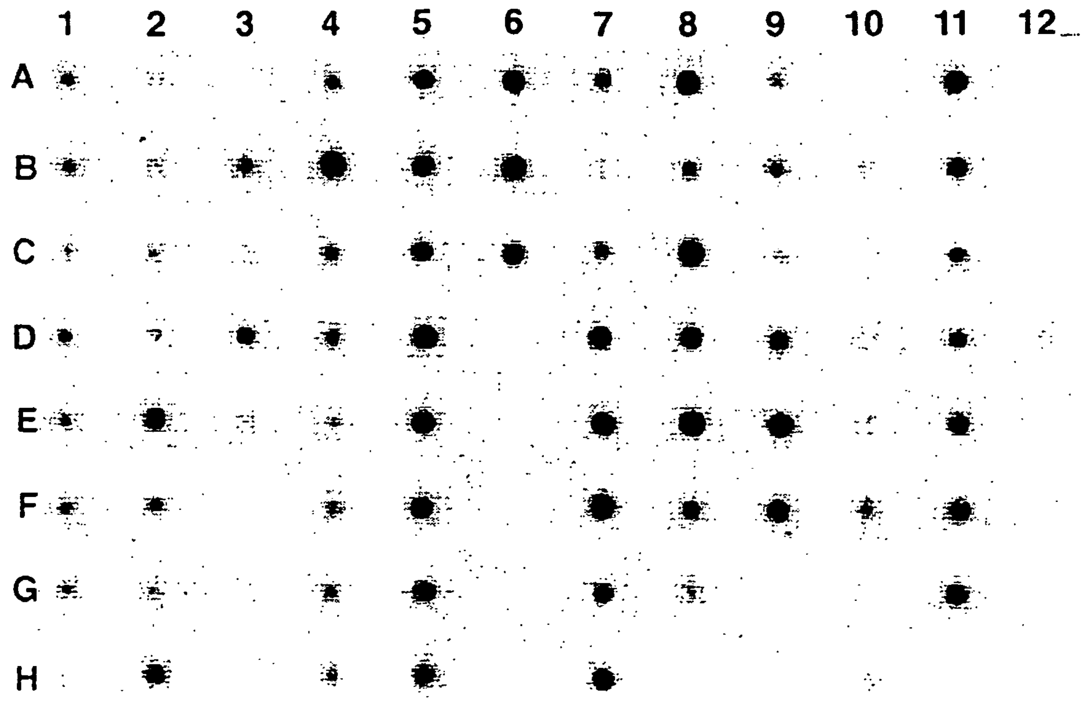


| | 1 | 2 | 3 | 4 | 5 | 6 | 7 | 8 | 9 | 10 | 11 | 12 |
|---|---|---|---|---|---|---|---|---|---|---|---|---|
| A | whole brain | cerebellum, left | substantia nigra | heart | esophagus | colon, transverse | kidney | lung | liver | leukemia, HL-60 | fetal brain | yeast total RNA |
| B | cerebral cortex | cerebellum, right | accumbens nucleus | aorta | stomach | colon, desending | skeletal muscle | placenta | pancreas | HeLa S3 | fetal heart | yeast tRNA |
| C | frontal lobe | corpus callosum | thalamus | atrium, left | duodenum | rectum | spleen | bladder | adrenal gland | leukemia, K-562 | fetal kidney | *E. coli* rRNA |
| D | parietal lobe | amygdala | pituitary gland | atrium, right | jejunum | | thymus | uterus | thyroid gland | leukemia, MOLT-4 | fetal liver | *E. coli* DNA |
| E | occipital lobe | caudate nucleus | spinal cord | ventricle, left | ileum | | peripheral blood leukocyte | prostate | salivary gland | Burkitt's lymphoma, Raji | fetal spleen | Poly r(A) |
| F | temporal lobe | hippo-campus | | ventricle, right | ilocecum | | lymph node | testis | mammary gland | Burkitt's lymphoma, Daudi | fetal thymus | human $C_0t$-1 DNA |
| G | p. g.* of cerebral cortex | medulla oblongata | | inter-ventricular septum | appendix | | bone marrow | ovary | | colorectal adeno-carcinoma, SW480 | fetal lung | human DNA 100 ng |
| H | pons | putamen | | apex of the heart | colon, ascending | | trachea | | | lung carcinoma, A549 | | human DNA 500 ng |

* paracentral gyrus

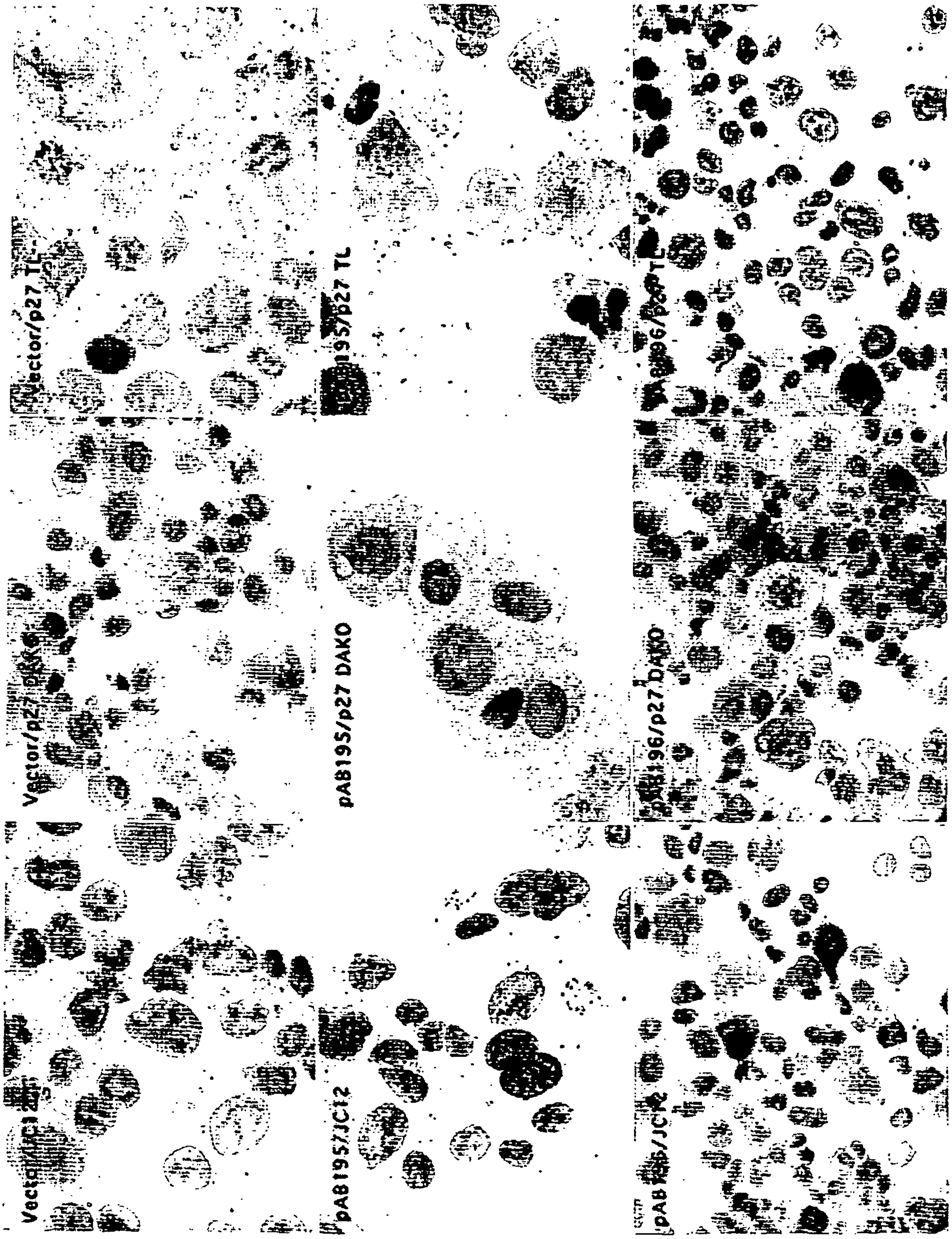
Vector/p27 TL
Vector/p27 DAKO
pAB195/p27 DAKO
pAB196/p27 DAKO
pAB195/JC12

FIG. 13.

FIG. 14.

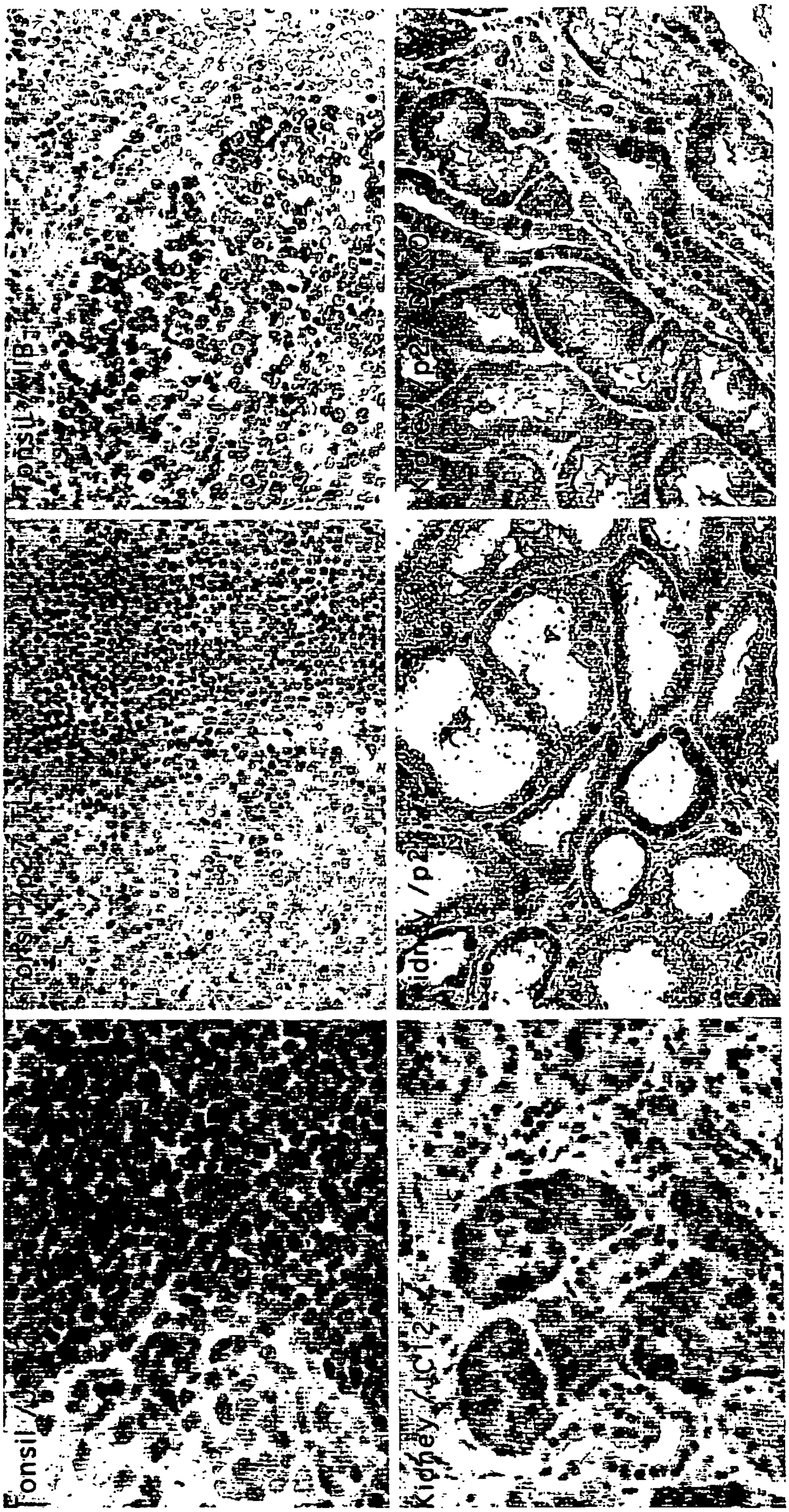

FIG. 15.

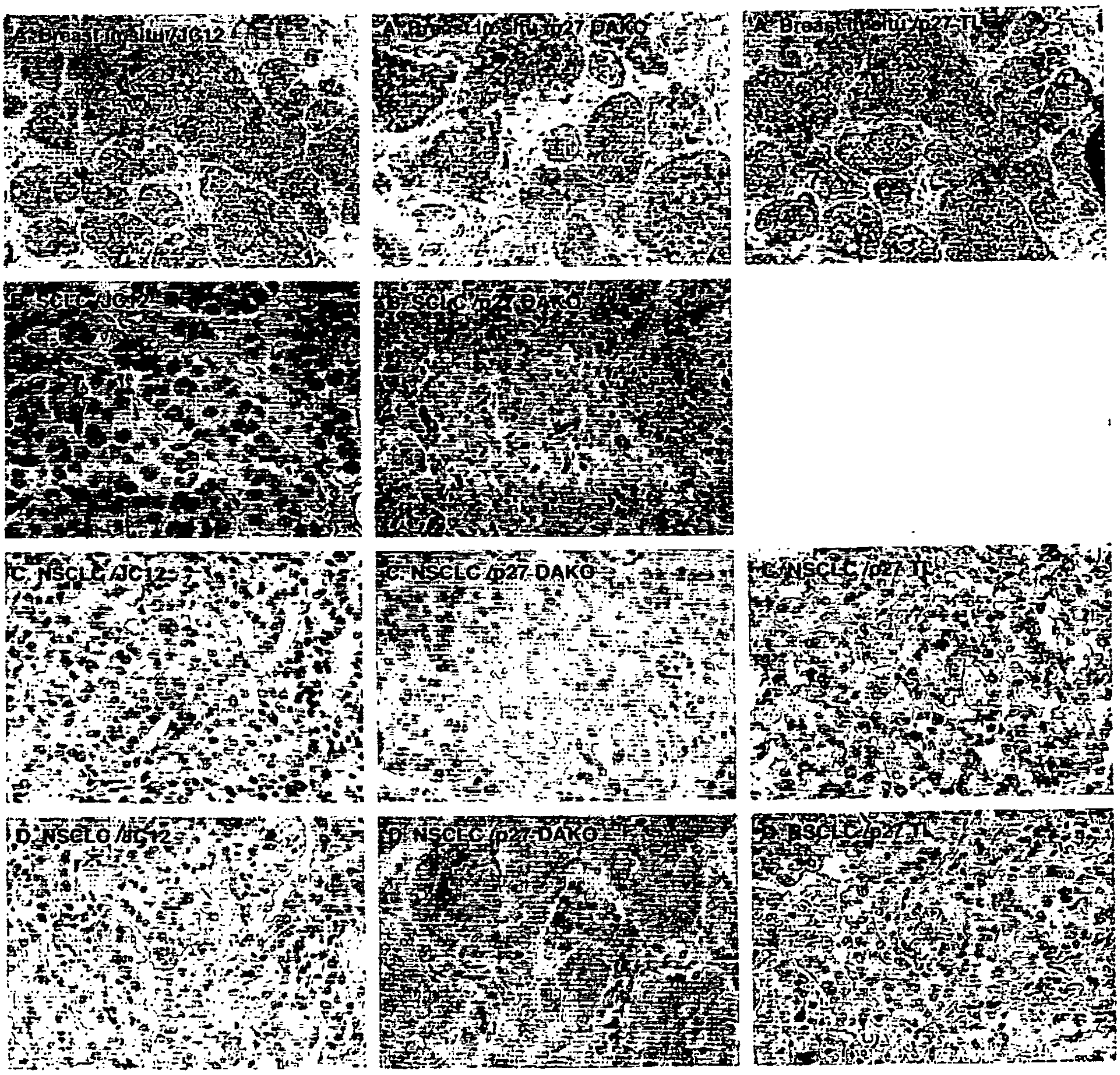
A Breast In Situ / JC12
A Breast In Situ / p27 DAKO
A Breast In Situ / p27 TL
B SCLC / JC12
B SCLC / p27 DAKO
C NSCLC / JC12
C NSCLC / p27 DAKO
C NSCLC / p27 TL
D NSCLC / JC12
D NSCLC / p27 DAKO
D NSCLC / p27 TL

FIG. 16.

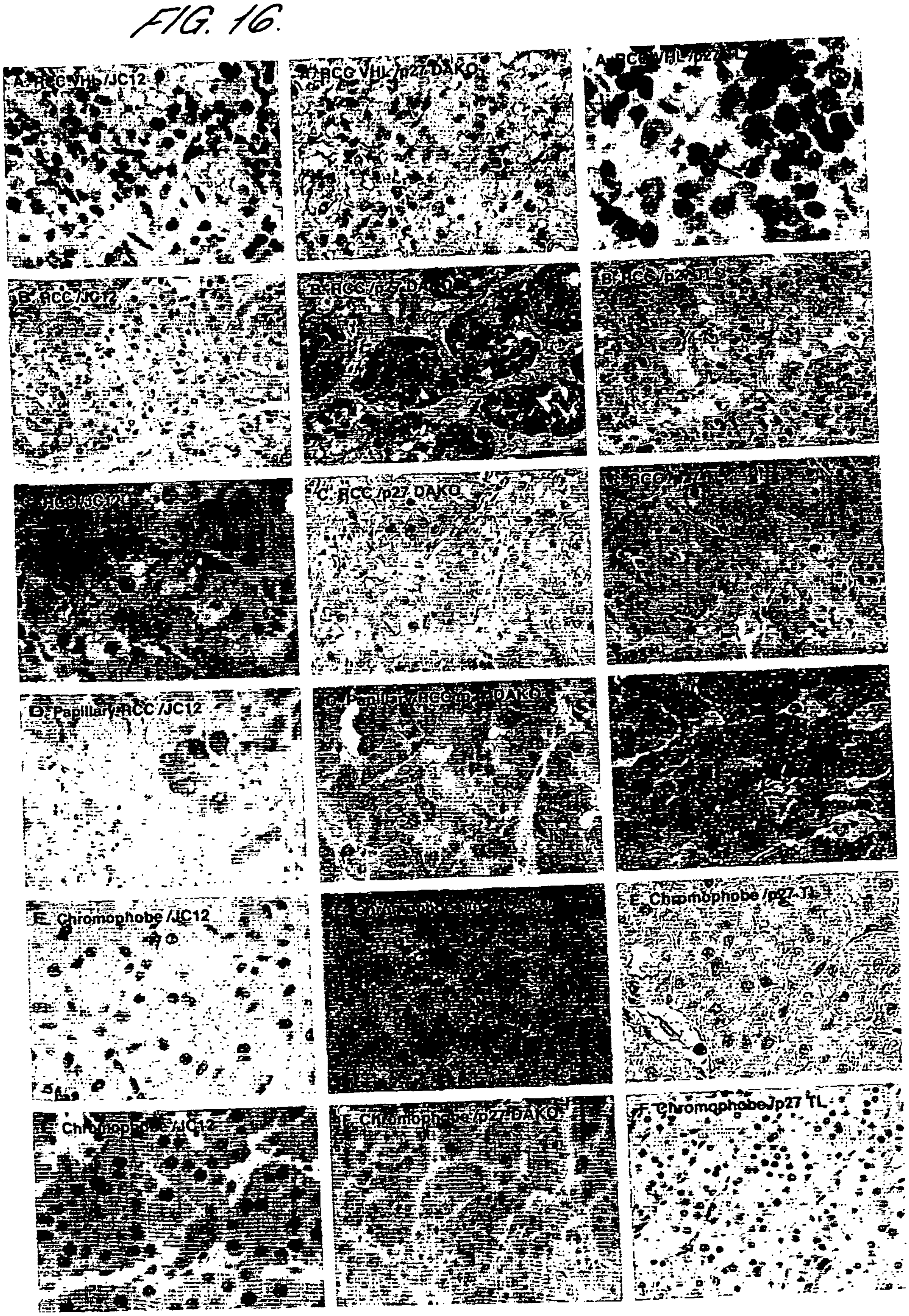
A. RCC VHL /JC12
A. RCC VHL /p27 DAKO
B. RCC /JC12
B. RCC /p27 DAKO
C. RCC /p27 DAKO
D. Papillary RCC /JC12
E. Chromophobe /JC12
E. Chromophobe /p27 TL
F. Chromophobe /JC12
F. Chromophobe /p27 DAKO
F. Chromophobe /p27 TL

FIG. 17.

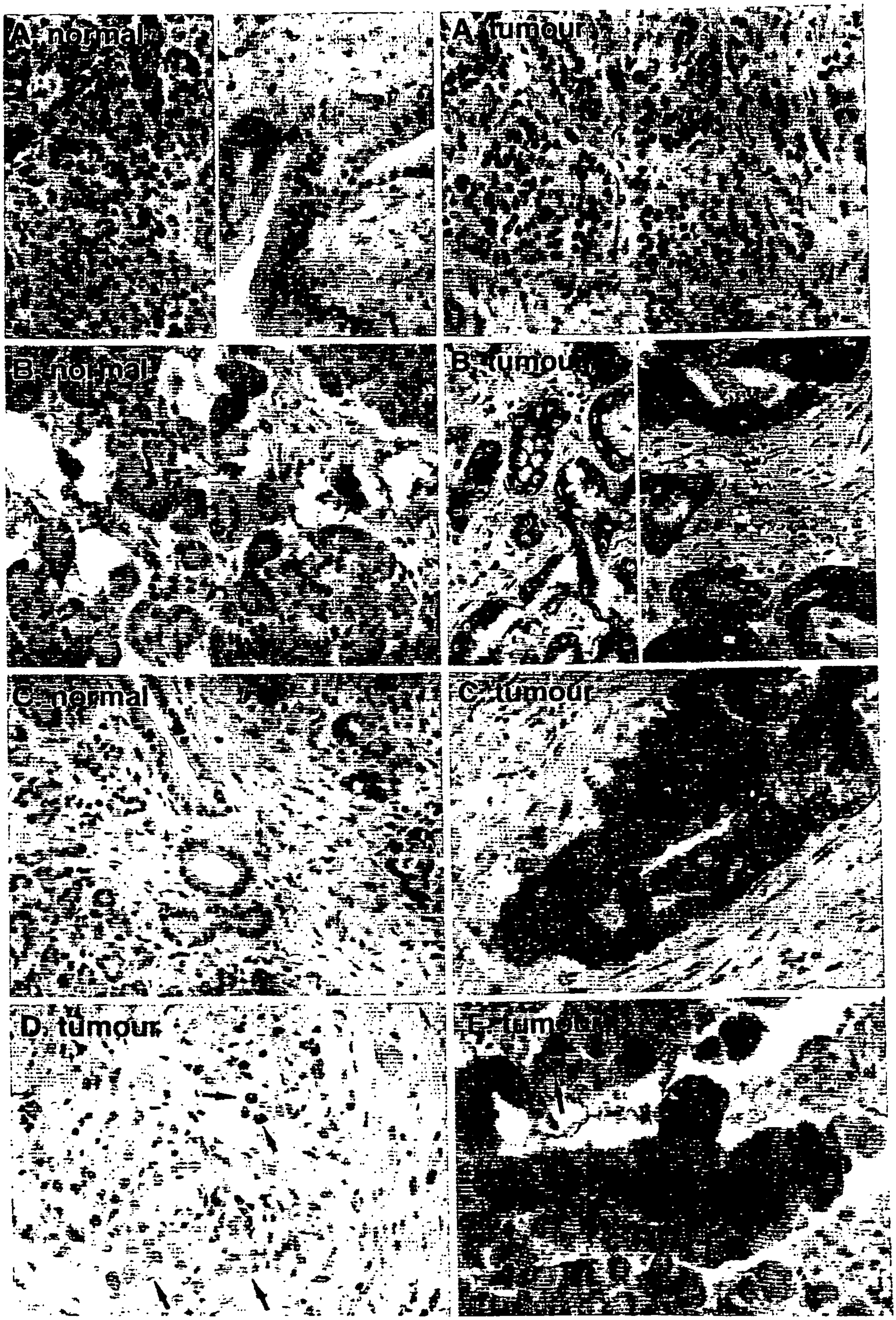
A normal
A tumour
B normal
B tumour
C normal
C tumour
D tumour
E tumour

FIG. 18.

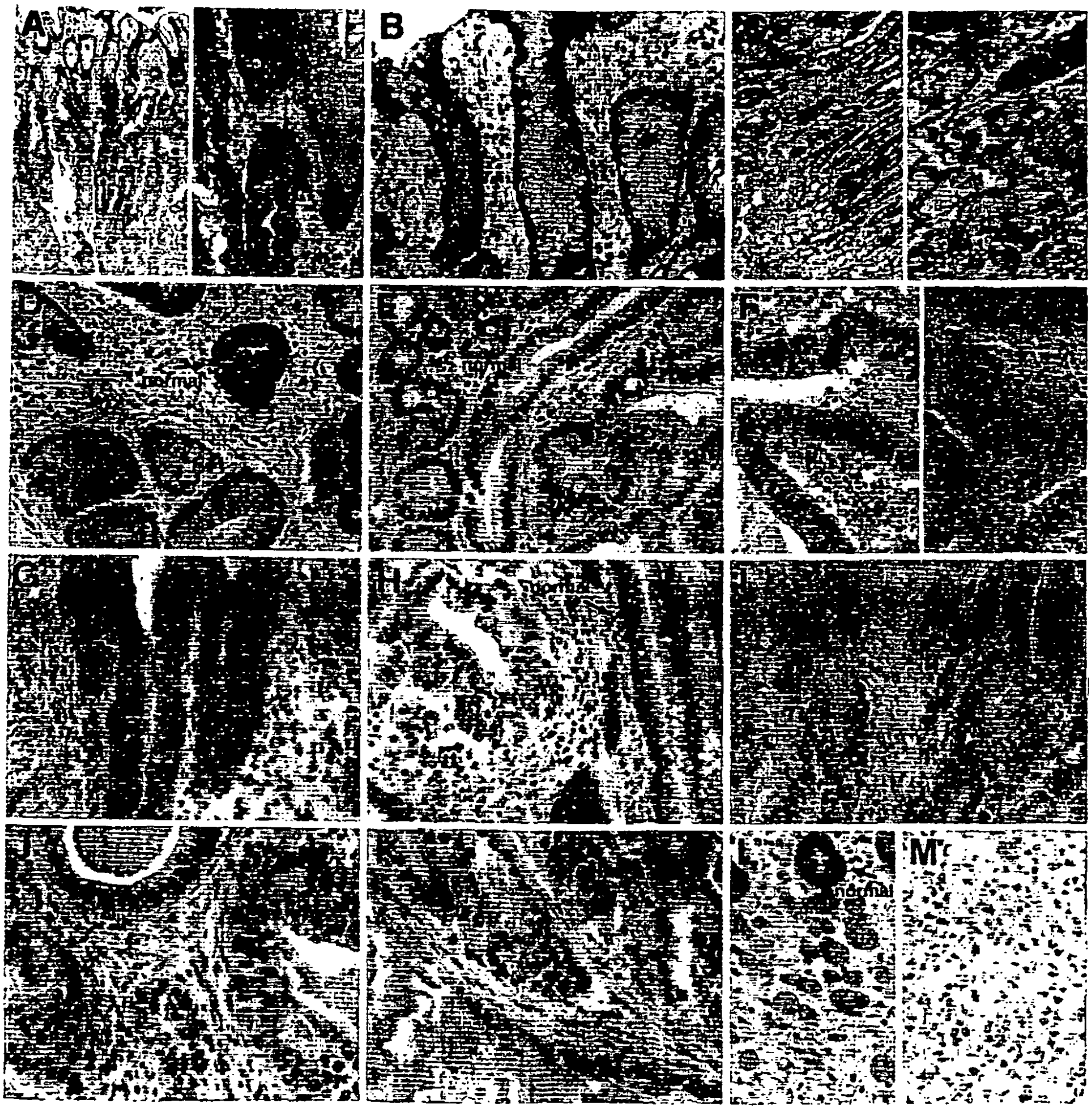

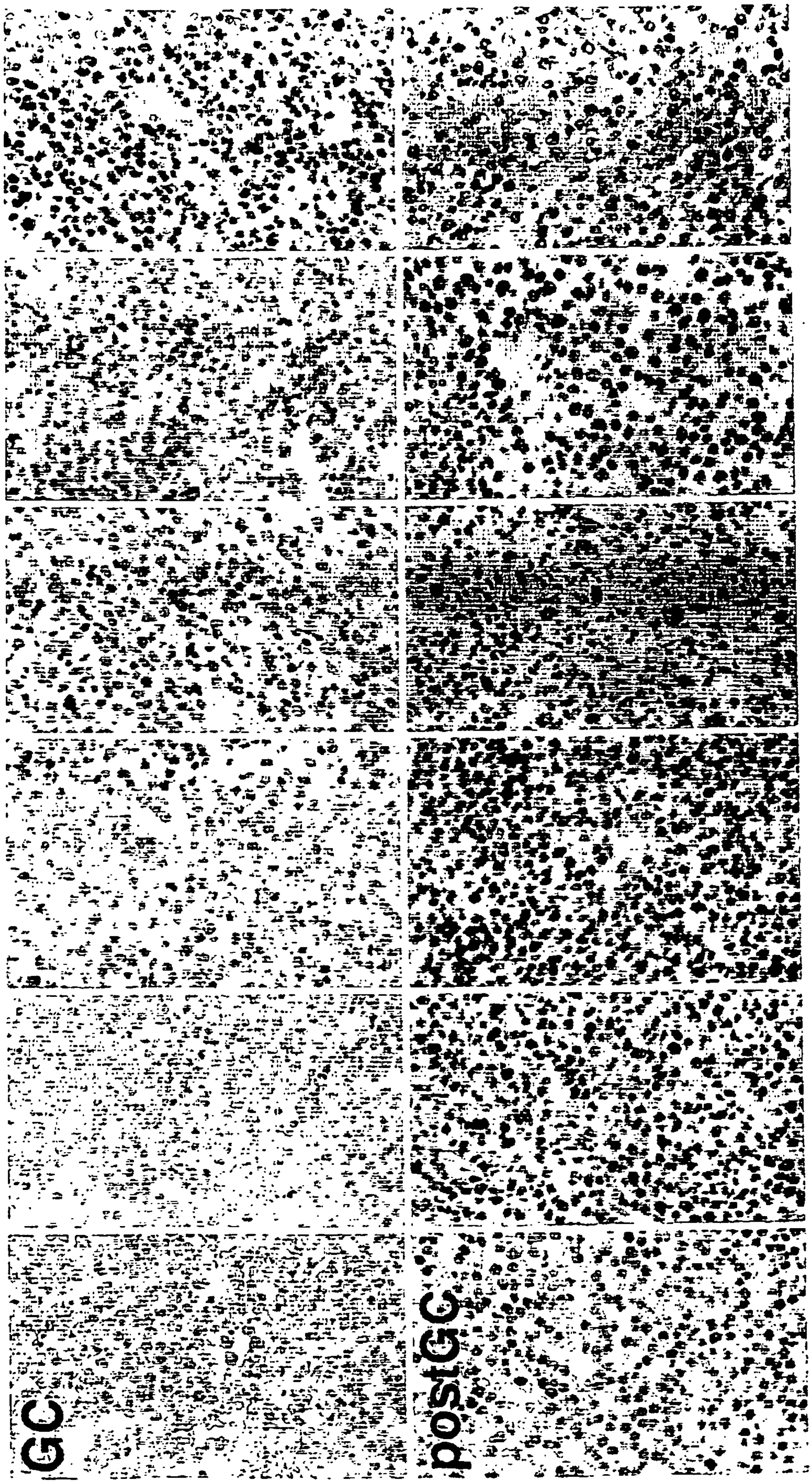


FIG. 19.

FIG. 20.

Kaplan-Meier Cum. Survival Plot for OVERAL SURVIVAL
Censor Variable: <None>
Stratification Variable: JC12 Intensity
Row exclusion: JC12Piris.doc

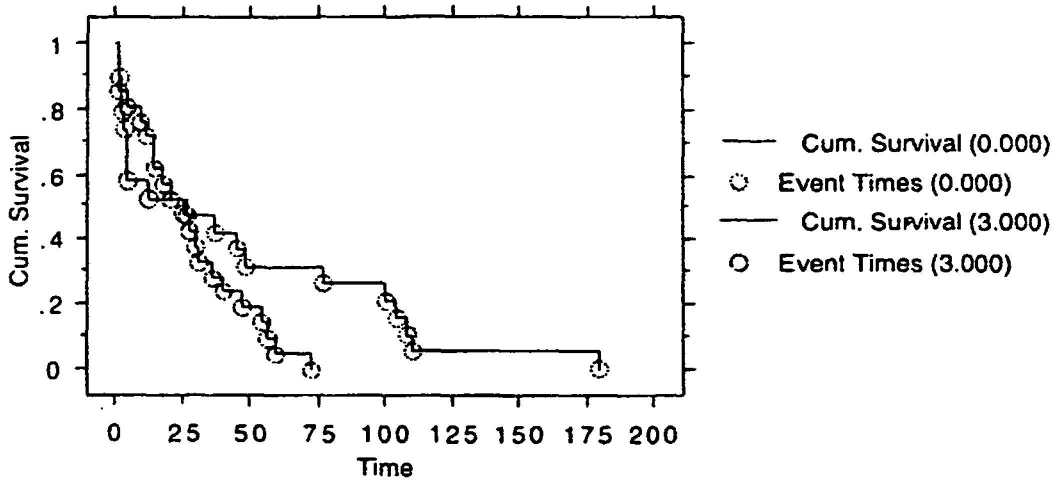


Kaplan-Meier Cum. Survival Plot for DISEASE FREE SURVIVAL
Censor Variable: <None>
Stratification Variable: JC12 Intensity
Row exclusion: JC12Piris.doc

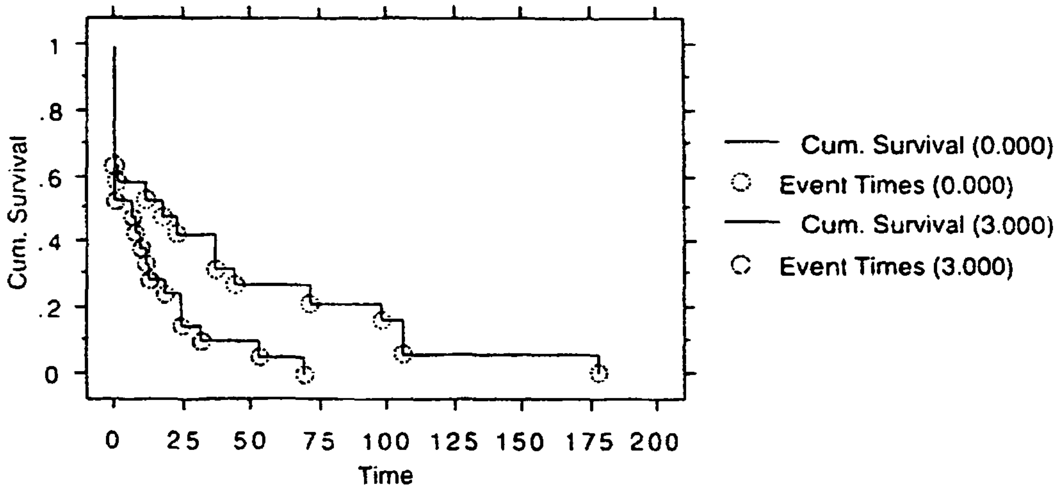

FIG. 21.

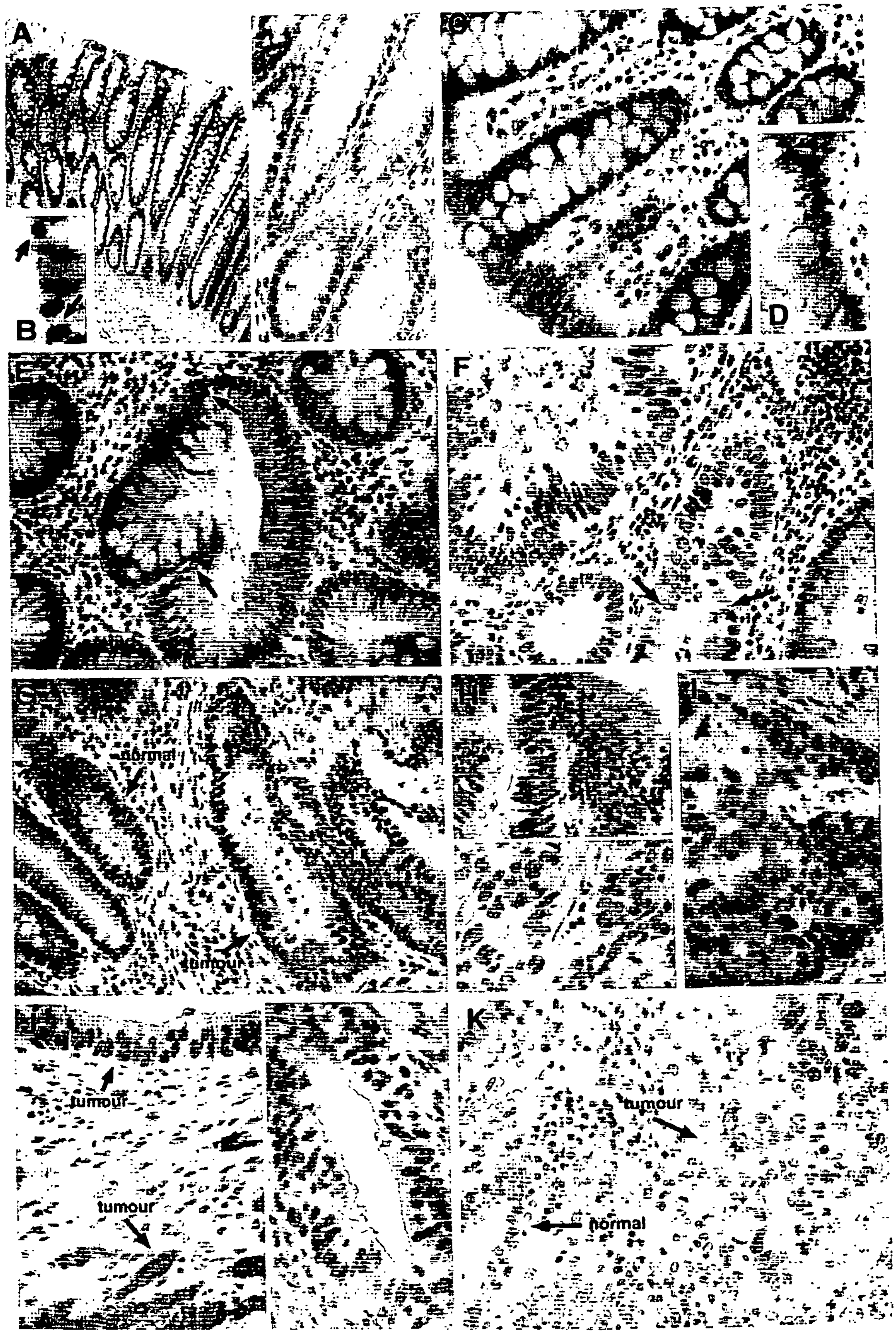

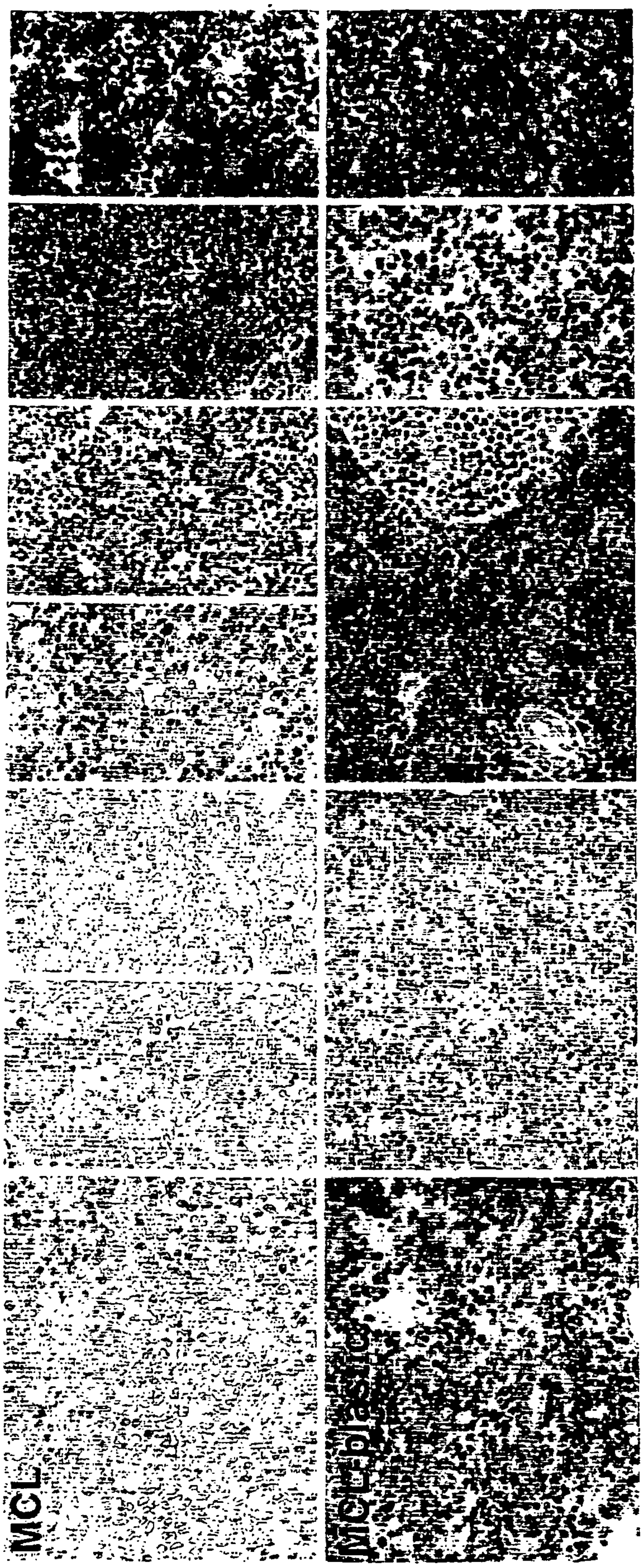


FIG. 22

FIG. 23.